# EUROPEAN PATENT APPLICATION

(11) **EP 3 205 663 A1**
(43) Date of publication of application: **16.08.2017**
(21) Application number: 16155719.4
(22) Date of filing: 15.02.2016
(51) Int. Cl.: C07K 14/435, C12N 5/00, C12N 15/62

(54) **MEANS AND METHODS FOR INHIBITING GENE EXPRESSION**

(71) Applicant: Max-Planck-Gesellschaft zur Förderung der Wissenschaften e.V., 80539 München (DE); Albert-Ludwigs-Universität Freiburg, 79098 Freiburg (DE)
(72) Inventor: The designation of the inventor has not yet been filed
(74) Representative: Vossius & Partner Patentanwälte Rechtsanwälte mbB

(57) **Abstract**

The present invention relates to a nucleic acid molecule encoding a polypeptide capable of binding to TFIIB and inhibiting gene expression, wherein the polypeptide (a) has the amino acid sequence of SEQ ID NO: 1; (b) is a fragment of the amino acid sequence of (a), said fragment lacking up to five amino acids at the C-terminus of the amino acid sequence of (a); or (c) has an amino acid sequence being at least 80% identical to the amino acid sequence of (a) or (b).

SEQUENCE LISTING

<110> Max-Planck-Gesellschaft zur Förderung der Wissenschaften e.V.

<120> Means and methods for inhibiting gene expression

<130> Y2237 EP

<160> 11

<170> BiSSAP 1.3

<210> 1
<211> 80
<212> PRT
<213> Artificial Sequence

<220>
<223> THOV-ML AMINO ACIDS 225-304

<400> 1
Leu Ile Gly Trp Met Asp Glu Ile Ile Lys Ile Phe Ser Lys Pro Asp
1               5                   10                  15
Tyr Leu Glu Ala Lys Gly Val Ser Tyr Gln Val Leu Lys Asn Val Ser
            20                  25                  30
Asn Lys Val Ala Leu Leu Arg Glu Ser Ile Trp Trp Val Thr Glu Leu
        35                  40                  45
Asp Gly Arg Glu Tyr Leu Phe Phe Asp Glu Ser Trp Tyr Leu His Gly
    50                  55                  60
Met Ser Ala Phe Ser Asp Gly Val Pro Gly Tyr Glu Asp Phe Ile Tyr
65                  70                  75                  80

<210> 2
<211> 70
<212> PRT
<213> Artificial Sequence

<220>
<223> THOV-ML AMINO ACIDS 235-304

<400> 2
Ile Phe Ser Lys Pro Asp Tyr Leu Glu Ala Lys Gly Val Ser Tyr Gln
1               5                   10                  15
Val Leu Lys Asn Val Ser Asn Lys Val Ala Leu Leu Arg Glu Ser Ile
            20                  25                  30
Trp Trp Val Thr Glu Leu Asp Gly Arg Glu Tyr Leu Phe Phe Asp Glu
        35                  40                  45
Ser Trp Tyr Leu His Gly Met Ser Ala Phe Ser Asp Gly Val Pro Gly
    50                  55                  60
Tyr Glu Asp Phe Ile Tyr
65                  70

<210> 3
<211> 60
<212> PRT
<213> Artificial Sequence

<220>
<223> THOV-ML AMINO ACIDS 245-304

<400> 3

Lys Gly Val Ser Tyr Gln Val Leu Lys Asn Val Ser Asn Lys Val Ala
1               5                   10                  15
Leu Leu Arg Glu Ser Ile Trp Trp Val Thr Glu Leu Asp Gly Arg Glu
            20                  25                  30
Tyr Leu Phe Phe Asp Glu Ser Trp Tyr Leu His Gly Met Ser Ala Phe
        35                  40                  45
Ser Asp Gly Val Pro Gly Tyr Glu Asp Phe Ile Tyr
    50                  55                  60

<210> 4
<211> 58
<212> PRT
<213> Artificial Sequence

<220>
<223> THOV-ML AMINO ACIDS 247-304

<400> 4
Val Ser Tyr Gln Val Leu Lys Asn Val Ser Asn Lys Val Ala Leu Leu
1               5                   10                  15
Arg Glu Ser Ile Trp Trp Val Thr Glu Leu Asp Gly Arg Glu Tyr Leu
            20                  25                  30
Phe Phe Asp Glu Ser Trp Tyr Leu His Gly Met Ser Ala Phe Ser Asp
        35                  40                  45
Gly Val Pro Gly Tyr Glu Asp Phe Ile Tyr
    50                  55

<210> 5
<211> 57
<212> PRT
<213> Artificial Sequence

<220>
<223> THOV-ML AMINO ACIDS 248-304

<400> 5
Ser Tyr Gln Val Leu Lys Asn Val Ser Asn Lys Val Ala Leu Leu Arg
1               5                   10                  15
Glu Ser Ile Trp Trp Val Thr Glu Leu Asp Gly Arg Glu Tyr Leu Phe
            20                  25                  30
Phe Asp Glu Ser Trp Tyr Leu His Gly Met Ser Ala Phe Ser Asp Gly
        35                  40                  45
Val Pro Gly Tyr Glu Asp Phe Ile Tyr
    50                  55

<210> 6
<211> 56
<212> PRT
<213> Artificial Sequence

<220>
<223> THOV-ML AMINO ACIDS 249-304

<400> 6
Tyr Gln Val Leu Lys Asn Val Ser Asn Lys Val Ala Leu Leu Arg Glu
1               5                   10                  15
Ser Ile Trp Trp Val Thr Glu Leu Asp Gly Arg Glu Tyr Leu Phe Phe
            20                  25                  30
Asp Glu Ser Trp Tyr Leu His Gly Met Ser Ala Phe Ser Asp Gly Val
        35                  40                  45
Pro Gly Tyr Glu Asp Phe Ile Tyr
    50                  55

<210> 7
<211> 58
<212> PRT
<213> Artificial Sequence

<220>
<223> THOV-ML AMINO ACIDS 247-304 (not binding SOLH)

<400> 7
Val Ser Tyr Gln Val Leu Lys Asn Val Ser Asn Lys Val Ala Leu Leu
1               5                   10                  15
Arg Glu Ser Ile Trp Trp Val Ala Ala Leu Asp Gly Arg Glu Tyr Leu
            20                  25                  30
Phe Phe Asp Glu Ser Trp Tyr Leu His Gly Met Ser Ala Phe Ser Asp
        35                  40                  45
Gly Val Pro Gly Tyr Glu Asp Phe Ile Tyr
    50                  55

<210> 8
<211> 57
<212> PRT
<213> Artificial Sequence

<220>
<223> THOV-ML AMINO ACIDS 248-304 (not binding SOLH)

<400> 8
Ser Tyr Gln Val Leu Lys Asn Val Ser Asn Lys Val Ala Leu Leu Arg
1               5                   10                  15
Glu Ser Ile Trp Trp Val Ala Ala Leu Asp Gly Arg Glu Tyr Leu Phe
            20                  25                  30
Phe Asp Glu Ser Trp Tyr Leu His Gly Met Ser Ala Phe Ser Asp Gly
        35                  40                  45
Val Pro Gly Tyr Glu Asp Phe Ile Tyr
    50                  55

<210> 9
<211> 56
<212> PRT
<213> Artificial Sequence

<220>
<223> THOV-ML AMINO ACIDS 249-304 (not binding SOLH)

<400> 9
Tyr Gln Val Leu Lys Asn Val Ser Asn Lys Val Ala Leu Leu Arg Glu
1               5                   10                  15
Ser Ile Trp Trp Val Ala Ala Leu Asp Gly Arg Glu Tyr Leu Phe Phe
            20                  25                  30
Asp Glu Ser Trp Tyr Leu His Gly Met Ser Ala Phe Ser Asp Gly Val
        35                  40                  45
Pro Gly Tyr Glu Asp Phe Ile Tyr
    50                  55

<210> 10
<211> 304
<212> PRT
<213> Thogoto virus

<220>
<223> ML amino acid sequence

<400> 10

Met Ala Thr Ser Leu Pro Val Arg Ala Phe Ser Glu Val Cys Cys Gln
1               5                   10                  15
Glu Ala Lys Ala Ala Ile Ile Gln Met Glu Asn Asn Pro Asp Glu Thr
            20                  25                  30
Val Cys Asn Arg Ile Trp Lys Ile His Arg Asp Met Gln Ser Ser Asp
        35                  40                  45
Leu Thr Thr Thr Val Gln Val Met Met Val Tyr Arg Phe Ile Ser Lys
    50                  55                  60
Arg Val Pro Glu Gly Cys Phe Thr Ile Leu Ser Gly Ile Asn Thr Ser
65                  70                  75                  80
Leu Tyr Asn Pro Arg Glu Leu Lys Arg Ser Tyr Val Gln Ala Ile Ser
                85                  90                  95
Thr Gly Thr Ser Cys Glu Phe Leu Lys Ser Leu Asp Lys Leu Ala Arg
            100                 105                 110
Asn Leu Leu Ala Val His Val Cys Ser Asp Val Lys Met Ser Leu Ser
        115                 120                 125
Lys Arg Gln Val Ile Asp Phe Leu Ser Gly Glu Asn Asp Pro Ala Leu
    130                 135                 140
His Ala Ala Glu His Leu Thr Ser Leu Ala Leu Asp Asp Ser Pro Ser
145                 150                 155                 160
Ala Val Met Tyr Ser Gly Trp Gln Gln Glu Ala Val Lys Leu His Asn
                165                 170                 175
Thr Ile Arg Lys Ile Ala Thr Met Arg Pro Ala Asp Cys Lys Ala Gly
            180                 185                 190
Lys Phe Tyr Ser Asp Ile Leu Ser Ala Cys Asp Gln Thr Lys Glu Leu
        195                 200                 205
Leu Asp Ser Phe Asp Gln Gly Lys Leu Ser Tyr Asp Arg Asp Val Val
    210                 215                 220
Leu Ile Gly Trp Met Asp Glu Ile Ile Lys Ile Phe Ser Lys Pro Asp
225                 230                 235                 240
Tyr Leu Glu Ala Lys Gly Val Ser Tyr Gln Val Leu Lys Asn Val Ser
                245                 250                 255
Asn Lys Val Ala Leu Leu Arg Glu Ser Ile Trp Trp Val Thr Glu Leu
            260                 265                 270
Asp Gly Asn Glu Tyr Leu Phe Phe Asp Asp Ser Trp Tyr Leu His Gly
        275                 280                 285
Ile Ser Ala Phe Ser Asp Gly Val Pro Gly Tyr Glu Asp Phe Ile His
    290                 295                 300

<210> 11
<211> 915
<212> DNA
<213> Thogoto virus

<220>
<223> ML complete cDNA sequence

<400> 11
atggcttcca acctaccagt gaggtctttc agcgaagtct gctgtgctga ggcacgggcg      60

gctataatac agatggagaa caaccccgac gaaaccgtgt gtaataggat ctggaagatc     120

caccgggacc tacagtcaag tgacctcacc accactgtgc aagtaatgat ggtgtatcgt     180

ttcatatcaa aacgggtgcc agaaggctgc ttcgccatcc tttctggtgt gaacactgga     240

atgtacaacc caagagagct taaacgaagt tatgtgcaat ccctgtcttc aggaacctcc     300

tgtgagtttc tgagatcatt agacaagcta gcaaagaact tgctggctgt acatgtctgc     360

tctgatgtga agatgtccct caacaaaagg caggtgatag attttataag cggtgaagaa     420

gatcccaccc tccacacagc agagcatcta acaagcttgg ccctggatga cagcccttct 480

gcagtggtgt acagtggatg gcagcaggaa gcgatcaagc ttcacaacac catcaggaaa 540

attgctacga tgaggcctgc agactgcaaa gcagggaagt tctactcgga catcttatcc 600

gcctgcgacc agacaaaaga acttctagac gccttcgacc agggaaaact tgcctatgac 660

cgagacgtag tgctcatcgg gtggatggat gagatcatca aaatcttctc aaaacctgat 720

tatctggagg cgaagggagt ctcatatcag gtcctcaaga acgtcagcaa caaagtcgcc 780

cttctccggg aaagcatttg gtgggttaca gaattagatg gaagagaata tctcttcttc 840

gatgagtctt ggtacttaca tggcatgtct gctttttcag atggtgtgcc tggatacgaa 900

gacttcattt actaa 915

## Description

The present invention relates to a nucleic acid molecule encoding a polypeptide capable of binding to TFIIB and inhibiting gene expression, wherein the polypeptide (a) has the amino acid sequence of SEQ ID NO: 1; (b) is a fragment of the amino acid sequence of (a), said fragment lacking up to five amino acids at the C-terminus of the amino acid sequence of (a); or (c) has an amino acid sequence being at least 80% identical to the amino acid sequence of (a) or (b).

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Central to all gene transcriptional activity in the cell is the assembly of a pre-initiation complex (PIC) containing the DNA template, RNA polymerase II (RNAPII), the general transcription factors (GTFs) TFIIA, TFIIB, TFIID, TFIIE, TFIIF, TFIIH and Mediator. A fully assembled PIC has a molecular size of approximately 4 MDa. TFIID nucleates PIC assembly by binding promoter sequences through its TATA binding protein (TBP subunit). TFIIB interacts with TBP to recruit an RNAPII-TFIIF complex. TFIIE, TFIIH and RNAPII catalyze promoter melting and transition from transcriptional initiation to elongation (Kandiah et al., 2014). The activity of single components of the PIC has been studied in detail, e.g. by assays using transcription factor depleted nuclear extracts and evaluating transcription from template DNA.

Inducible gene expression requires general as well as promoter/signal specific transcription factors that are activated upon specific cues and bind to distinct DNA sequences in the promoter region. The virus-induced expression of the human IFN-b gene is one of the best-characterized examples for inducible gene expression in higher eukaryotes. IFN-b is not expressed under steady state conditions but is rapidly synthesized upon virus infection. This relies on sensing of virus infection or of other stimuli by dedicated pattern recognition receptors (PRRs), which initiate a signaling cascade that leads to activation of transcription factors including NF-κB, IRF-3/IRF-7 and ATF-2/c-JUN and local disassembly of nucleosomes. After activation these transcription factors get phosphorylated, dimerize, translocate to the nucleus, engage the cellular polymerase-II transcription complex and finally bind to a transcription factor specific promoter region to activate expression of antiviral cytokines and type-I interferon (IFN-a/b) genes. IFN-a/b is secreted and binds to the interferon type A receptor, thereby triggering a second signaling event involving signal transducer and activator of transcription (STAT)-1 and STAT-2 which similarly dimerize, translocate to the nucleus and lead to transcription of genes encoding proteins with antiviral properties. During an antiviral immune response several hundred genes are transcriptionally upregulated and synergistically mount an efficient antiviral barrier. This barrier encompasses proteins with direct antiviral properties such as IFIT and MX proteins, but also includes increased expression of PRRs, enzymes and signaling molecules.

Several viruses evolved proteins to target the above-described cellular gene transcription machinery in order to overcome the cellular antiviral defense system. There are several different viral strategies to overcome the cellular antiviral defense system. For example, pox- and herpesviruses express a number of open reading frames (ORFs) acting in parallel to diminish the interferon response at various steps. In contrast, small RNA viruses with limited genetic coding capacity preferentially perturb the antiviral system at critical hubs. The Npro protein encoded by the positive strand RNA virus bovine viral diarrhea virus (BVDV), for instance, specifically leads to proteasomal degradation of IRF3 and thus alleviates induction of IFN-a/b (Chen et al., 2007; Hilton et al., 2006). Rift valley fever virus (RVFV), a negative strand RNA virus belonging to the virus family of *Bunyaviridae,* targets polymerase-II subunit TFIIH through its non-structural protein small (NSs) (Le May et al., 2004). Infection with wt RVFV or transfection of NSs results in general inhibition of transcription, followed by broad inhibition of protein expression and detrimental effects on cell viability. NSs recruits a SCF (Skp1, cullin, F-Box) ligase complex containing FBXO3 as E3 ligase, which leads to proteasomal degradation of TFIIH. Interfering with FBXO3 expression rescues TFIIH and alleviates antiviral effects imposed by NSs (Kainulainen et al., 2014).

A closely related *Orthomyxovirus,* thogoto virus (THOV) also targets the polymerase complex. THOV is the prototype of tick-transmitted orthomyxoviruses. Its genome consists of six single-stranded RNA (ssRNA) segments of negative polarity, each coding for a structural protein. Members of the genus THOV are structurally and genetically similar to influenza viruses. Dhori virus, a member of the genus THOV, induces disease and cytokine response patterns upon infection of mice which are similar to those of highly pathogenic influenza virus infections in humans. In contrast to its close relative influenza virus, which is usually restricted to the respiratory system, THOV as a tick-transmitted virus is systemically distributed.

THOV was shown to be sensitive to the IFN-induced host protein Mx1, which acts against orthomyxoviruses by blocking primary transcription of the viral RNA genome in the nucleus of infected cells. Consequently, Mx1-deficient mice are more susceptible to infection with THOV than Mx1-competent animals. Like many viruses such as VACV, influenza viruses A and B, herpes simplex virus I, HIV, and Epstein-Barr virus, THOV evolved an evasion strategy to prevent induction of IFNs and/or IFNAR signaling. The THOV sixth segment encodes two different transcripts: a spliced transcript that is translated into the matrix (M) protein and an unspliced transcript coding for an elongated form of M called ML. ML was shown to suppress the induction of type I IFN *in vitro* by blocking the action of IFN-regulatory factors and by directly interacting with general transcription factor TFIIB (Vogt et al., 2008). As mentioned, TFIIB is a central part of the RNA polymerase II PIC. TFIIB binds to the TATA-binding protein (TBP) subunit of the transcription factor IID, which initially recognizes the promoter sequence, and the RBP1 subunit of RNA polymerase II (Bushnell et al., 2004; Tang et al., 1996). THOV ML expression does not lead to general inhibition of gene expression and plasmid-driven ML expression does not show an apparent toxicity effect. Instead it is believed that ML specifically impairs expression of type-I interferon genes through targeting the IRF3 and NF-κB (Hagmaier et al., 2003; Jennings et al., 2005; Pichlmair et al., 2004; Vogt et al., 2008).

As is evident from the above, several viral proteins that inhibit the transcription machinery of infected cells are known in the art. The present invention aims at providing novel and advantageous means and methods for inhibiting gene expression.

Accordingly, the present invention relates in a first aspect to a nucleic acid molecule encoding a polypeptide capable of binding to TFIIB and inhibiting gene expression, wherein the polypeptide (a) has the amino acid sequence of SEQ ID NO: 1; (b) is a fragment of the amino acid sequence of (a), said fragment lacking up to five amino acids, preferably up to four amino acids, more preferably up to three amino acids, even more preferably up to two amino acids and most preferably one amino acid at the C-terminus of the amino acid sequence of (a); or (c) has an amino acid sequence being at least 80% identical, preferably at least 85% identical, more preferably at least 90% identical and most preferably at least 95% identical to the amino acid sequence of (a) or (b).

In accordance with the present invention the term "nucleic acid molecule" defines a linear molecular chain consisting of preferably more than 177 nucleotides. The term "nucleic acid molecule" is interchangeably used herein with the term "polynucleotide".

The term "nucleic acid molecule" in accordance with the present invention includes DNA, such as cDNA or double or single stranded genomic DNA and RNA. In this regard, "DNA" (deoxyribonucleic acid) means any chain or sequence of the chemical building blocks adenine (A), guanine (G), cytosine (C) and thymine (T), called nucleotide bases that are linked together on a deoxyribose sugar backbone. DNA can have one strand of nucleotide bases, or two complimentary strands which may form a double helix structure. "RNA" (ribonucleic acid) means any chain or sequence of the chemical building blocks adenine (A), guanine (G), cytosine (C) and uracil (U), called nucleotide bases that are linked together on a ribose sugar backbone. RNA typically has one strand of nucleotide bases. Included are also single-and double-stranded hybrid molecules, i.e., DNA-DNA, DNA-RNA and RNA-RNA. The nucleic acid molecule may also be modified by many means known in the art. Non-limiting examples of such modifications include methylation, "caps", substitution of one or more of the naturally occurring nucleotides with an analog, and internucleotide modifications such as, for example, those with uncharged linkages (e.g., methyl phosphonates, phosphotriesters, phosphoroamidates, carbamates, etc.) and with charged linkages (e.g., phosphorothioates, phosphorodithioates, etc.). Polynucleotides may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), and alkylators. The polynucleotides may be derivatized by formation of a methyl or ethyl phosphotriester or an alkyl phosphorarnidate linkage. Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA), peptide nucleic acid (PNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. Also included are nucleic acids containing modified bases, for example thio-uracil, thio-guanine and fluoro-uracil. A nucleic acid molecule typically carries genetic information, including the information used by cellular machinery to make proteins and/or polypeptides. The nucleic acid molecule of the invention may additionally comprise promoters, enhancers, response elements, signal sequences, polyadenylation sequences, introns, 5'- and 3'- noncoding regions, and the like.

The term "polypeptide" as used herein interchangeably with the term "protein" describes linear molecular chains of amino acids, including single chain proteins, preferably containing 59 or more amino acids.

Polypeptides may further form oligomers consisting of at least two identical or different molecules. The corresponding higher order structures of such multimers are, correspondingly, termed homo- or heterodimers, homo- or heterotrimers etc.. The polypeptides of the invention may form heteromultimers or homomultimers, such as heterodimers or homodimers. Furthermore, peptidomimetics of such proteins/polypeptides where amino acid(s) and/or peptide bond(s) have been replaced by functional analogues are also encompassed by the invention. Such functional analogues include all known amino acids other than the 20 gene-encoded amino acids, such as selenocysteine. The terms "polypeptide" and "protein" also refer to naturally modified polypeptides and proteins where the modification is affected e.g. by glycosylation, acetylation, phosphorylation, ubiqitinylation and similar modifications which are well known in the art.

The term "a polypeptide capable of binding to TFIIB and inhibiting gene expression" as used herein means a polypeptide which reduces the transcription of at least one gene, wherein the transcription of this at least one gene is requires the transcription factor II B. Transcription is the first step of gene expression, in which a particular segment of DNA is copied into RNA (mostly mRNA) by the enzyme RNA polymerase. In this respect the inhibition of the gene expression of the at least one gene means with increasing preference that gene expression is reduced by at least 20%, at least 30%, at least 40%, at least 50%, at least 60%, at least 70%, at least 80%, at least 90%, at least 95% and most preferably completely abolished in the presence of the polypeptide of the invention as compared to corresponding conditions in the absence of the polypeptide of the invention. Means and method for the quantification of genes expression are known to the skilled person. A non-limiting and preferred example is real-time quantitative PCR (RT-qPCR) being well-established approach for measuring mRNA abundance and thereby gene expression. In this technique, reverse transcription is followed by quantitative PCR. Reverse transcription first generates a DNA template from the mRNA; this single-stranded template is called cDNA. The cDNA template is then amplified in the quantitative step, during which the fluorescence emitted by labeled hybridization probes or intercalating dyes changes as the DNA amplification process progresses. With a constructed standard curve, qPCR can produce an absolute measurement of the number of copies of original mRNA, typically in units of copies per nanolitre of homogenized tissue or copies per cell.

The polypeptide of SEQ ID NO: 1 corresponds to amino acid positions 225 to 304 of the ML protein of THOV as shown in SEQ ID NO: 10, noting that SEQ ID NO: 10 has a length of 304 amino acids. The fragments of the polypeptide of SEQ ID NO: 1 lacking one, two, three, four or five amino acids at the C-terminus consequently correspond to amino acid positions 225 to 303, 225 to 302, 225 to 301, 225 to 300 and 225 to 299 of the ML protein, respectively. The cDNA of the ML protein of THOV is shown in SEQ ID NO: 11.

In accordance with the present invention, the term "percent (%) sequence identity" describes the number of matches ("hits") of identical nucleotides/amino acids of two or more aligned nucleic acid or amino acid sequences as compared to the number of nucleotides or amino acid residues making up the overall length of the template nucleic acid or amino acid sequences. In other terms, using an alignment, for two or more sequences or subsequences the percentage of amino acid residues or nucleotides that are the same (e.g. 80% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of any sequence to be aligned. Amino acid sequence analysis and alignment in connection with the present invention is preferably carried out using the NCBI BLAST algorithm (Stephen F. Altschul, Thomas L. Madden, Alejandro A. Schaffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402). The skilled person is aware of additional suitable programs to align nucleic acid sequences.

As defined in the embodiments herein above, certain amino acid sequence identities are envisaged by the invention. In more detail, envisaged by the invention are with increasing preference amino acid sequence identities of at least 80%, at least 85%, at least 90%, and at least 95% identity.

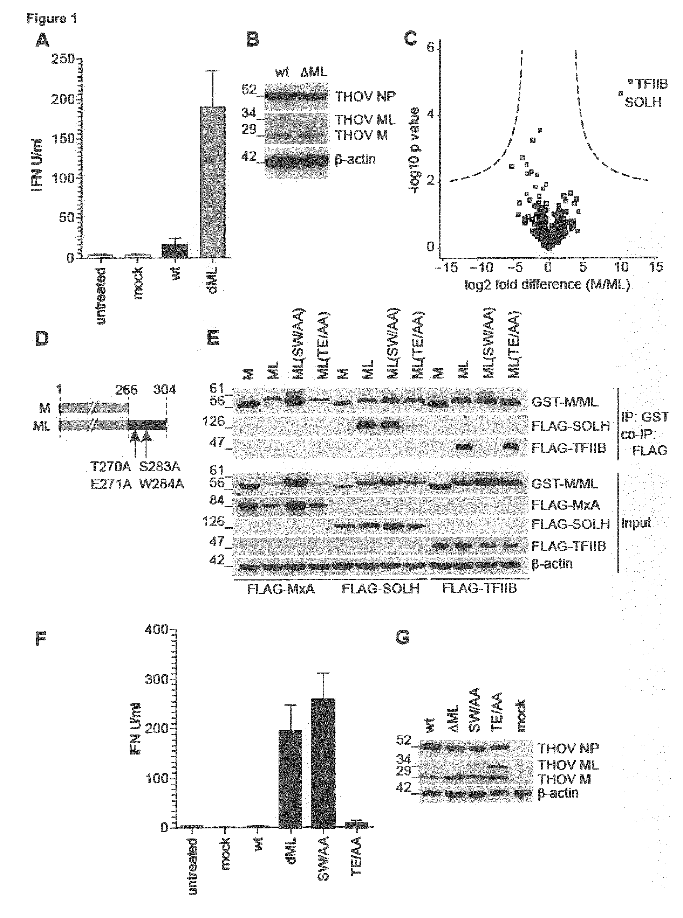

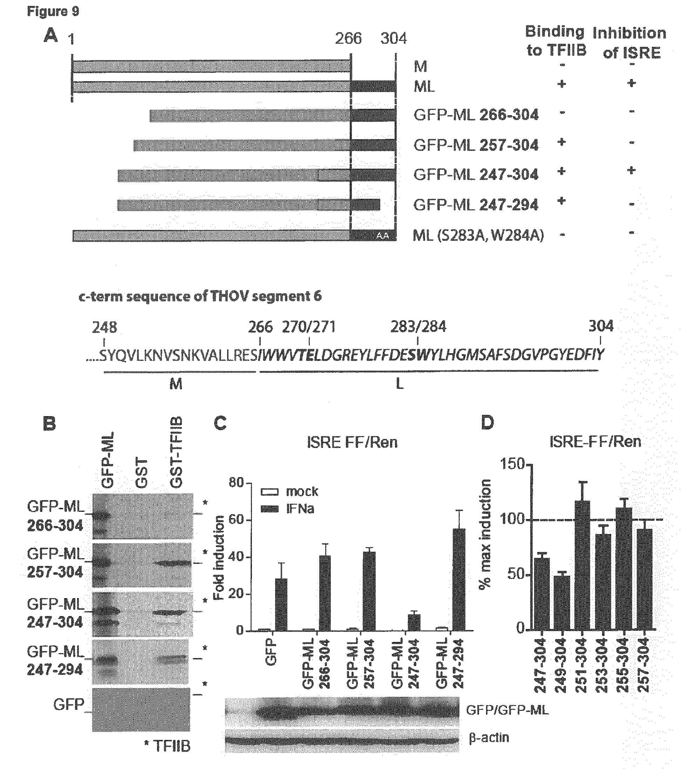

As it is evident from the data shown in the examples herein below, in connection with the present invention the part of the ML protein of THOV was identified which is essential for binding to TFIIB and inhibiting gene expression. In more detail, the M protein of THOV consists of 266 amino acids while the ML protein consists of 304 amino acids. As shown in Figure 1, the fragment of ML consisting of the amino acids not found in M (i.e. amino acids (aa) 266-304) neither binds to TFIIB nor inhibits gene expression (as exemplified for gene expression via the Interferon-Stimulated Response Element (ISRE) promoter). In the above-discussed publication Vogt et al., 2008 it was postulated that the TFIIB-binding site on ML is confined to aa 257 to 294 of ML. It was, though, surprisingly found in connection with the present invention that although the ML fragment of aa 257 to 294 is capable of binding to TFIIB this fragment fails to inhibit gene expression (Fig. 9B, C). Also the three longer fragments of aa 255 to 304, aa 253 to 304 and aa 251 to 304 were all found not to inhibit gene expression (Figures 9B). As shown, for example, in Figure 9 it was surprisingly found in connection with the present invention that ML fragments of aa 249 to 304 and aa 247 to 304 both bind to TFIIB and inhibit gene expression. In addition, it was also revealed that the ML fragment of aa 247 to 294 binds to TFIIB but does not inhibit gene expression.

In summary, these data demonstrate that the C-terminal fragment of ML comprising at least aa 249 to 304 of the full-length ML protein is sufficient in order to bind to TFIIB and inhibit gene expression. Deleting further amino acids at the N-terminus of the ML fragment of aa 249 to 304 results in abolishing the gene expression inhibitory activity of ML. Also the deletion of 10 amino acids at the C-terminus of this fragment results in abolishing the gene expression inhibitory activity of ML. It is furthermore shown in the examples herein below that amino acids in the ML protein comprising at least aa 247 to 304 may be changed without interfering with the TFIIB binding and the gene expression inhibitory activity of ML. In more detail, with the TFIIB binding site aa 270 and aa 271 may both be replaced by alanine. Such derivatized ML protein is still capable of binding to TFIIB and inhibiting gene expression.

Hence, the first aspect of the present invention is directed to a nucleic acid molecule encoding a short ML fragment which retains the capabilities of the full-length ML of binding to TFIIB and inhibiting gene expression. This fragment is considerably shorter than the full-length ML with 304 amino acids. For instance, SEQ ID NO: 1 has 80 amino acids and is thus more about three times shorter as compared to the full-length ML. A short and functionally active ML fragment is technically advantageous for several reasons. First, the short ML fragment is an ideal fusion partner. It can be fused, for example, to a fusion partner which increases the solubility or half-life time of the ML fragment. The ML fragment may also be fused to a fusion partner with a different functionality, for example, a fusion partner which binds to another component of the PIC, or which fusion partner targets a particular tissue/cell type or cellular compartment. The short and functionally active ML fragment can moreover be expected to have a reduced immunogenicity as compared to the full-length ML, because the short fragment comprises significantly less antigenic sites as compared to the full-length ML. Due to the short amino acid length also high expression yields in bacteria can be expected in case the ML fragment is to be produced recombinantly. The small size can also be expected to provide better tissue penetration and local delivery as compared to the full-length ML.

In accordance with a preferred embodiment of the first aspect of the present invention the polypeptide of (a) has the amino acid sequence of SEQ ID NO: 2, and preferably of SEQ ID NO: 3.

SEQ ID NO: 2 consists of aa 235-304 of ML and SEQ ID NO: 3 consists of aa 245-304 of ML. These ML fragments are preferred over the ML fragment of SEQ ID NO: 1 because they are shorter but still comprise the C-terminal part of ML which confers binding to TFIIB and inhibition of gene expression. Also encompassed by this preferred embodiment of the first aspect of the present invention is a nucleic acid molecule encoding a polypeptide capable of binding to TFIIB and inhibiting gene expression, wherein the polypeptide (i) is a fragment of SEQ ID NO: 2 or 3, said fragment lacking up to five amino acids, preferably up to four amino acids, more preferably up to three amino acids, even more preferably up to two amino acids and most preferably one amino acid at the C-terminus of the amino acid sequence of SEQ ID NO: 2 or 3; or (ii) has an amino acid sequence being at least 80% identical, preferably at least 85% identical, more preferably at least 90% identical and most preferably at least 95% identical to the amino acid sequence of SEQ ID NO: 2 or 3 including fragments of SEQ ID NO: 2 or 3 as defined in (i).

In accordance with another preferred embodiment of the first aspect of the present invention the polypeptide of (a) is selected from the group consisting of SEQ ID NOs 4 to 9.

SEQ ID NOs 4, 5 and 6 are the ML fragments consisting of aa 247-304, aa 248-304 and aa 249-304, respectively of the full-length ML. SEQ ID NOs 7, 8 and 9 are the ML fragments consisting of aa 247-304, aa 248-304 and aa 249-304, respectively of the full-length ML, wherein in three fragments the amino acid T (threonine) in aa position 270 of the ML protein and the amino acid E (glutamate) in aa position 271 of the ML protein are both replaced by A (alanine). As discussed herein above, the examples section evidences for each ML fragments of SEQ ID NOs 4 to 9 their capability of binding to TFIIB and inhibiting gene expression although these fragments consist of only between 55 to 57 amino acids. This is around 1/5 of the size of the full-length ML protein. Also encompassed by this preferred embodiment of the first aspect of the present invention is a nucleic acid molecule encoding a polypeptide capable of binding to TFIIB and inhibiting gene expression, wherein the polypeptide (i) is a fragment of any one of SEQ ID NOs 4 to 9, said fragment lacking up to five amino acids, preferably up to four amino acids, more preferably up to three amino acids, even more preferably up to two amino acids and most preferably one amino acid at the C-terminus of the amino acid sequence of any one of SEQ ID NOs 4 to 9; or (ii) has an amino acid sequence being at least 80% identical, preferably at least 85% identical, more preferably at least 90% identical and most preferably at least 95% identical to an amino acid sequence selected from SEQ ID NOs 4 to 9 including fragments of SEQ ID NOs 4 to 9 as defined in (i).

In a second aspect the present invention relates to a fusion construct comprising the nucleic acid molecule of the invention fused with a heterologous polynucleotide.

A "fusion construct" used herein defines the fusion of the nucleic acid of the invention to a heterologous polynucleotide. The term "heterologous" requires in this respect that the fusion partner is not a polynucleotide encoding the ML protein or a part of the ML protein. In this connection the term "part of the ML protein" preferably means 20 or less, preferably 10 or less and most preferably 5 or less consecutive amino acids of the ML protein. The heterologous polynucleotide may either be fused to the 3' and/or the 5' end of the nucleic acid molecule of the invention. The heterologous polynucleotide may be directly fused to the nucleic acid molecule of the invention or via a linker. The heterologous polynucleotide and the linker preferably each encode an amino acid sequence. In case such amino acid sequences are encoded the fusion construct encodes a fusion protein as will be defined in more detail herein below. Also preferred examples of heterologous polynucleotide and amino acid linkers will be described herein below. It is to be understood that also the linker is not the ML protein or a part of the ML protein as defined herein above.

In a third aspect the present invention relates to a vector comprising the nucleic acid molecule of the invention or the fusion construct of the invention.

The term "vector" in accordance with the invention means a plasmid, cosmid, virus, bacteriophage or another vector used e.g. conventionally in genetic engineering which carries the nucleic acid molecule of the invention or the fusion construct of the invention. Accordingly, the nucleic acid molecule or the fusion construct of the invention may be inserted into several commercially available vectors. Non-limiting examples include prokaryotic plasmid vectors, such as of the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for yeast, in particular *Pichia pastoris* comprise e.g. the plasmids pAO815, pPIC9K and pPIC3.5K (all Intvitrogen).

The nucleic acid molecule of the present invention referred to above may also be inserted into vectors such that a translational fusion with another nucleic acid molecule is generated thereby arriving at the fusion construct of the invention. To this aim, overlap extension PCR can be applied (e.g. Wurch, T., Lestienne, F., and Pauwels, P.J., A modified overlap extension PCR method to create chimeric genes in the absence of restriction enzymes, Biotechn. Techn. 12, 9, Sept. 1998, 653-657). The other nucleic acid molecules may encode a protein which may e.g. increase the solubility and/or facilitate the purification of the protein encoded by the nucleic acid molecule of the invention. Non-limiting examples include pET32, pET41, pET43. The vectors may also contain an additional expressible nucleic acid coding for one or more chaperones to facilitate correct protein folding. Suitable bacterial expression hosts comprise e.g. strains derived from BL21 (such as BL21(DE3), BL21(DE3)PlysS, BL21(DE3)RIL, BL21 (DE3)PRARE) or Rosetta®.

Particularly preferred plasmids which can be used to introduce the nucleic acid or fusion construct of the invention into a host cell are: pUC18/19 (Roche Biochemicals), pKK-177-3H (Roche Biochemicals), pBTac2 (Roche Biochemicals), pKK223-3 (Amersham Pharmacia Biotech), pKK-233-3 (Stratagene) and pET (Novagen). Further suitable plasmids are listed in PCT/EP03/07148. Particular preference is given to an expression system which is based on plasmids belonging to the pET series.

For vector modification techniques, see Sambrook and Russel, 2001. Generally, vectors can contain one or more origins of replication (ori) and inheritance systems for cloning or expression, one or more markers for selection in the host, e.g., antibiotic resistance, and one or more expression cassettes. Suitable origins of replication include, for example, the Col E1, the SV40 viral and the M13 origins of replication.

The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of the transcription (e. g., translation initiation codon, transcriptional termination sequences, promoters, enhancers, and/or insulators), internal ribosomal entry sites (IRES) (Owens *et al.,* 2001 ) and optionally poly-A signals ensuring termination of transcription and stabilization of the transcript. Additional regulatory elements may include transcriptional as well as translational enhancers, and/or naturally-associated or heterologous promoter regions. Preferably, the nucleic acid molecule of the invention is operably linked to such expression control sequences allowing expression in prokaryotes or eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the nucleic acid molecule of the invention. Such leader sequences are well known in the art. Specifically designed vectors allow the shuttling of DNA between different hosts, such as bacteria-fungal cells or bacteria-animal cells.

The co-transfection with a selectable marker such as kanamycin or ampicillin resistance genes for culturing in *E*. *coli* and other bacteria allows the identification and isolation of the transfected cells. Selectable markers for mammalian cell culture are the dhfr, gpt, neomycin, hygromycin resistance genes. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al. 1991; Bebbington et al. 1992).

Using such markers, the cells are grown in selective medium and the cells with the highest resistance are selected.

In a fourth aspect the present invention relates to a cell comprising the nucleic acid molecule of the invention, the fusion construct of the invention, the vector of the invention or a combination thereof.

The "cell" in accordance with the invention may be produced by introducing the nucleic acid molecule, fusion construct or vector(s) of the invention into the cell which upon its/their presence mediates the expression of the nucleic acid molecule of the invention encoding the polypeptide of the invention. To this end, the cell may be transformed, transduced or transfected with the nucleic acid molecule of the invention, the fusion construct of the invention, the vector of the invention or a combination thereof. Such methods are well established in the art.

A variety of host-expression systems may be used in accordance with the invention. The host from which the host cell is derived may be any prokaryote or eukaryotic cell. A suitable eukaryotic host cell may, for example, be a vertebrate cell, an amphibian cell, a fish cell, an insect cell, a fungal/yeast cell, a nematode cell or a plant cell. The insect cell may be a *Spodoptera frugiperda* cell, a Drosophila S2 cell or a Spodoptera Sf9 cell, the fungal/yeast cell may a *Saccharomyces cerevisiae* cell, *Pichia pastoris* cell or an *Aspergillus* cell. It is preferred that the vertebrate cell is a mammalian cell such as a human cell, CHO, COS, 293 or Bowes melanoma cell. The plant cell is preferably selected independently from a cell of *Anacardium, Anona, Arachis, Artocarpus, Asparagus, Atropa, Avena, Brassica, Carica, Citrus, Citrullus, Capsicum, Carthamus, Cocos, Coffea, Cucumis, Cucurbita, Daucus, Elaeis, Fragaria, Glycine, Gossypium, Helianthus, Heterocallis, Hordeum, Hyoseyamus, Lactuca, Linum, Lolium, Lupinus, Lycopersicon, Malus, Manihot, Majorana, Medicago, Nicotiana, Olea, Oryza, Panieum, Pannesetum, Passiflora, Persea, Phaseolus, Pistachia, Pisum, Pyrus, Prunus, Psidium, Raphanus, Ricinus, Secale, Senecio, Sinapis, Solanum, Sorghum, Theobromus, Trigonella, Triticum, Vicia, Vitis, Vigna* and Zea. The cell may be a part of a cell line. The cell from a plant may, e.g., be derived from root, leave, bark, needle, bole or caulis.

Suitable prokaryotes (bacteria) useful as hosts for the invention are those generally used for cloning and/or expression like *E. coli* (e.g., *E coli* strains BL21, HB101, DH5a, XL1 Blue, Y1090 and JM101), *Salmonella typhimurium, Serratia marcescens, Burkholderia glumae, Pseudomonas putida, Pseudomonas fluorescens*, *Pseudomonas stutzeri, Streptomyces lividans*, *Lactococcus lactis*, *Mycobacterium smegmatis, Streptomyces* or *Bacillus subtilis.* Appropriate culture mediums and conditions for the above-described host cells are known in the art.

In a fifth aspect the present invention relates to a method of producing a polypeptide capable of binding to TFIIB and inhibiting gene expression, wherein the method comprises (a) culturing the cell of the invention, and (b) isolating the produced polypeptide capable of binding to TFIIB and inhibiting gene expression.

Suitable conditions for culturing a prokaryotic or eukaryotic host are well known to the person skilled in the art. For example, suitable conditions for culturing bacteria are growing them under aeration in Luria Bertani (LB) medium. To increase the yield and the solubility of the expression product, the medium can be buffered or supplemented with suitable additives known to enhance or facilitate both. For example, *E. coli* can be cultured from 4 to about 37 °C, the exact temperature or sequence of temperatures depending on the molecule to be overexpressed. In general, *Aspergillus sp.* may be grown on Sabouraud dextrose agar, or potato dextrose agar at about 10°C to about 40°C, and preferably at about 25°C. Suitable conditions for yeast cultures are known, for example from Guthrie and Fink, "Guide to Yeast Genetics and Molecular Cell Biology" (2002); Academic Pr Inc.. The skilled person is also aware of all these conditions and may further adapt these conditions to the needs of a particular host species and the requirements of the polypeptide expressed. In case an inducible promoter controls the nucleic acid of the invention in the vector present in the host cell, expression of the polypeptide can be induced by addition of an appropriate inducing agent. Suitable expression protocols and strategies are known in the art. Depending on the cell type and its specific requirements, mammalian cell culture can e.g. be carried out in RPMI or DMEM medium containing 10% (v/v) FCS, 2mM L-glutamine and 100 U/ml penicillin/streptomycin. The cells can be kept at 37 °C in a 5% CO₂, water saturated atmosphere. Suitable expression protocols for eukaryotic cells are well known and can be retrieved e.g. from Sambrook, 2001.

Methods of isolation of the polypeptide produced are well-known in the art and comprise without limitation method steps such as ion exchange chromatography, gel filtration chromatography (size exclusion chromatography), affinity chromatography, high pressure liquid chromatography (HPLC), reversed phase HPLC, disc gel electrophoresis or immunoprecipitation, see, for example, Sambrook, 2001.

The step of protein isolation is preferably a step of protein purification. Protein purification in accordance with the invention specifies a process or a series of processes intended to further isolate the polypeptide of the invention from a complex mixture preferably to homogeneity. Purification steps, for example, exploit differences in protein size, physico-chemical properties and binding affinity. For example, proteins may be purified according to their isoelectric points by running them through a pH graded gel or an ion exchange column. Further, proteins may be separated according to their size or molecular weight via size exclusion chromatography or by SDS-PAGE (sodium dodecyl sulfate-polyacrylamide gel electrophoresis) analysis. In the art, proteins are often purified by using 2D-PAGE and are then further analysed by peptide mass fingerprinting to establish the protein identity. This is very useful because the detection limits for protein are very low and nanogram amounts of protein are in general sufficient for their analysis. Proteins may also be separated by polarity/hydrophobicity via high performance liquid chromatography or reversed-phase chromatography. Thus, methods for protein purification are well known to the skilled person and some are exemplified in the examples of the invention.

In a sixth aspect the present invention relates to a polypeptide capable of binding to TFIIB and inhibiting gene expression encoded by the nucleic acid molecule of the invention, the fusion construct of the invention, the vector of the invention or produced by the above described method of the invention.

In a preferred embodiment, the polypeptide of the invention is a fusion construct. In connection with the polypeptide of the invention it is to be understood that the term "fusion construct" defines the fusion of the polypeptide of the invention to a further compound. The compound may either be a proteinous compound or a non-proteinous compound. In the case the compound is a proteinous compound (e.g. a cytokine or chemokine as described herein below), the fusion construct may also be designated as fusion protein. The term "fusion protein" as used herein is in general terms directed to a polypeptide construct generated through the joining and expression of two or more nucleotide sequences which code for separate polypeptides. In other words, translation of this fusion gene results in a single polypeptide with functional properties derived from each of the original polypeptides. In general, fusion proteins are generated artificially by recombinant DNA technology well know to the skilled person (e.g. Alberts et al., Molecular Biology of the Cell, 4th ed. Garland Science, p. 518-519). However, polypeptides and fusion proteins of the invention may be prepared by any of the many conventional and well known techniques such as plain organic synthetic strategies, solid phase-assisted synthesis techniques or by commercially available automated synthesizers. Fusion proteins may be used in biological research or therapeutics. Examples of non-proteinous compounds as the further compound are, for example, small organic molecules, such as polyethylenglycol (PEG) or Alexa Fluor, or radionuclides; see also the discussion below. Further specific examples of non-proteinous further compounds are discussed herein below.

The further compounds may be directly fused to the polypeptide of the invention or indirectly, via a linker. Suitable linkers are at the skilled person's disposal. The linker according to the invention is preferably selected from the group consisting of alkyl with 1 to 30 carbon atoms, polyethylene glycol with 1 to 20 ethylene moieties, polyalanine with 1 to 20 residues, caproic acid, substituted or unsubstituted poly-p-phenylene and triazol. Preference is also given to peptidic linkers, more specifically to oligopeptides having a length from 2 to 30 amino acids. Use of a single amino acid is also deliberately envisaged. Preferred length ranges are from 5 to 15 amino acids. Other preferred lengths are 3, 4, 6, 7, 8, 9, 10, 11, 12, 13, 14, 16, 17, 18, 19 or 20 amino acids. Particularly preferred are linkers which are peptides which consist of at least 50%, at least 60%, at least 70%, at least 80%, at least 90% or 100% of small amino acids such as glycine, serine and alanine. Particularly preferred are linkers consisting of glycines and serines only.

In accordance with a preferred embodiment the further compound is a pharmaceutically active compound, a prodrug, a pharmaceutically-acceptable carrier, a diagnostically active compound, a cell penetrating enhancer, and/or a compound modulating serum half-life.

A pharmaceutically active compound is a compound having a biologically activity upon administration to a subject, which brings about a beneficial effect for the subject. As described herein below in further detail, the polypeptide of the invention is pharmaceutically active. Hence, by fusing another pharmaceutically active compound to the polypeptide of the invention either the inhibitory pharmaceutical activity of polypeptide of the invention may be enhanced or a second pharmaceutical activity may be conferred to the polypeptide of the invention. In the first case a further inhibitor of the PIC may, for example, be used as fusion partner, such as an inhibitor of RNAPII, or one or more of the GTFs TFIIA, TFIID, TFIIE, TFIIF, TFIIH and Mediator. For example, antibodies to these constituents of the PIC are commercially available and may be used. Moreover, several chemical inhibitors of the PIC are known and may be fused to the polypeptide of the invention. Non-limiting examples are α-amanitin, adriamycin, daunorubicin, mythramycin, heparin and rifamycin. By using a fusion partner conferring a second pharmaceutical activity, for example, bispecific fusion constructs can be designed being particularly suitable for the treatment of a selected disease. For example, in case a hyperproliferative disease is to be treated the fusion partner may be a cytostatic compound. Various cytostatic drugs are available in the art, and include but are not limited to alkylating agents, nitrosourea, antimetabolites or mitotic inhibitors. If viral diseases were to be treated the fusion partner may be a vaccine. A vaccine typically contains an agent that resembles the disease-causing virus and is often made from weakened or killed forms of the virus, its toxins or one of its surface proteins. In case the disease to be treated is an inflammatory disease the fusion partner may be an anti-inflammatory compound, such as, for example, an anti-inflammatory cytokine (e.g. IL-4, IL-10, IL-13 and IFN-alpha) or an anti-inflammatory chemical compounds (e.g. aspirin, diclofenac or ibuprofen). A prodrug is a compound that is administered in an inactive (or less than fully active) form to a subject, and is subsequently converted to a pharmaceutically active or pharmaceutically fully active compound through metabolic processes in the subject. The use of prodrugs is a well-established tool for improving, for example, how a medicine is absorbed, distributed, metabolized, and excreted within the patient to be treated. Prodrugs are generally classified into class I and class II. Type I prodrugs are bioactivated inside the cells (intracellularly). Examples of type I prodrugs are anti-viral nucleoside analogs that must be phosphorylated and the lipid-lowering statins. Lipid-lowering statins are, for example, of particular interest for the treatment of hyperproliferatve diseases. Type II prodrugs are bioactivated outside cells (extracellularly), for example, in digestive fluids or in the body's circulatory system, particularly in the blood. Non-limiting examples of Type II prodrugs are salicin and certain antibody-, gene- or virus-directed enzyme prodrugs used. Type II prodrugs may, for example, be used for the treatment of localized immune disorders, such that ulcerative colitis which requires activity in the digestive tract.

The pharmaceutically (fully) active compound is preferably a compound suitable for the treatment of prevention of any of the specific diseases defined herein below.

A diagnostically active compound is a compound having an activity upon administration to a subject, which allows determining or identifying a possible disease or disorder. Examples of diagnostically active compounds include detectable markers such as fluorescent dyes, radionuclides or contrast agents for medical imaging. Specific examples of fluorescent dyes, radionuclides and contrast agents for medical imaging are described herein below. A diagnostically active compound fused to a polypeptide of the invention can in particular be used to determine or identify any one of the specific diseases defined herein below which have in common that in the course of said diseases previously inactive or only moderately active genes become activated and this activation *inter alia* requires *de novo* recruitment of TFIIB to the promoter sites of these genes. The sides of such disease can be detected or identified by the polypeptide of the invention fused to the diagnostically active compound of the invention, because the polypeptide of the invention binds to TFIIB and the diagnostically active compound allows for identifying the binding sites.

A cell penetrating enhancer is a compound facilitating the delivery of the polypeptide of the invention into a (*in vitro, ex vivo* or *in vivo*) cell. For example, several cell-penetrating peptides are known in the art. The cell-penetrating peptides (generally short peptides with 5 to 30 amino acids) is particularly advantageous for use in vaccine systems and hence for treatment of viral infections. This is because viruses are infectious agents that only replicate inside living cells of an organism.

A compound modulating serum half-life is a compound which allows for either extending or shortening the *in vivo* half-life of the polypeptides of the invention, in particular in the blood circulation system. The component extending serum half-life is preferably polyethylene glycol (PEG). The component shortening serum half-life is preferably ubiquitin. In practice, it is mostly desired to extend the *in vivo* half-life of a polypeptide in order to achieve optimal exposure to and targeting of the disease to be treated. The serum half-life needs to be sufficiently long to ensure that the polypeptide of the invention reaches the diseased sites in the body and comes into contact with the diseased sites for a time sufficient to alleviate the disease. This is of particular importance for systemic application modes, such as intravenous infusion or injection.

A pharmaceutically-acceptable carrier is a compound that improves the delivery and/or the effectiveness of the polypeptide of the invention upon administration to a subject. Suitable pharmaceutically-acceptable carriers are well known in the art and include, for example, stabilizers, antioxidants, pH-regulating substances, etc. The pharmaceutically-acceptable carrier may, for example, render the polypeptide of the invention more suitable for a particular application mode, such as transdermal administration. In the case of transdermal administration skin penetration enhancer may be used. Known skin penetration enhancers includes but are not limited to sulphoxides (such as dimethylsulphoxide, DMSO), azones (e.g. laurocapram), pyrrolidones (e.g. 2-pyrrolidone), alcohols and alkanols (e.g. ethanol, or decanol), glycols (e.g. propylene glycol), surfactants and terpenes.

Compounds of the invention selected from the group consisting of (a) a fluorescent dye, (b) a photosensitizer, (c) a radionuclide, (d) a contrast agent for medical imaging, (e) a cytokine, (f) a toxic compound, (g) a chemokine, (h) a pro-coagulant factor, (i) an enzyme for pro-drug activation, (j) an albumin binder, (k) an albumin, (I) an IgG binder, (m) polyethylene glycol, or (n) an affinity and solubility-enhancing polypeptide "tag" are preferred embodiments of the invention.

The fluorescent dye is preferably a component selected from Alexa Fluor or Cy dyes.

The photosensitizer is preferably phototoxic red fluorescent protein KillerRed or haematoporphyrin.

The radionuclide is preferably either selected from the group of gamma-emitting isotopes (such as and preferably ^{99m}Tc, ¹²³I, ¹¹¹In), from the group of positron emitters (such as and preferably ¹⁸F, ⁶⁴Cu, ¹⁸Ga, ⁸⁶Y, ¹²⁴I) from the group of beta-emitter ( such as and preferably ¹³¹I, ⁹⁰Y, ¹⁷⁷Lu, ⁶⁷Cu), and/or from the group of alpha-emitter(such as and preferably ²¹³Bi, ²¹¹At).

A contrast agent as used herein is a substance used to enhance the contrast of structures or fluids within the body in medical imaging. Common contrast agents work based on X-ray attenuation and magnetic resonance signal enhancement.

The above-described fluorescent dyes, a photosensitizers, radionuclides, and contrast agents for medical imaging are non-limiting examples of diagnostically active compounds as defined herein above. These compounds may of course also serve a further or an alternative purpose.

The cytokine is preferably selected from the group consisting of IL-2, IL-12, TNF-alpha, IFN alpha, IFN beta, IFN gamma, IL-10, IL-15, IL-24, GM-CSF, IL-3, IL-4, IL-5, IL-6, IL-7, IL-9, IL-11, IL-13, LIF, CD80, B70, TNF beta, LT-beta, CD-40 ligand, Fas-ligand, TGF-beta, IL-1 alpha and IL-1 beta. As it is well-known in the art, cytokines may favour a pro-inflammatory or an anti-inflammatory response of the immune system. Thus, depending on the disease to be treated either fusion constructs with a pro-inflammatory or an anti-inflammatory cytokine may be favoured. For example, for the treatment of inflammatory diseases in general fusion constructs comprising anti-inflammatory cytokines are preferred, whereas for the treatment of a hyperproliferative disease in general fusion constructs comprising pro-inflammatory cytokines are preferred.

The toxic compound is preferably a small organic compound or a polypeptide, more preferably a toxic compound selected from the group consisting of calicheamicin, maytansinoid, neocarzinostatin, esperamicin, dynemicin, kedarcidin, maduropeptin, doxorubicin, daunorubicin, auristatin, Ricin-A chain, modeccin, truncated Pseudomonas exotoxin A, diphtheria toxin, mistletoe lectin and recombinant gelonin.

The chemokine is preferably selected from the group consisting of IL-8, GRO alpha, GRO beta, GRO gamma, ENA-78, LDGF-PBP, GCP-2, PF4, Mig, IP-10, SDF-1alpha/beta, BUNZO/STRC33, I-TAC, BLC/BCA-1, MIP-1alpha, MIP-1 beta, MDC, TECK, TARC, RANTES, HCC-1, HCC-4, DC-CK1, MIP-3 alpha, MIP-3 beta, MCP-1-5, eotaxin, Eotaxin-2, I-309, MPIF-1, 6Ckine, CTACK, MEC, lymphotactin and fractalkine.

The pro-coagulant factor is preferably the tissue factor (TF) or cancer procoagulant (CP).

The enzyme for pro-drug activation is preferably an enzyme selected from the group consisting of carboxy-peptidases, glucuronidases and glucosidases.

The above-described cytokines, toxic compounds, chemokines, pro-coagulant factors, and enzymes for pro-drug activation are non-limiting examples of pharmaceutically active compounds as defined herein above. These compounds may of course also serve a further or an alternative purpose.

Examples of an albumin binder, and an IgG binder are described in Gebauer and Skerra (2009), 13:245-255. Accordingly, preferred examples of albumin binders and an IgG binders are human single Ig domains (dubbled Albumin Dab), nanobodies, naturally occurring albumin binding domain (ABD) derived from streptococcal protein G, and a domain that binds to IgG. Such fusion constructs, for example, increase the half life of the polypeptide of the invention upon administration to a patient, in particular in the blood circulation system.

The above described albumin binder, albumin, an IgG binder, and PEG are non-limiting examples of serum half-life extending compounds as defined herein above. These compounds may of course also serve a further or an alternative purpose.

In accordance with another preferred embodiment the further compound of the invention consists of or comprises an antibody.

The term "antibody" as used in accordance with the present invention comprises, for example, polyclonal or monoclonal antibodies. Furthermore, also derivatives or fragments thereof, which still retain the binding specificity, are comprised in the term "antibody". Antibody fragments or derivatives comprise, inter alia, Fab or Fab' fragments, Fd, F(ab')₂, Fv or scFv fragments, single domain V_{H} or V-like domains, such as VhH or V-NAR-domains, as well as multimeric formats such as minibodies, diabodies, tribodies, tetrabodies or chemically conjugated Fab'-multimers (see, for example, Altshuler et *al.,* 2010., Holliger and Hudson, 2005). The term "antibody" also includes embodiments such as chimeric (human constant domain, non-human variable domain), single chain and humanized (human antibody with the exception of non-human CDRs) antibodies.

Various techniques for the production of antibodies and fragments thereof are well known in the art and described, e.g. in Altshuler et al., 2010. Thus, polyclonal antibodies can be obtained from the blood of an animal following immunisation with an antigen in mixture with additives and adjuvants and monoclonal antibodies can be produced by any technique which provides antibodies produced by continuous cell line cultures. Examples for such techniques are described, e.g. by Harlow and Lane (1988) and (1999) and include the hybridoma technique originally described by Kohler and Milstein, 1975, the trioma technique, the human B-cell hybridoma technique (see e.g. Kozbor, 1983; Li et al., 2006) and the EBV-hybridoma technique to produce human monoclonal antibodies (Cole et al., 1985). Furthermore, recombinant antibodies may be obtained from monoclonal antibodies or can be prepared *de novo* using various display methods such as phage, ribosomal, mRNA, or cell display. A suitable system for the expression of the recombinant (humanized) antibodies or fragments thereof may be selected from, for example, bacteria, yeast, insects, mammalian cell lines or transgenic animals or plants (see, e.g., US patent 6,080,560; Holliger and Hudson, 2005). Further, techniques described for the production of single chain antibodies (see, inter alia, US Patent 4,946,778) can be adapted to produce single chain antibodies specific for the target of this invention. Surface plasmon resonance as employed in the BIAcore system can be used to increase the efficiency of phage antibodies

The term "monoclonal" or "polyclonal antibody" (see Harlow and Lane, (1988), loc. cit.) also relates to derivatives of said antibodies which retain or essentially retain their binding specificity. Whereas particularly preferred embodiments of said derivatives are specified further herein below, other preferred derivatives of such antibodies are chimeric antibodies mentioned above comprising, for example, a mouse or rat variable region and a human constant region.

The term "scFv fragment" (single-chain Fv fragment) is well understood in the art. A scFv fragment is preferred due to its small size and the possibility to recombinantly produce it.

The antibody may be a humanized antibody. The term "humanized antibody" means, in accordance with the present invention, an antibody of non-human origin, whose protein sequence has been modified to increase its similarity to antibody variants produced naturally in humans. Creation of a humanized antibody may be accomplished, for example, by inserting the appropriate CDR coding segments (responsible for the desired binding properties), such as CDR 3 and preferably all 6 CDRs, into a human antibody "scaffold". Methods for the production of humanized antibodies are described in, e.g., EP-A1 0 239 400 and WO 90/07861.

A "functional Fc domain" of an antibody is a term well known to the skilled artisan and defined on the basis of papain cleavage of antibodies. Depending on the amino acid sequence of the constant region of their heavy chains, immunoglobulins are divided in the classes: IgA, IgD, IgE, IgG and IgM, and several of these may be further divided into subclasses (isotypes), e.g. IgGl, IgG2, IgG3, and IgG4, !gA1, and IgA2. According to the heavy chain constant regions the different classes of immunoglobulins are called [alpha], [delta], [epsilon], [gamma], and [mu], respectively. The functional Fc domain of an antibody is directly involved in ADCC (antibody-dependent cell-mediated cytotoxicity) and CDC (complement-dependent cytotoxicity) based on complement activation, C1q binding and Fc receptor binding. The four human IgG isotypes bind different receptors, such as the neonatal Fc receptor, the activating Fc gamma receptors, FcγRI, FcγRIIa, and FcγRIIIa, the inhibitory receptor FcγRIIb, and C1q with different affinities, yielding very different activities. It is known that the affinities to activating and inhibiting receptors of an Fc domain of a human antibody can be engineered and modified (see StrohI W. (2009) Curr Opin Biotechnol, 20, p. 685-691).

Preferably, the Fc domain is one or more human functional Fc domains which allow(s) for extending the *in vivo* half-life of the polypeptides of the invention and some of which direct a mammal's immune response to a site of specific target binding of the inventive polypeptide component of the fusion protein, e.g. in therapeutic, prophylactic and/or diagnostic applications as described herein below. More preferably such a human functional Fc domain is of an IgG1 antibody. The polypeptides of the invention can be fused either to the N- or C-terminus of one or more functional Fc domains or to both the N- and the C-terminus of one or more Fc domains.

The above described antibodies and antibody fragments may be used as further compound in the fusion construct of the invention for several purposes. For example and as mentioned, antibodies to compounds to the PIC (in particular compounds other than TFIIB) may further enhance the pharmaceutical activity of the polypeptide of the invention. Antibodies may also be functionalized with fluorescent dyes thereby conferring to them a diagnostic activity. Moreover, antibodies and antibody fragments may be used as fusion partner to increase serum-half life of a polypeptide. For example, binding to Fc receptors is known to enhance serum half life and accordingly Fc domain-engineering of polypeptides is a means for increased serum half life.

Affinity and solubility-enhancing polypeptide "tag" are widely used in the art for recombinantly producing polypeptides. In general, fusion of the N-terminus of poylpeptide to the C-terminus of a soluble fusion partner is a means for improving the solubility of the polypeptide to be produced. Several purification schemes have been developed in the art for isolating recombinantly produced polypeptides by using a tag at either terminus which binds specifically to an affinity resin. Protein G B1 domain, protein D, the Z domain of Staphylococcal protein A and thioredoxin are non-limiting examples of solubility-enhancing tags while His, GST and MBP are non-limiting examples of affinity tags.

In a seventh aspect the present invention relates to an *in vitro* method for inhibiting the expression of a gene, said method comprising contacting (i) a cell comprising the gene, or (ii) an *in vitro* expression system comprising the gene with the nucleic acid molecule of the invention, the fusion construct of the invention, the vector of the invention, the cell of the invention, the polypeptide of the invention or a combination thereof.

It is to be understood that the cell comprising the gene is capable of expressing the gene in the absence of an *in vitro* expression system comprising the nucleic acid molecule or the fusion construct of the invention, the vector of the invention, the cell of the invention, the polypeptide of the invention or a combination thereof. For instance, the claimed method may comprise further steps, wherein the cell is contacted with one or more compounds stimulating the expression of the gene (e.g. transcription factors) before the expression is inhibited by the *in vitro* expression system comprising the nucleic acid molecule or the fusion construct of the invention, the vector of the invention, the cell of the invention, the polypeptide of the invention or a combination thereof.

In accordance with a preferred embodiment of this *in vitro* method the gene requires transcription Factor II B (TFIIB).

As explained herein above, the polypeptide encoded by the nucleic acid molecule of the invention is capable of binding to TFIIB and inhibiting gene expression. For this reason in particular gene expression of genes that require transcription Factor II B (TFIIB) may be inhibited. In this respect the term "require TFIIB" means with increasing preference that in the absence of TFIIB the expression of the respective gene is at least reduced by at least 50%, at least 60%, at least 70%, at least 80%, at least 90%, at least 95% and most preferably is not detectable. Means and method for the quantification of genes expression are known to the skilled person and have been discussed herein above in more detail.

In accordance with a further preferred embodiment of this *in vitro* method the gene is under the control of one or more transcription factors selected from the group consisting of an IRF, a STAT, RELB, NFκB2, SPI1, RELA, NFκB1, an ELF and CEB-PB.

As is evident from the examples herein below, genes that are regulated through IRF (such as IRF 3 and IRF 7), STAT (such as STAT1 RELB, NFκB2, SPI1, RELA, NFκB1, an ELF and CEB-PB were identified to be inhibited by the polypeptide encoded by the nucleic acid molecule of the invention.

In accordance with another preferred embodiment of this *in vitro* method of the invention the gene is selected from Tables 1, 2 and/or 3.

Accordingly the gene may be selected from any one of Tables 1, 2 and 3, Tables 1 and 3, Tables 2 and 3, Table 1, Table 2 or Table 3. The genes listed in Tables 1 to 3 are expressed in course of certain immune responses but are silent (as evidenced by corresponding histone marks) under "steady-state conditions". In more detail, Table 1 lists genes which are *de novo* expressed in the course of an inflammatory immune response (as determined by a micro array study with sample obtained from LPS-stimulated humans). The genes in Table 2 are activated in the course of an innate immune response. Table 3 provides an overview of genes being expressed upon the activation of the mitogen-activated protein kinase (MAPK)/nuclear factor-κB (NFκB) pathway, noting that this pathway is known to be actiovatede in several immune responses. Thus, Table 3 lists genes being important in the p38, ERK, JNK pathways and the NFκB pathway.

**Table 1**

| **Gene Symbol** | **Species** | **Gene Name** |
|---|---|---|
| CD300LB | Homo sapiens | CD300 molecule-like family member b |
| C1QB | Homo sapiens | complement component 1, q subcomponent, B chain |
| CXCR5 | Homo sapiens | chemokine (C-X-C motif) receptor 5 |
| FUT3 | Homo sapiens | fucosyltransferase 3 (galactoside 3(4)-L-fucosyltransferase, Lewis blood group) |
| EBF1 | Homo sapiens | early B-cell factor 1 |
| Sh2d1a | Homo sapiens | SH2 domain protein 1A |
| tlr9 | Homo sapiens | toll-like receptor 9 |
| LAMP3 | Homo sapiens | lysosomal-associated membrane protein 3 |
| TCF7 | Homo sapiens | transcription factor 7 (T-cell specific, HMG-box) |
| IL1F6 | Homo sapiens | interleukin 1 family, member 6 (epsilon) |
| IL1F5 | Homo sapiens | interleukin 1 family, member 5 (delta) |
| IL1F10 | Homo sapiens | interleukin 1 family, member 10 (theta) |
| NFATC4 | Homo sapiens | nuclear factor of activated T-cells, cytoplasmic, calcineurin-dependent 4 |
| DARC | Homo sapiens | Duffy blood group, chemokine receptor |
| CCR2 | Homo sapiens | chemokine (C-C motif) receptor 2 |
| ITGAL | Homo sapiens | integrin, alpha L (antigen CD11A (p180), lymphocyte function-associated antigen 1; alpha polypeptide) |
| C1 QL2 | Homo sapiens | complement component 1, q subcomponent-like 2 |
| Gypa | Homo sapiens | glycophorin A (MNS blood group) |
| II31 | Homo sapiens | interleukin 31 |
| Ptprc | Homo sapiens | protein tyrosine phosphatase, receptor type, C |
| ITGAD | Homo sapiens | integrin, alpha D |
| HLA-DQB1 | Homo sapiens | major histocompatibility complex, class II, DQ beta 1; similar to major histocompatibility complex, class II, DQ beta 1 |
| LOC100133583 | Homo sapiens | major histocompatibility complex, class II, DQ beta 1; similar to major histocompatibility complex, class II, DQ beta 1 |
| C1qa | Homo sapiens | complement component 1, q subcomponent, A chain |
| IL22RA2 | Homo sapiens | interleukin 22 receptor, alpha 2 |
| CD300A | Homo sapiens | CD300a molecule |
| Tnfsf8 | Homo sapiens | tumor necrosis factor (ligand) superfamily, member 8 |
| Cd300c | Homo sapiens | CD300c molecule |
| Cytl1 | Homo sapiens | cytokine-like 1 |
| Ctla4 | Homo sapiens | cytotoxic T-lymphocyte-associated protein 4 |
| PLA2R1 | Homo sapiens | phospholipase A2 receptor 1, 180kDa |
| Xcr1 | Homo sapiens | chemokine (C motif) receptor 1 |
| STAT4 | Homo sapiens | signal transducer and activator of transcription 4 |
| C1 QL3 | Homo sapiens | complement component 1, q subcomponent-like 3 |
| Ncr1 | Homo sapiens | natural cytotoxicity triggering receptor 1 |
| PROM1 | Homo sapiens | prominin 1 |
| CCRL1 | Homo sapiens | chemokine (C-C motif) receptor-like 1 |
| CFP | Homo sapiens | complement factor properdin |
| HLA-DRB5 | Homo sapiens | major histocompatibility complex, class II, DR beta 5 |
| IGLL1 | Homo sapiens | immunoglobulin lambda-like polypeptide 1 |
| CD14 | Homo sapiens | CD14 molecule |
| FCER2 | Homo sapiens | Fc fragment of IgE, low affinity II, receptor for (CD23) |
| itga2b | Homo sapiens | integrin, alpha 2b (platelet glycoprotein IIb of IIb/IIIa complex, antigen CD41) |
| CCR9 | Homo sapiens | chemokine (C-C motif) receptor 9 |
| CCL24 | Homo sapiens | chemokine (C-C motif) ligand 24 |
| Cd3g | Homo sapiens | CD3g molecule, gamma (CD3-TCR complex) |
| CCR5 | Homo sapiens | chemokine (C-C motif) receptor 5 |
| KDR | Homo sapiens | kinase insert domain receptor (a type III receptor tyrosine kinase) |
| Cd8a | Homo sapiens | CD8a molecule |
| HLA-DOA | Homo sapiens | major histocompatibility complex, class II, DO alpha |
| Pglyrp1 | Homo sapiens | peptidoglycan recognition protein 1 |
| Cd79b | Homo sapiens | CD79b molecule, immunoglobulin-associated beta |
| Cr1I | Homo sapiens | complement component (3b/4b) receptor 1-like |
| IL1RN | Homo sapiens | interleukin 1 receptor antagonist |
| II1f9 | Homo sapiens | interleukin 1 family, member 9 |
| CEACAM1 | Homo sapiens | carcinoembryonic antigen-related cell adhesion molecule 1 (biliary glycoprotein) |
| LOC652799 | Homo sapiens | similar to Mast/stem cell growth factor receptor precursor (SCFR) (Proto-oncogene tyrosine-protein kinase Kit) (c-kit) (CD117 antigen); v-kit Hardy-Zuckerman 4 feline sarcoma viral oncogene homolog |
| LOC653882 | Homo sapiens | similar to Mast/stem cell growth factor receptor precursor (SCFR) (Proto-oncogene tyrosine-protein kinase Kit) (c-kit) (CD117 antigen); v-kit Hardy-Zuckerman 4 feline sarcoma viral oncogene homolog |
| KIT | Homo sapiens | similar to Mast/stem cell growth factor receptor precursor (SCFR) (Proto-oncogene tyrosine-protein kinase Kit) (c-kit) (CD117 antigen); v-kit Hardy-Zuckerman 4 feline sarcoma viral oncogene homolog |
| FCER1A | Homo sapiens | Fc fragment of IgE, high affinity I, receptor for; alpha polypeptide |
| IL12RB2 | Homo sapiens | interleukin 12 receptor, beta 2 |
| rac2 | Homo sapiens | ras-related C3 botulinum toxin substrate 2 (rho family, small GTP binding protein Rac2) |
| CD200R1L | Homo sapiens | CD200 receptor 1-like |
| BTLA | Homo sapiens | B and T lymphocyte associated |
| KEL | Homo sapiens | Kell blood group, metallo-endopeptidase |
| Siglec15 | Homo sapiens | sialic acid binding Ig-like lectin 15 |
| MS4A1 | Homo sapiens | membrane-spanning 4-domains, subfamily A, member 1 |
| SIGLEC5 | Homo sapiens | sialic acid binding Ig-like lectin 5 |
| Bst1 | Homo sapiens | bone marrow stromal cell antigen 1 |
| Ms4a5 | Homo sapiens | membrane-spanning 4-domains, subfamily A, member 5 |
| GP9 | Homo sapiens | glycoprotein IX (platelet) |
| IL29 | Homo sapiens | interleukin 29 (interferon, lambda 1) |
| LILRA4 | Homo sapiens | leukocyte immunoglobulin-like receptor, subfamily A (with TM domain), member 4 |
| CD40LG | Homo sapiens | CD40 ligand |
| GYPC | Homo sapiens | glycophorin C (Gerbich blood group) |
| CCL22 | Homo sapiens | chemokine (C-C motif) ligand 22 |
| GP5 | Homo sapiens | glycoprotein V (platelet) |
| IL16 | Homo sapiens | interleukin 16 (lymphocyte chemoattractant factor) |
| CLEC4M | Homo sapiens | C-type lectin domain family 4, member M |
| EPX | Homo sapiens | eosinophil peroxidase |
| Vcam1 | Homo sapiens | vascular cell adhesion molecule 1 |
| Cd247 | Homo sapiens | CD247 molecule |
| LILRA6 | Homo sapiens | leukocyte immunoglobulin-like receptor, subfamily A (with TM domain), member 6 |
| HLA-DRA | Homo sapiens | major histocompatibility complex, class II, DR alpha |
| IL2 | Homo sapiens | interleukin 2 |
| CcI17 | Homo sapiens | chemokine (C-C motif) ligand 17 |
| II3 | Homo sapiens | interleukin 3 (colony-stimulating factor, multiple) |
| pIxnc1 | Homo sapiens | plexin C1 |
| CCL19 | Homo sapiens | chemokine (C-C motif) ligand 19 |
| CXCR6 | Homo sapiens | chemokine (C-X-C motif) receptor 6 |
| CD160 | Homo sapiens | CD160 molecule |
| C8B | Homo sapiens | complement component 8, beta polypeptide |
| Cd48 | Homo sapiens | CD48 molecule |
| Tnfsf811 1 | Homo sapiens | tumor necrosis factor (ligand) superfamily, member 11 |
| SLAMF1 | Homo sapiens | signaling lymphocytic activation molecule family member 1 |
| CD28 | Homo sapiens | CD28 molecule |
| Fcgr2b | Homo sapiens | Fc fragment of IgG, low affinity IIb, receptor (CD32); Fc fragment of IgG, low affinity IIc, receptor for (CD32) |
| FCGR2C | Homo sapiens | Fc fragment of IgG, low affinity lib, receptor (CD32); Fc fragment of IgG, low affinity IIc, receptor for (CD32) |
| POU2AF1 | Homo sapiens | POU class 2 associating factor 1 |
| CLEC6A | Homo sapiens | C-type lectin domain family 6, member A |
| CLEC12A | Homo sapiens | C-type lectin domain family 12, member A; C-type lectin domain family 12, member B |
| CLEC12B | Homo sapiens | C-type lectin domain family 12, member A; C-type lectin domain family 12, member B |
| Cfhr1 | Homo sapiens | complement factor H-related 1 |
| TLR4 | Homo sapiens | toll-like receptor 4 |
| IFIT1B | Homo sapiens | interferon-induced protein with tetratricopeptide repeats 1-like |
| CD3D | Homo sapiens | CD3d molecule, delta (CD3-TCR complex) |
| ANPEP | Homo sapiens | alanyl (membrane) aminopeptidase |
| C 1 qtnf7 | Homo sapiens | C1q and tumor necrosis factor related protein 7 |
| SELP | Homo sapiens | selectin P (granule membrane protein 140kDa, antigen CD62) |
| Cd19 | Homo sapiens | CD19 molecule |
| Ceacam3 | Homo sapiens | carcinoembryonic antigen-related cell adhesion molecule 3 |
| ICOS | Homo sapiens | inducible T-cell co-stimulator |
| C1QTNF2 | Homo sapiens | C1q and tumor necrosis factor related protein 2 |
| TNFSF13B | Homo sapiens | tumor necrosis factor (ligand) superfamily, member 13b |
| CD3E | Homo sapiens | CD3e molecule, epsilon (CD3-TCR complex) |
| THY1 | Homo sapiens | Thy-1 cell surface antigen |
| IL18RAP | Homo sapiens | interleukin 18 receptor accessory protein |
| LY96 | Homo sapiens | lymphocyte antigen 96 |
| LCK | Homo sapiens | lymphocyte-specific protein tyrosine kinase |
| CEBPE | Homo sapiens | CCAAT/enhancer binding protein (C/EBP), epsilon |
| DEFB119 | Homo sapiens | defensin, beta 119 |
| TNFRSF1B | Homo sapiens | tumor necrosis factor receptor superfamily, member 1 B |
| FCER1G | Homo sapiens | Fc fragment of IgE, high affinity I, receptor for; gamma polypeptide |
| IL1RL1 | Homo sapiens | interleukin 1 receptor-like 1 |
| CD80 | Homo sapiens | CD80 molecule |
| TNFRSF17 | Homo sapiens | tumor necrosis factor receptor superfamily, member 17 |
| CD34 | Homo sapiens | CD34 molecule |
| tal1 | Homo sapiens | T-cell acute lymphocytic leukemia 1 |
| CD200R1 | Homo sapiens | CD200 receptor 1 |
| C1 qtnf4 | Homo sapiens | C1q and tumor necrosis factor related protein 4 |
| CLEC7A | Homo sapiens | C-type lectin domain family 7, member A |
| FCGR3B | Homo sapiens | Fc fragment of IgG, low affinity IIIb, receptor (CD16b) |
| IL21R | Homo sapiens | interleukin 21 receptor |
| KIR2DS2 | Homo sapiens | killer cell immunoglobulin-like receptor, two domains, short cytoplasmic tail, 2; killer cell immunoglobulin-like receptor, two domains, short cytoplasmic tail, 4 |
| KIR2DS4 | Homo sapiens | killer cell immunoglobulin-like receptor, two domains, short cytoplasmic tail, 2; killer cell immunoglobulin-like receptor, two domains, short cytoplasmic tail, 4 |
| CXCL12 | Homo sapiens | chemokine (C-X-C motif) ligand 12 (stromal cell-derived factor 1) |
| Ccl11 | Homo sapiens | chemokine (C-C motif) ligand 11 |
| Cd79a | Homo sapiens | CD79a molecule, immunoglobulin-associated alpha |
| IL13 | Homo sapiens | interleukin 13 |
| GZMK | Homo sapiens | granzyme K (granzyme 3; tryptase II) |
| CD163 | Homo sapiens | CD163 molecule |
| CFHR5 | Homo sapiens | complement factor H-related 5 |
| MME | Homo sapiens | membrane metallo-endopeptidase |
| CCR1 | Homo sapiens | chemokine (C-C motif) receptor 1 |
| TNFSF15 | Homo sapiens | tumor necrosis factor (ligand) superfamily, member 15 |
| SLC4A1 | Homo sapiens | solute carrier family 4, anion exchanger, member 1 (erythrocyte membrane protein band 3, Diego blood group) |
| zap70 | Homo sapiens | zeta-chain (TCR) associated protein kinase 70kDa |
| ABCB1 | Homo sapiens | ATP-binding cassette, sub-family B (MDR/TAP), member 1 |
| CTSS | Homo sapiens | cathepsin S |
| LOC100133678 | Homo sapiens | similar to hCG2042724; similar to HLA class II histocompatibility antigen, DQ(1) alpha chain precursor (DC-4 alpha chain); major histocompatibility complex, class II, DQ alpha 1 |
| hla-dqa1 | Homo sapiens | similar to hCG2042724; similar to HLA class II histocompatibility antigen, DQ(1) alpha chain precursor (DC-4 alpha chain); major histocompatibility complex, class II, DQ alpha 1 |
| DEFA1B | Homo sapiens | defensin, alpha 1 |
| DEFA1 | Homo sapiens | defensin, alpha 1 |
| Prf1 | Homo sapiens | perforin 1 (pore forming protein) |
| lyg2 | Homo sapiens | lysozyme G-like 2 |
| EBF2 | Homo sapiens | early B-cell factor 2 |
| ICAM4 | Homo sapiens | intercellular adhesion molecule 4 (Landsteiner-Wiener blood group) |
| IL1F7 | Homo sapiens | interleukin 1 family, member 7 (zeta) |
| CAMP | Homo sapiens | cathelicidin antimicrobial peptide |
| CD207 | Homo sapiens | CD207 molecule, langerin |
| il2rb | Homo sapiens | interleukin 2 receptor, beta |
| IL12RB1 | Homo sapiens | interleukin 12 receptor, beta 1 |
| CFHR3 | Homo sapiens | complement factor H-related 3 |
| Cd6 | Homo sapiens | CD6 molecule |
| cd38 | Homo sapiens | CD38 molecule |
| CXCL1 | Homo sapiens | chemokine (C-X-C motif) ligand 1 (melanoma growth stimulating activity, alpha) |
| Cxcl14 | Homo sapiens | chemokine (C-X-C motif) ligand 14 |
| IL23R | Homo sapiens | interleukin 23 receptor |
| PF4 | Homo sapiens | platelet factor 4 |
| Cx3cl1 | Homo sapiens | chemokine (C-X3-C motif) ligand 1 |
| SELL | Homo sapiens | selectin L |
| IRF5 | Homo sapiens | interferon regulatory factor 5 |
| Ciita | Homo sapiens | class II, major histocompatibility complex, transactivator |
| WIPF1 | Homo sapiens | WAS/WASL interacting protein family, member 1 |
| FOXP3 | Homo sapiens | forkhead box P3 |
| FCRLA | Homo sapiens | Fc receptor-like A |
| DEFB123 | Homo sapiens | defensin, beta 123 |
| IL10RA | Homo sapiens | interleukin 10 receptor, alpha |
| IL13RA2 | Homo sapiens | interleukin 13 receptor, alpha 2 |
| NCF1C | Homo sapiens | neutrophil cytosolic factor 1; neutrophil cytosolic factor 1C pseudogene |
| ncf1 | Homo sapiens | neutrophil cytosolic factor 1; neutrophil cytosolic factor 1 C pseudogene |
| KIR2DL1 | Homo sapiens | killer cell immunoglobulin-like receptor, two domains, long cytoplasmic tail, 2; killer cell immunoglobulin-like receptor, two domains, long cytoplasmic tail, 1; killer-cell Ig-like receptor; killer cell immunoglobulin-like receptor, three domains, pseudogene 1 |
| KIR3DP1 | Homo sapiens | killer cell immunoglobulin-like receptor, two domains, long cytoplasmic tail, 2; killer cell immunoglobulin-like receptor, two domains, long cytoplasmic tail, 1; killer-cell Ig-like receptor; killer cell immunoglobulin-like receptor, three domains, pseudogene 1 |
| KIR2DL2 | Homo sapiens | killer cell immunoglobulin-like receptor, two domains, long cytoplasmic tail, 2; killer cell immunoglobulin-like receptor, two domains, long cytoplasmic tail, 1; killer-cell lg-like receptor; killer cell immunoglobulin-like receptor, three domains, pseudogene 1 |
| Cd96 | Homo sapiens | CD96 molecule |
| SIGLEC6 | Homo sapiens | sialic acid binding Ig-like lectin 6 |
| IL9 | Homo sapiens | interleukin 9 |
| II24 | Homo sapiens | interleukin 24 |
| IL20 | Homo sapiens | interleukin 20 |
| Ceacam6 | Homo sapiens | carcinoembryonic antigen-related cell adhesion molecule 6 (nonspecific cross reacting antigen) |
| Cd101 | Homo sapiens | immunoglobulin superfamily, member 2 |
| CD300LF | Homo sapiens | CD300 molecule-like family member f |
| ITGAM | Homo sapiens | integrin, alpha M (complement component 3 receptor 3 subunit) |
| FCAMR | Homo sapiens | Fc receptor, IgA, IgM, high affinity |
| WASF3 | Homo sapiens | WAS protein family, member 3 |
| CD8B | Homo sapiens | CD8b molecule |
| II27 | Homo sapiens | interleukin 27 |
| SLAMF6 | Homo sapiens | SLAM family member 6 |
| GZMA | Homo sapiens | granzyme A (granzyme 1, cytotoxic T-lymphocyte-associated serine esterase 3) |
| CXCL11 | Homo sapiens | chemokine (C-X-C motif) ligand 11 |
| CD4 | Homo sapiens | CD4 molecule |
| ccI1 | Homo sapiens | chemokine (C-C motif) ligand 1 |
| CD244 | Homo sapiens | CD244 molecule, natural killer cell receptor 2B4 |
| Ly9 | Homo sapiens | lymphocyte antigen 9 |
| ccl8 | Homo sapiens | chemokine (C-C motif) ligand 8 |
| TCN2 | Homo sapiens | transcobalamin II; macrocytic anemia |
| CSF3 | Homo sapiens | colony stimulating factor 3 (granulocyte) |
| CD5 | Homo sapiens | CD5 molecule |
| CXCL5 | Homo sapiens | chemokine (C-X-C motif) ligand 5 |
| IL19 | Homo sapiens | interleukin 19 |
| masp2 | Homo sapiens | mannan-binding lectin serine peptidase 2 |
| PPBP | Homo sapiens | pro-platelet basic protein (chemokine (C-X-C motif) ligand 7) |
| C6 | Homo sapiens | complement component 6 |
| CMTM8 | Homo sapiens | CKLF-like MARVEL transmembrane domain containing 8 |
| SIGLEC1 | Homo sapiens | sialic acid binding Ig-like lectin 1, sialoadhesin |
| II1a | Homo sapiens | interleukin 1, alpha |
| CCL20 | Homo sapiens | chemokine (C-C motif) ligand 20 |
| MbI2 | Homo sapiens | mannose-binding lectin (protein C) 2, soluble (opsonic defect) |
| GYPB | Homo sapiens | glycophorin E; glycophorin B (MNS blood group) |
| GYPE | Homo sapiens | glycophorin E; glycophorin B (MNS blood group) |
| TNFRSF8 | Homo sapiens | tumor necrosis factor receptor superfamily, member 8 |
| Scgb3a1 | Homo sapiens | secretoglobin, family 3A, member 1 |
| irak3 | Homo sapiens | interleukin-1 receptor-associated kinase 3 |
| HTN3 | Homo sapiens | histatin 3 |
| CD248 | Homo sapiens | CD248 molecule, endosialin |
| C8A | Homo sapiens | complement component 8, alpha polypeptide |
| CD164L2 | Homo sapiens | CD164 sialomucin-like 2 |
| BANK1 | Homo sapiens | B-cell scaffold protein with ankyrin repeats 1 |
| IL12A | Homo sapiens | interleukin 12A (natural killer cell stimulatory factor 1, cytotoxic lymphocyte maturation factor 1, p35) |
| CHL1 | Homo sapiens | cell adhesion molecule with homology to L1CAM (close homolog of L1) |
| Spn | Homo sapiens | sialophorin |
| Traf1 | Homo sapiens | TNF receptor-associated factor 1 |
| il22 | Homo sapiens | interleukin 22 |
| Cxcr1 | Homo sapiens | interleukin 8 receptor, alpha |
| TEK | Homo sapiens | TEK tyrosine kinase, endothelial |
| FCRL5 | Homo sapiens | Fc receptor-like 5 |
| Cd300Ig | Homo sapiens | CD300 molecule-like family member g |
| DOCK2 | Homo sapiens | dedicator of cytokinesis 2 |
| Cx3cr1 | Homo sapiens | chemokine (C-X3-C motif) receptor 1 |
| PGLYRP2 | Homo sapiens | peptidoglycan recognition protein 2 |
| WFDC12 | Homo sapiens | WAP four-disulfide core domain 12 |
| PLA2G7 | Homo sapiens | phospholipase A2, group VII (platelet-activating factor acetylhydrolase, plasma) |
| SLAMF7 | Homo sapiens | SLAM family member 7 |
| LY86 | Homo sapiens | lymphocyte antigen 86 |
| GZMM | Homo sapiens | granzyme M (lymphocyte met-ase 1) |
| PDCD1 LG2 | Homo sapiens | programmed cell death 1 ligand 2 |
| C1QC | Homo sapiens | complement component 1, q subcomponent, C chain |
| GZMB | Homo sapiens | granzyme B (granzyme 2, cytotoxic T-lymphocyte-associated serine esterase 1) |
| DEFA5 | Homo sapiens | defensin, alpha 5, Paneth cell-specific |
| dcd | Homo sapiens | dermcidin |
| CcI2 | Homo sapiens | chemokine (C-C motif) ligand 2 |
| CLEC4A | Homo sapiens | C-type lectin domain family 4, member A |
| IL1RAPL1 | Homo sapiens | interleukin 1 receptor accessory protein-like 1 |
| IL26 | Homo sapiens | interleukin 26 |
| C1ql4 | Homo sapiens | complement component 1, q subcomponent-like 4 |
| IFNG | Homo sapiens | interferon, gamma |
| CEACAM5 | Homo sapiens | carcinoembryonic antigen-related cell adhesion molecule 5 |
| TNFRSF18 | Homo sapiens | tumor necrosis factor receptor superfamily, member 18 |
| POMC | Homo sapiens | proopiomelanocortin |
| PTPN22 | Homo sapiens | protein tyrosine phosphatase, non-receptor type 22 (lymphoid) |
| Itgax | Homo sapiens | integrin, alpha X (complement component 3 receptor 4 subunit) |
| DEFB4A | Homo sapiens | defensin, beta 4 |
| Ncam1 | Homo sapiens | neural cell adhesion molecule 1 |
| CD7 | Homo sapiens | CD7 molecule |
| TLR5 | Homo sapiens | toll-like receptor 5 |
| GNLY | Homo sapiens | granulysin |
| Gpr183 | Homo sapiens | G protein-coupled receptor 183 |
| Ncf4 | Homo sapiens | neutrophil cytosolic factor 4, 40kDa |
| CSF2RB | Homo sapiens | colony stimulating factor 2 receptor, beta, low-affinity (granulocyte-macrophage) |
| Defa4 | Homo sapiens | defensin, alpha 4, corticostatin |
| TNFRSF13B | Homo sapiens | tumor necrosis factor receptor superfamily, member 13B |
| DEFA6 | Homo sapiens | defensin, alpha 6, Paneth cell-specific |
| TLR1 | Homo sapiens | toll-like receptor 1 |
| AICDA | Homo sapiens | activation-induced cytidine deaminase |
| PTAFR | Homo sapiens | platelet-activating factor receptor |
| CCL13 | Homo sapiens | chemokine (C-C motif) ligand 13 |
| ALK | Homo sapiens | anaplastic lymphoma receptor tyrosine kinase |
| HRH2 | Homo sapiens | histamine receptor H2 |
| C3AR1 | Homo sapiens | complement component 3a receptor 1 |
| XCL1 | Homo sapiens | chemokine (C motif) ligand 1 |
| IFNB1 | Homo sapiens | interferon, beta 1, fibroblast |
| TNFRSF13C | Homo sapiens | tumor necrosis factor receptor superfamily, member 13C |
| CD84 | Homo sapiens | CD84 molecule |
| XCL2 | Homo sapiens | chemokine (C motif) ligand 2 |
| TLR2 | Homo sapiens | toll-like receptor 2 |
| CCL7 | Homo sapiens | chemokine (C-C motif) ligand 7 |
| Vpreb1 | Homo sapiens | pre-B lymphocyte 1 |
| CLEC4C | Homo sapiens | C-type lectin domain family 4, member C |
| NFATC2 | Homo sapiens | nuclear factor of activated T-cells, cytoplasmic, calcineurin-dependent 2 |
| LAX1 | Homo sapiens | lymphocyte transmembrane adaptor 1 |
| Cfi | Homo sapiens | complement factor I |
| lcp2 | Homo sapiens | lymphocyte cytosolic protein 2 (SH2 domain containing leukocyte protein of 76kDa) |
| FLT3 | Homo sapiens | fms-related tyrosine kinase 3 |
| IL2RA | Homo sapiens | interleukin 2 receptor, alpha |
| CD74 | Homo sapiens | CD74 molecule, major histocompatibility complex, class II invariant chain |
| CD93 | Homo sapiens | CD93 molecule |
| pdgfra | Homo sapiens | platelet-derived growth factor receptor, alpha polypeptide |
| CFHR4 | Homo sapiens | complement factor H-related 4 |
| CTSG | Homo sapiens | cathepsin G |
| Sirpa | Homo sapiens | signal-regulatory protein alpha |
| IL8 | Homo sapiens | interleukin 8 |
| CD36 | Homo sapiens | CD36 molecule (thrombospondin receptor) |
| MYLK | Homo sapiens | myosin light chain kinase |
| Hamp | Homo sapiens | hepcidin antimicrobial peptide |
| IL1RAPL2 | Homo sapiens | interleukin 1 receptor accessory protein-like 2 |
| SERPING1 | Homo sapiens | serpin peptidase inhibitor, clade G (C1 inhibitor), member 1 |
| cmklr1 | Homo sapiens | chemokine-like receptor 1 |
| CCR6 | Homo sapiens | cyclin L2; chemokine (C-C motif) receptor 6 |
| Ccnl2 | Homo sapiens | cyclin L2; chemokine (C-C motif) receptor 6 |
| btk | Homo sapiens | Bruton agammaglobulinemia tyrosine kinase |
| ITGA4 | Homo sapiens | integrin, alpha 4 (antigen CD49D, alpha 4 subunit of VLA-4 receptor) |
| CD180 | Homo sapiens | CD180 molecule |
| NCR2 | Homo sapiens | natural cytotoxicity triggering receptor 2 |
| PRG2 | Homo sapiens | proteoglycan 2, bone marrow (natural killer cell activator, eosinophil granule major basic protein) |
| KIR2DL4 | Homo sapiens | killer cell immunoglobulin-like receptor, two domains, long cytoplasmic tail, 4 |
| HRH4 | Homo sapiens | histamine receptor H4 |
| Cd2 | Homo sapiens | CD2 molecule |
| RHAG | Homo sapiens | Rh-associated glycoprotein |
| LILRA1 | Homo sapiens | leukocyte immunoglobulin-like receptor, subfamily A (with TM domain), member 1 |
| LILRB1 | Homo sapiens | leukocyte immunoglobulin-like receptor, subfamily B (with TM and ITIM domains), member 1 |
| SELE | Homo sapiens | selectin E |
| II1rl2 | Homo sapiens | interleukin 1 receptor-like 2 |
| Cxcl9 | Homo sapiens | chemokine (C-X-C motif) ligand 9 |
| CD27 | Homo sapiens | CD27 molecule |
| IL1R2 | Homo sapiens | interleukin 1 receptor, type II |
| II28a | Homo sapiens | interleukin 28A (interferon, lambda 2) |
| EPO | Homo sapiens | erythropoietin |
| Clec4e | Homo sapiens | C-type lectin domain family 4, member E |
| LOC727787 | Homo sapiens | killer cell immunoglobulin-like receptor, three domains, long cytoplasmic tail, 2; similar to killer cell immunoglobulin-like receptor 3DL2 precursor (MHC class I NK cell receptor) (Natural killer-associated transcript 4) (NKAT-4) (p70 natural killer cell receptor clone CL-5) (CD158k antigen) |
| KIR3DL2 | Homo sapiens | killer cell immunoglobulin-like receptor, three domains, long cytoplasmic tail, 2; similar to killer cell immunoglobulin-like receptor 3DL2 precursor (MHC class I NK cell receptor) (Natural killer-associated transcript 4) (NKAT-4) (p70 natural killer cell receptor clone CL-5) (CD158k antigen) |
| C7 | Homo sapiens | complement component 7 |
| Fcgr3a | Homo sapiens | Fc fragment of IgG, low affinity IIIa, receptor (CD16a) |
| Cmtm3 | Homo sapiens | CKLF-like MARVEL transmembrane domain containing 3 |
| CLEC4D | Homo sapiens | C-type lectin domain family 4, member D |
| CMTM2 | Homo sapiens | CKLF-like MARVEL transmembrane domain containing 2 |
| CD300E | Homo sapiens | CD300e molecule |
| KLRB1 | Homo sapiens | killer cell lectin-like receptor subfamily B, member 1 |
| IL12B | Homo sapiens | interleukin 12B (natural killer cell stimulatory factor 2, cytotoxic lymphocyte maturation factor 2, p40) |
| IL17RD | Homo sapiens | interleukin 17 receptor D |
| 1131 ra | Homo sapiens | interleukin 31 receptor A |
| Batf | Homo sapiens | basic leucine zipper transcription factor, ATF-like |
| LTF | Homo sapiens | lactotransferrin |
| csf3r | Homo sapiens | colony stimulating factor 3 receptor (granulocyte) |
| II17f | Homo sapiens | interleukin 17F |
| IL17A | Homo sapiens | interleukin 17A |
| IL17C | Homo sapiens | interleukin 17C |
| TNFSF4 | Homo sapiens | tumor necrosis factor (ligand) superfamily, member 4 |
| fcgr1a | Homo sapiens | Fc fragment of IgG, high affinity Ic, receptor (CD64); Fc fragment of IgG, high affinity la, receptor (CD64) |
| FCGR1 C | Homo sapiens | Fc fragment of IgG, high affinity Ic, receptor (CD64); Fc fragment of IgG, hiqh affinity la, receptor (CD64) |
| Ly75 | Homo sapiens | CD302 molecule; lymphocyte antigen 75 |
| CD302 | Homo sapiens | CD302 molecule; lymphocyte antigen 75 |
| crp | Homo sapiens | C-reactive protein, pentraxin-related |
| CD226 | Homo sapiens | CD226 molecule |
| CD1B | Homo sapiens | CD1b molecule |
| CXCR2 | Homo sapiens | interleukin 8 receptor, beta |
| CXCL13 | Homo sapiens | chemokine (C-X-C motif) ligand 13 |
| Ccr3 | Homo sapiens | chemokine (C-C motif) receptor 3 |
| CD1E | Homo sapiens | CD1 e molecule |
| PIK3CG | Homo sapiens | phosphoinositide-3-kinase, catalytic, gamma polypeptide |
| LILRB4 | Homo sapiens | leukocyte immunoglobulin-like receptor, subfamily B (with TM and ITIM domains), member 4 |
| FCGR2A | Homo sapiens | Fc fragment of IgG, low affinity IIa, receptor (CD32) |
| BCAM | Homo sapiens | basal cell adhesion molecule (Lutheran blood group) |
| Csf2 | Homo sapiens | colony stimulating factor 2 (granulocyte-macrophage) |
| Ccl27 | Homo sapiens | chemokine (C-C motif) ligand 27 |
| Pglyrp3 | Homo sapiens | peptidoglycan recognition protein 3 |
| CD1C | Homo sapiens | CD1c molecule |
| Colec12 | Homo sapiens | collectin sub-family member 12 |
| CD1A | Homo sapiens | CD1a molecule |
| IL21 | Homo sapiens | interleukin 21 |
| MS4A3 | Homo sapiens | membrane-spanning 4-domains, subfamily A, member 3 (hematopoietic cell-specific) |
| Lilra5 | Homo sapiens | leukocyte immunoglobulin-like receptor, subfamily A (with TM domain), member 5 |
| CR1 | Homo sapiens | complement component (3b/4b) receptor 1 (Knops blood group) |
| PPP3R2 | Homo sapiens | protein phosphatase 3 (formerly 2B), regulatory subunit B, beta isoform |
| CXCR3 | Homo sapiens | chemokine (C-X-C motif) receptor 3 |
| Pdcd1 | Homo sapiens | programmed cell death 1 |
| LILRB2 | Homo sapiens | leukocyte immunoglobulin-like receptor, subfamily B (with TM and ITIM domains), member 2 |
| ccr4 | Homo sapiens | chemokine (C-C motif) receptor 4 |
| RNASE7 | Homo sapiens | ribonuclease, RNase A family, 7 |
| Msr1 | Homo sapiens | macrophage scavenger receptor 1 |
| ccl25 | Homo sapiens | chemokine (C-C motif) ligand 25 |
| FASLG | Homo sapiens | Fas ligand (TNF superfamily, member 6) |
| CD1D | Homo sapiens | CD1d molecule |
| CCL28 | Homo sapiens | chemokine (C-C motif) ligand 28 |
| A2ML1 | Homo sapiens | alpha-2-macroglobulin-like 1 |
| CEACAM8 | Homo sapiens | carcinoembryonic antigen-related cell adhesion molecule 8 |
| FGFR2 | Homo sapiens | fibroblast growth factor receptor 2 |
| Cd5l | Homo sapiens | CD5 molecule-like |
| Fcar | Homo sapiens | Fc fragment of IgA, receptor for |
| DEFB106A | Homo sapiens | defensin, beta 106A; defensin, beta 106B |
| DEFB106B | Homo sapiens | defensin, beta 106A; defensin, beta 106B |
| IRF8 | Homo sapiens | interferon regulatory factor 8 |
| lgj | Homo sapiens | immunoglobulin J polypeptide, linker protein for immunoglobulin alpha and mu polypeptides |
| ACE | Homo sapiens | angiotensin I converting enzyme (peptidyl-dipeptidase A) 1 |
| MPO | Homo sapiens | myeloperoxidase |
| CD86 | Homo sapiens | CD86 molecule |
| CD40 | Homo sapiens | CD40 molecule, TNF receptor superfamily member 5 |
| CD209 | Homo sapiens | CD209 molecule |
| KIR3DS1 | Homo sapiens | killer cell immunoglobulin-like receptor, three domains, short cytoplasmic tail, 1; killer cell immunoglobulin-like receptor, three domains, long cytoplasmic tail, 1 |
| Kir3dl1 | Homo sapiens | killer cell immunoglobulin-like receptor, three domains, short cytoplasmic tail, 1; killer cell immunoglobulin-like receptor, three domains, long cytoplasmic tail, 1 |
| mpl | Homo sapiens | myeloproliferative leukemia virus oncogene |
| Tnfrsf4 | Homo sapiens | tumor necrosis factor receptor superfamily, member 4 |
| IL17B | Homo sapiens | interleukin 17B |
| PSTPIP1 | Homo sapiens | proline-serine-threonine phosphatase interacting protein 1 |
| DPP4 | Homo sapiens | dipeptidyl-peptidase 4 |
| AIRE | Homo sapiens | autoimmune regulator |
| Cd53 | Homo sapiens | CD53 molecule |
| tbx21 | Homo sapiens | T-box 21 |
| THBD | Homo sapiens | thrombomodulin |
| CCR7 | Homo sapiens | chemokine (C-C motif) receptor 7 |
| CLEC10A | Homo sapiens | C-type lectin domain family 10, member A |
| CD274 | Homo sapiens | CD274 molecule |
| Cxcl6 | Homo sapiens | chemokine (C-X-C motif) ligand 6 (granulocyte chemotactic protein 2) |
| CD69 | Homo sapiens | CD69 molecule |
| CD22 | Homo sapiens | CD22 molecule |
| IL23A | Homo sapiens | interleukin 23, alpha subunit p19 |
| NOD2 | Homo sapiens | nucleotide-binding oligomerization domain containing 2 |
| TNFRSF9 | Homo sapiens | tumor necrosis factor receptor superfamily, member 9 |
| LPO | Homo sapiens | lactoperoxidase |
| nfatc1 | Homo sapiens | nuclear factor of activated T-cells, cytoplasmic, calcineurin-dependent1 |
| CMTM5 | Homo sapiens | CKLF-like MARVEL transmembrane domain containing 5 |
| IL25 | Homo sapiens | interleukin 25 |
| Lair1 | Homo sapiens | leukocyte-associated immunoglobulin-like receptor 1 |
| LAIR2 | Homo sapiens | leukocyte-associated immunoglobulin-like receptor 2 |
| NOS2 | Homo sapiens | nitric oxide synthase 2, inducible |
| NCR3 | Homo sapiens | natural cytotoxicity triggering receptor 3 |
| SOD3 | Homo sapiens | superoxide dismutase 3, extracellular |
| IL28B | Homo sapiens | interleukin 28B (interferon, lambda 3) |
| RHD | Homo sapiens | Rh blood group, D antigen |
| KLRD1 | Homo sapiens | killer cell lectin-like receptor subfamily D, member 1 |
| Tnfsf14 | Homo sapiens | tumor necrosis factor (ligand) superfamily, member 14 |
| IL9R | Homo sapiens | interleukin 9 receptor |
| il10 | Homo sapiens | interleukin 10 |
| CCR10 | Homo sapiens | chemokine (C-C motif) receptor 10 |
| CR2 | Homo sapiens | complement component (3d/Epstein Barr virus) receptor 2 |
| DEFB105B | Homo sapiens | defensin, beta 105A; defensin, beta 105B |
| DEFB105A | Homo sapiens | defensin, beta 105A; defensin, beta 105B |
| LYZ | Homo sapiens | lysozyme (renal amyloidosis) |
| DEFB103A | Homo sapiens | defensin, beta 103B; defensin, beta 103A |
| DEFB103B | Homo sapiens | defensin, beta 103B; defensin, beta 103A |
| CMTM7 | Homo sapiens | CKLF-like MARVEL transmembrane domain containing 7 |
| LILRB5 | Homo sapiens | leukocyte immunoglobulin-like receptor, subfamily B (with TM and ITIM domains), member 5 |
| IL4 | Homo sapiens | interleukin 4 |

**Table 2**

| **Gene Symbol** | **Species** | **Gene Name** |
|---|---|---|
| CD300LB | Homo sapiens | CD300 molecule-like family member b |
| C1QB | Homo sapiens | complement component 1, q subcomponent, B chain |
| CXCR5 | Homo sapiens | chemokine (C-X-C motif) receptor 5 |
| FUT3 | Homo sapiens | fucosyltransferase 3 (galactoside 3(4)-L-fucosyltransferase, Lewis blood group) |
| EBF1 | Homo sapiens | early B-cell factor 1 |
| Sh2d1a | Homo sapiens | SH2 domain protein 1A |
| tlr9 | Homo sapiens | toll-like receptor 9 |
| LAMP3 | Homo sapiens | lysosomal-associated membrane protein 3 |
| TCF7 | Homo sapiens | transcription factor 7 (T-cell specific, HMG-box) |
| IL1F6 | Homo sapiens | interleukin 1 family, member 6 (epsilon) |
| IL1F5 | Homo sapiens | interleukin 1 family, member 5 (delta) |
| IL1F10 | Homo sapiens | interleukin 1 family, member 10 (theta) |
| NFATC4 | Homo sapiens | nuclear factor of activated T-cells, cytoplasmic, calcineurin-dependent 4 |
| DARC | Homo sapiens | Duffy blood group, chemokine receptor |
| CCR2 | Homo sapiens | chemokine (C-C motif) receptor 2 |
| ITGAL | Homo sapiens | integrin, alpha L (antigen CD11A (p180), lymphocyte function-associated antigen 1; alpha polypeptide) |
| C1QL2 | Homo sapiens | complement component 1, q subcomponent-like 2 |
| Gypa | Homo sapiens | glycophorin A (MNS blood group) |
| Il31 | Homo sapiens | interleukin 31 |
| Ptprc | Homo sapiens | protein tyrosine phosphatase, receptor type, C |
| ITGAD | Homo sapiens | integrin, alpha D |
| HLA-DQB1 | Homo sapiens | major histocompatibility complex, class II, DQ beta 1; similar to major histocompatibility complex, class II, DQ beta 1 |
| LOC100133583 | Homo sapiens | major histocompatibility complex, class II, DQ beta 1; similar to major histocompatibility complex, class II, DQ beta 1 |
| C1qa | Homo sapiens | complement component 1, q subcomponent, A chain |
| IL22RA2 | Homo sapiens | interleukin 22 receptor, alpha 2 |
| CD300A | Homo sapiens | CD300a molecule |
| Tnfsf8 | Homo sapiens | tumor necrosis factor (ligand) superfamily, member 8 |
| Cd300c | Homo sapiens | CD300c molecule |
| Cytl1 | Homo sapiens | cytokine-like 1 |
| Ctla4 | Homo sapiens | cytotoxic T-lymphocyte-associated protein 4 |
| PLA2R1 | Homo sapiens | phospholipase A2 receptor 1,180kDa |
| Xcr1 | Homo sapiens | chemokine (C motif) receptor 1 |
| STAT4 | Homo sapiens | signal transducer and activator of transcription 4 |
| C1QL3 | Homo sapiens | complement component 1, q subcomponent-like 3 |
| Ncr1 | Homo sapiens | natural cytotoxicity triggering receptor 1 |
| PROM1 | Homo sapiens | prominin 1 |
| CCRL1 | Homo sapiens | chemokine (C-C motif) receptor-like 1 |
| CFP | Homo sapiens | complement factor properdin |
| HLA-DRB5 | Homo sapiens | major histocompatibility complex, class II, DR beta 5 |
| IGLL1 | Homo sapiens | immunoglobulin lambda-like polypeptide 1 |
| CD14 | Homo sapiens | CD14 molecule |
| FCER2 | Homo sapiens | Fc fragment of IgE, low affinity II, receptor for (CD23) |
| itga2b | Homo sapiens | integrin, alpha 2b (platelet glycoprotein lib of IIb/IIIa complex, antigen CD41) |
| CCR9 | Homo sapiens | chemokine (C-C motif) receptor 9 |
| CCL24 | Homo sapiens | chemokine (C-C motif) ligand 24 |
| Cd3g | Homo sapiens | CD3g molecule, gamma (CD3-TCR complex) |
| CCR5 | Homo sapiens | chemokine (C-C motif) receptor 5 |
| KDR | Homo sapiens | kinase insert domain receptor (a type III receptor tyrosine kinase) |
| Cd8a | Homo sapiens | CD8a molecule |
| HLA-DOA | Homo sapiens | major histocompatibility complex, class II, DO alpha |
| Pglyrp1 | Homo sapiens | peptidoglycan recognition protein 1 |
| Cd79b | Homo sapiens | CD79b molecule, immunoglobulin-associated beta |
| Cr1l | Homo sapiens | complement component (3b/4b) receptor 1-like |
| IL1RN | Homo sapiens | interleukin 1 receptor antagonist |
| Il1f9 | Homo sapiens | interleukin 1 family, member 9 |
| CEACAM1 | Homo sapiens | carcinoembryonic antigen-related cell adhesion molecule 1 (biliary glycoprotein) |
| LOC652799 | Homo sapiens | similar to Mast/stem cell growth factor receptor precursor (SCFR) (Proto-oncogene tyrosine-protein kinase Kit) (c-kit) (CD117 antigen); v-kit Hardy-Zuckerman 4 feline sarcoma viral oncogene homolog |
| LOC653882 | Homo sapiens | similar to Mast/stem cell growth factor receptor precursor (SCFR) (Proto-oncogene tyrosine-protein kinase Kit) (c-kit) (CD117 antigen); v-kit Hardy-Zuckerman 4 feline sarcoma viral oncogene homolog |
| KIT | Homo sapiens | similar to Mast/stem cell growth factor receptor precursor (SCFR) (Proto-oncogene tyrosine-protein kinase Kit) (c-kit) (CD117 antigen); v-kit Hardy-Zuckerman 4 feline sarcoma viral oncogene homolog |
| FCER1A | Homo sapiens | Fc fragment of IgE, high affinity I, receptor for; alpha polypeptide |
| IL12RB2 | Homo sapiens | interleukin 12 receptor, beta 2 |
| rac2 | Homo sapiens | ras-reiated C3 botulinum toxin substrate 2 (rho family, small GTP binding protein Rac2) |
| CD200R1 L | Homo sapiens | CD200 receptor 1-like |
| BTLA | Homo sapiens | B and T lymphocyte associated |
| KEL | Homo sapiens | Kell blood group, metallo-endopeptidase |
| Siglec15 | Homo sapiens | sialic acid binding Ig-like lectin 15 |
| MS4A1 | Homo sapiens | membrane-spanning 4-domains, subfamily A, member 1 |
| SIGLEC5 | Homo sapiens | sialic acid binding Ig-like lectin 5 |
| Bst1 | Homo sapiens | bone marrow stromal cell antigen 1 |
| Ms4a5 | Homo sapiens | membrane-spanning 4-domains, subfamily A, member 5 |
| GP9 | Homo sapiens | glycoprotein IX (platelet) |
| IL29 | Homo sapiens | interleukin 29 (interferon, lambda 1) |
| LILRA4 | Homo sapiens | leukocyte immunoglobulin-like receptor, subfamily A (with TM domain), member 4 |
| CD40LG | Homo sapiens | CD40 ligand |
| GYPC | Homo sapiens | glycophorin C (Gerbich blood group) |
| CCL22 | Homo sapiens | chemokine (C-C motif) ligand 22 |
| GP5 | Homo sapiens | glycoprotein V (platelet) |
| IL16 | Homo sapiens | interleukin 16 (lymphocyte chemoattractant factor) |
| CLEC4M | Homo sapiens | C-type lectin domain family 4, member M |
| EPX | Homo sapiens | eosinophil peroxidase |
| Vcam1 | Homo sapiens | vascular cell adhesion molecule 1 |
| Cd247 | Homo sapiens | CD247 molecule |
| LILRA6 | Homo sapiens | leukocyte immunoglobulin-like receptor, subfamily A (with TM domain), member 6 |
| HLA-DRA | Homo sapiens | major histocompatibility complex, class II, DR alpha |
| IL2 | Homo sapiens | interleukin 2 |
| Ccl17 | Homo sapiens | chemokine (C-C motif) ligand 17 |
| Il3 | Homo sapiens | interleukin 3 (colony-stimulating factor, multiple) |
| plxnc1 | Homo sapiens | plexin C1 |
| CCL19 | Homo sapiens | chemokine (C-C motif) ligand 19 |
| CXCR6 | Homo sapiens | chemokine (C-X-C motif) receptor 6 |
| CD160 | Homo sapiens | CD160 molecule |
| C8B | Homo sapiens | complement component 8, beta polypeptide |
| Cd48 | Homo sapiens | CD48 molecule |
| Tnfsf11 | Homo sapiens | tumor necrosis factor (ligand) superfamily, member 11 |
| SLAMF1 | Homo sapiens | signaling lymphocytic activation molecule family member 1 |
| CD28 | Homo sapiens | CD28 molecule |
| Fcgr2b | Homo sapiens | Fc fragment of IgG, low affinity IIb, receptor (CD32); Fc fragment of IgG, low affinity IIc, receptor for (CD32) |
| FCGR2C | Homo sapiens | Fc fragment of IgG, low affinity IIb, receptor (CD32); Fc fragment of IgG, low affinity IIc, receptor for (CD32) |
| POU2AF1 | Homo sapiens | POU class 2 associating factor 1 |
| CLEC6A | Homo sapiens | C-type lectin domain family 6, member A |
| CLEC12A | Homo sapiens | C-type lectin domain family 12, member A; C-type lectin domain family 12, member B |
| CLEC12B | Homo sapiens | C-type lectin domain family 12, member A; C-type lectin domain family 12, member B |
| Cfhr1 | Homo sapiens | complement factor H-related 1 |
| TLR4 | Homo sapiens | toll-like receptor 4 |
| IFIT1B | Homo sapiens | interferon-induced protein with tetratricopeptide repeats 1-like |
| CD3D | Homo sapiens | CD3d molecule, delta (CD3-TCR complex) |
| ANPEP | Homo sapiens | alanyl (membrane) aminopeptidase |
| C1 qtnf7 | Homo sapiens | C1q and tumor necrosis factor related protein 7 |
| SELP | Homo sapiens | selectin P (granule membrane protein 140kDa, antigen CD62) |
| Cd19 | Homo sapiens | CD19 molecule |
| Ceacam3 | Homo sapiens | carcinoembryonic antigen-related cell adhesion molecule 3 |
| ICOS | Homo sapiens | inducible T-cell co-stimulator |
| C1 QTNF2 | Homo sapiens | C1 q and tumor necrosis factor related protein 2 |
| TNFSF13B | Homo sapiens | tumor necrosis factor (ligand) superfamily, member 13b |
| CD3E | Homo sapiens | CD3e molecule, epsilon (CD3-TCR complex) |
| THY1 | Homo sapiens | Thy-1 cell surface antigen |
| IL18RAP | Homo sapiens | interleukin 18 receptor accessory protein |
| LY96 | Homo sapiens | lymphocyte antigen 96 |
| LCK | Homo sapiens | lymphocyte-specific protein tyrosine kinase |
| CEBPE | Homo sapiens | CCAAT/enhancer binding protein (C/EBP), epsilon |
| DEFB119 | Homo sapiens | defensin, beta 119 |
| TNFRSF1B | Homo sapiens | tumor necrosis factor receptor superfamily, member 1 B |
| FCER1G | Homo sapiens | Fc fragment of IgE, high affinity I, receptor for; gamma polypeptide |
| IL1RL1 | Homo sapiens | interleukin 1 receptor-like 1 |
| CD80 | Homo sapiens | CD80 molecule |
| TNFRSF17 | Homo sapiens | tumor necrosis factor receptor superfamily, member 17 |
| CD34 | Homo sapiens | CD34 molecule |
| tal1 | Homo sapiens | T-cell acute lymphocytic leukemia 1 |
| CD200R1 | Homo sapiens | CD200 receptor 1 |
| C1 qtnf4 | Homo sapiens | C1 q and tumor necrosis factor related protein 4 |
| CLEC7A | Homo sapiens | C-type lectin domain family 7, member A |
| FCGR3B | Homo sapiens | Fc fragment of IgG, low affinity IIIb, receptor (CD16b) |
| IL21 R | Homo sapiens | interleukin 21 receptor |
| KIR2DS2 | Homo sapiens | killer cell immunoglobulin-like receptor, two domains, short cytoplasmic tail, 2; killer cell immunoglobulin-like receptor, two domains, short cytoplasmic tail, 4 |
| KIR2DS4 | Homo sapiens | killer cell immunoglobulin-like receptor, two domains, short cytoplasmic tail, 2; killer cell immunoglobulin-like receptor, two domains, short cytoplasmic tail, 4 |
| CXCL12 | Homo sapiens | chemokine (C-X-C motif) ligand 12 (stromal cell-derived factor 1) |
| Ccl11 | Homo sapiens | chemokine (C-C motif) ligand 11 |
| Cd79a | Homo sapiens | CD79a molecule, immunoglobulin-associated alpha |
| IL13 | Homo sapiens | interleukin 13 |
| GZMK | Homo sapiens | granzyme K (granzyme 3; tryptase II) |
| CD163 | Homo sapiens | CD163 molecule |
| CFHR5 | Homo sapiens | complement factor H-related 5 |
| MME | Homo sapiens | membrane metallo-endopeptidase |
| CCR1 | Homo sapiens | chemokine (C-C motif) receptor 1 |
| TNFSF15 | Homo sapiens | tumor necrosis factor (ligand) superfamily, member 15 |
| SLC4A1 | Homo sapiens | solute carrier family 4, anion exchanger, member 1 (erythrocyte membrane protein band 3, Diego blood group) |
| zap70 | Homo sapiens | zeta-chain (TCR) associated protein kinase 70kDa |
| ABCB1 | Homo sapiens | ATP-binding cassette, sub-family B (MDR/TAP), member 1 |
| CTSS | Homo sapiens | cathepsin S |
| LOC100133678 | Homo sapiens | similar to hCG2042724; similar to HLA class II histocompatibility antigen, DQ(1) alpha chain precursor (DC-4 alpha chain); major histocompatibility complex, class II, DQ alpha 1 |
| hla-dqa1 | Homo sapiens | similar to hCG2042724; similar to HLA class II histocompatibility antigen, DQ(1) alpha chain precursor (DC-4 alpha chain); major histocompatibility complex, class II, DQ alpha 1 |
| DEFA1B | Homo sapiens | defensin, alpha 1 |
| DEFA1 | Homo sapiens | defensin, alpha 1 |
| Prf1 | Homo sapiens | perforin 1 (pore forming protein) |
| lyg2 | Homo sapiens | lysozyme G-like 2 |
| EBF2 | Homo sapiens | early B-cell factor 2 |
| ICAM4 | Homo sapiens | intercellular adhesion molecule 4 (Landsteiner-Wiener blood group) |
| IL1F7 | Homo sapiens | interleukin 1 family, member 7 (zeta) |
| CAMP | Homo sapiens | cathelicidin antimicrobial peptide |
| CD207 | Homo sapiens | CD207 molecule, langerin |
| il2rb | Homo sapiens | interleukin 2 receptor, beta |
| IL12RB1 | Homo sapiens | interleukin 12 receptor, beta 1 |
| CFHR3 | Homo sapiens | complement factor H-related 3 |
| Cd6 | Homo sapiens | CD6 molecule |
| cd38 | Homo sapiens | CD38 molecule |
| CXCL1 | Homo sapiens | chemokine (C-X-C motif) ligand 1 (melanoma growth stimulating activity, alpha) |
| Cxcl14 | Homo sapiens | chemokine (C-X-C motif) ligand 14 |
| IL23R | Homo sapiens | interleukin 23 receptor |
| PF4 | Homo sapiens | platelet factor 4 |
| Cx3cl1 | Homo sapiens | chemokine (C-X3-C motif) ligand 1 |
| SELL | Homo sapiens | selectin L |
| IRF5 | Homo sapiens | interferon regulatory factor 5 |
| Ciita | Homo sapiens | class II, major histocompatibility complex, transactivator |
| WIPF1 | Homo sapiens | WAS/WASL interacting protein family, member 1 |
| FOXP3 | Homo sapiens | forkhead box P3 |
| FCRLA | Homo sapiens | Fc receptor-like A |
| DEFB123 | Homo sapiens | defensin, beta 123 |
| IL10RA | Homo sapiens | interleukin 10 receptor, alpha |
| IL13RA2 | Homo sapiens | interleukin 13 receptor, alpha 2 |
| NCF1C | Homo sapiens | neutrophil cytosolic factor 1; neutrophil cytosolic factor 1C pseudogene |
| ncf1 | Homo sapiens | neutrophil cytosolic factor 1; neutrophil cytosolic factor 1C pseudogene |
| KIR2DL1 | Homo sapiens | killer cell immunoglobulin-like receptor, two domains, long cytoplasmic tail, 2; killer cell immunoglobulin-like receptor, two domains, long cytoplasmic tail, 1; killer-cell lg-like receptor; killer cell immunoglobulin-like receptor, three domains, pseudogene 1 |
| KIR3DP1 | Homo sapiens | killer cell immunoglobulin-like receptor, two domains, long cytoplasmic tail, 2; killer cell immunoglobulin-like receptor, two domains, long cytoplasmic tail, 1; killer-cell Ig-like receptor; killer cell immunoglobulin-like receptor, three domains, pseudogene 1 |
| KIR2DL2 | Homo sapiens | killer cell immunoglobulin-like receptor, two domains, long cytoplasmic tail, 2; killer cell immunoglobulin-like receptor, two domains, long cytoplasmic tail, 1; killer-cell Ig-like receptor; killer cell immunoglobulin-like receptor, three domains, pseudogene 1 |
| Cd96 | Homo sapiens | CD96 molecule |
| SIGLEC6 | Homo sapiens | sialic acid binding Ig-like lectin 6 |
| IL9 | Homo sapiens | interleukin 9 |
| Il24 | Homo sapiens | interleukin 24 |
| IL20 | Homo sapiens | interleukin 20 |
| Ceacam6 | Homo sapiens | carcinoembryonic antigen-related cell adhesion molecule 6 (non-specific cross reacting antigen) |
| Cd101 | Homo sapiens | immunoglobulin superfamily, member 2 |
| CD300LF | Homo sapiens | CD300 molecule-like family member f |
| ITGAM | Homo sapiens | integrin, alpha M (complement component 3 receptor 3 subunit) |
| FCAMR | Homo sapiens | Fc receptor, IgA, IgM, high affinity |
| WASF3 | Homo sapiens | WAS protein family, member 3 |
| CD8B | Homo sapiens | CD8b molecule |
| Il27 | Homo sapiens | interleukin 27 |
| SLAMF6 | Homo sapiens | SLAM family member 6 |
| GZMA | Homo sapiens | granzyme A (granzyme 1, cytotoxic T-lymphocyte-associated serine esterase 3) |
| CXCL11 | Homo sapiens | chemokine (C-X-C motif) ligand 11 |
| CD4 | Homo sapiens | CD4 molecule |
| cell | Homo sapiens | chemokine (C-C motif) ligand 1 |
| CD244 | Homo sapiens | CD244 molecule, natural killer cell receptor 2B4 |
| Ly9 | Homo sapiens | lymphocyte antigen 9 |
| ccl8 | Homo sapiens | chemokine (C-C motif) ligand 8 |
| TCN2 | Homo sapiens | transcobalamin II; macrocytic anemia |
| CSF3 | Homo sapiens | colony stimulating factor 3 (granulocyte) |
| CD5 | Homo sapiens | CD5 molecule |
| CXCL5 | Homo sapiens | chemokine (C-X-C motif) ligand 5 |
| IL19 | Homo sapiens | interleukin 19 |
| masp2 | Homo sapiens | mannan-binding lectin serine peptidase 2 |
| PPBP | Homo sapiens | pro-platelet basic protein (chemokine (C-X-C motif) ligand 7) |
| C6 | Homo sapiens | complement component 6 |
| CMTM8 | Homo sapiens | CKLF-like MARVEL transmembrane domain containing 8 |
| SIGLEC1 | Homo sapiens | sialic acid binding Ig-like lectin 1, sialoadhesin |
| Il1a | Homo sapiens | interleukin 1, alpha |
| CCL20 | Homo sapiens | chemokine (C-C motif) ligand 20 |
| Mbl2 | Homo sapiens | mannose-binding lectin (protein C) 2, soluble (opsonic defect) |
| GYPB | Homo sapiens | glycophorin E; glycophorin B (MNS blood group) |
| GYPE | Homo sapiens | glycophorin E; glycophorin B (MNS blood group) |
| TNFRSF8 | Homo sapiens | tumor necrosis factor receptor superfamily, member 8 |
| Scgb3a1 | Homo sapiens | secretoglobin, family 3A, member 1 |
| irak3 | Homo sapiens | interleukin-1 receptor-associated kinase 3 |
| HTN3 | Homo sapiens | histatin 3 |
| CD248 | Homo sapiens | CD248 molecule, endosialin |
| C8A | Homo sapiens | complement component 8, alpha polypeptide |
| CD164L2 | Homo sapiens | CD164 sialomucin-like 2 |
| BANK1 | Homo sapiens | B-cell scaffold protein with ankyrin repeats 1 |
| IL12A | Homo sapiens | interleukin 12A (natural killer cell stimulatory factor 1, cytotoxic lymphocyte maturation factor 1, p35) |
| CHL1 | Homo sapiens | cell adhesion molecule with homology to L1CAM (close homolog of L1) |
| Spn | Homo sapiens | sialophorin |
| Traf1 | Homo sapiens | TNF receptor-associated factor 1 |
| il22 | Homo sapiens | interleukin 22 |
| Cxcr1 | Homo sapiens | interleukin 8 receptor, alpha |
| TEK | Homo sapiens | TEK tyrosine kinase, endothelial |
| FCRL5 | Homo sapiens | Fc receptor-like 5 |
| Cd300lg | Homo sapiens | CD300 molecule-like family member g |
| DOCK2 | Homo sapiens | dedicator of cytokinesis 2 |
| Cx3cr1 | Homo sapiens | chemokine (C-X3-C motif) receptor 1 |
| PGLYRP2 | Homo sapiens | peptidoglycan recognition protein 2 |
| WFDC12 | Homo sapiens | WAP four-disulfide core domain 12 |
| PLA2G7 | Homo sapiens | phospholipase A2, group VII (platelet-activating factor acetylhydrolase, plasma) |
| SLAMF7 | Homo sapiens | SLAM family member 7 |
| LY86 | Homo sapiens | lymphocyte antigen 86 |
| GZMM | Homo sapiens | granzyme M (lymphocyte met-ase 1) |
| PDCD1LG2 | Homo sapiens | programmed cell death 1 ligand 2 |
| C1QC | Homo sapiens | complement component 1, q subcomponent, C chain |
| GZMB | Homo sapiens | granzyme B (granzyme 2, cytotoxic T-lymphocyte-associated serine esterase 1) |
| DEFA5 | Homo sapiens | defensin, alpha 5, Paneth cell-specific |
| dcd | Homo sapiens | dermcidin |
| Ccl2 | Homo sapiens | chemokine (C-C motif) ligand 2 |
| CLEC4A | Homo sapiens | C-type lectin domain family 4, member A |
| IL1RAPL1 | Homo sapiens | interleukin 1 receptor accessory protein-like 1 |
| IL26 | Homo sapiens | interleukin 26 |
| C1ql4 | Homo sapiens | complement component 1, q subcomponent-like 4 |
| IFNG | Homo sapiens | interferon, gamma |
| CEACAM5 | Homo sapiens | carcinoembryonic antigen-related cell adhesion molecule 5 |
| TNFRSF18 | Homo sapiens | tumor necrosis factor receptor superfamily, member 18 |
| POMC | Homo sapiens | proopiomelanocortin |
| PTPN22 | Homo sapiens | protein tyrosine phosphatase, non-receptor type 22 (lymphoid) |
| Itgax | Homo sapiens | integrin, alpha X (complement component 3 receptor 4 subunit) |
| DEFB4A | Homo sapiens | defensin, beta 4 |
| Ncam1 | Homo sapiens | neural cell adhesion molecule 1 |
| CD7 | Homo sapiens | CD7 molecule |
| TLR5 | Homo sapiens | toll-like receptor 5 |
| GNLY | Homo sapiens | granulysin |
| Gpr183 | Homo sapiens | G protein-coupled receptor 183 |
| Ncf4 | Homo sapiens | neutrophil cytosolic factor 4, 40kDa |
| CSF2RB | Homo sapiens | colony stimulating factor 2 receptor, beta, low-affinity (granulocyte-macrophage) |
| Defa4 | Homo sapiens | defensin, alpha 4, corticostatin |
| TNFRSF13B | Homo sapiens | tumor necrosis factor receptor superfamily, member 13B |
| DEFA6 | Homo sapiens | defensin, alpha 6, Paneth cell-specific |
| TLR1 | Homo sapiens | toll-like receptor 1 |
| AICDA | Homo sapiens | activation-induced cytidine deaminase |
| PTAFR | Homo sapiens | platelet-activating factor receptor |
| CCL13 | Homo sapiens | chemokine (C-C motif) ligand 13 |
| ALK | Homo sapiens | anaplastic lymphoma receptor tyrosine kinase |
| HRH2 | Homo sapiens | histamine receptor H2 |
| C3AR1 | Homo sapiens | complement component 3a receptor 1 |
| XCL1 | Homo sapiens | chemokine (C motif) ligand 1 |
| IFNB1 | Homo sapiens | interferon, beta 1, fibroblast |
| TNFRSF13C | Homo sapiens | tumor necrosis factor receptor superfamily, member 13C |
| CD84 | Homo sapiens | CD84 molecule |
| XCL2 | Homo sapiens | chemokine (C motif) ligand 2 |
| TLR2 | Homo sapiens | toll-like receptor 2 |
| CCL7 | Homo sapiens | chemokine (C-C motif) ligand 7 |
| Vpreb1 | Homo sapiens | pre-B lymphocyte 1 |
| CLEC4C | Homo sapiens | C-type lectin domain family 4, member C |
| NFATC2 | Homo sapiens | nuclear factor of activated T-cells, cytoplasmic, calcineurin-dependent 2 |
| LAX1 | Homo sapiens | lymphocyte transmembrane adaptor 1 |
| Cfi | Homo sapiens | complement factor I |
| Icp2 | Homo sapiens | lymphocyte cytosolic protein 2 (SH2 domain containing leukocyte protein of 76kDa) |
| FLT3 | Homo sapiens | fms-related tyrosine kinase 3 |
| IL2RA | Homo sapiens | interleukin 2 receptor, alpha |
| CD74 | Homo sapiens | CD74 molecule, major histocompatibility complex, class II invariant chain |
| CD93 | Homo sapiens | CD93 molecule |
| pdgfra | Homo sapiens | platelet-derived growth factor receptor, alpha polypeptide |
| CFHR4 | Homo sapiens | complement factor H-related 4 |
| CTSG | Homo sapiens | cathepsin G |
| Sirpa | Homo sapiens | signal-regulatory protein alpha |
| IL8 | Homo sapiens | interleukin 8 |
| CD36 | Homo sapiens | CD36 molecule (thrombospondin receptor) |
| MYLK | Homo sapiens | myosin light chain kinase |
| Hamp | Homo sapiens | hepcidin antimicrobial peptide |
| IL1RAPL2 | Homo sapiens | interleukin 1 receptor accessory protein-like 2 |
| SERPING1 | Homo sapiens | serpin peptidase inhibitor, clade G (C1 inhibitor), member 1 |
| cmklr1 | Homo sapiens | chemokine-like receptor 1 |
| CCR6 | Homo sapiens | cyclin L2; chemokine (C-C motif) receptor 6 |
| Ccnl2 | Homo sapiens | cyclin L2; chemokine (C-C motif) receptor 6 |
| btk | Homo sapiens | Bruton agammaglobulinemia tyrosine kinase |
| ITGA4 | Homo sapiens | integrin, alpha 4 (antigen CD49D, alpha 4 subunit of VLA-4 receptor) |
| CD180 | Homo sapiens | CD180 molecule |
| NCR2 | Homo sapiens | natural cytotoxicity triggering receptor 2 |
| PRG2 | Homo sapiens | proteoglycan 2, bone marrow (natural killer cell activator, eosinophil granule major basic protein) |
| KIR2DL4 | Homo sapiens | killer cell immunoglobulin-like receptor, two domains, long cytoplasmic tail, 4 |
| HRH4 | Homo sapiens | histamine receptor H4 |
| Cd2 | Homo sapiens | CD2 molecule |
| RHAG | Homo sapiens | Rh-associated glycoprotein |
| LILRA1 | Homo sapiens | leukocyte immunoglobulin-like receptor, subfamily A (with TM domain), member 1 |
| LILRB1 | Homo sapiens | leukocyte immunoglobulin-like receptor, subfamily B (with TM and ITIM domains), member 1 |
| SELE | Homo sapiens | selectin E |
| Il1rl2 | Homo sapiens | interleukin 1 receptor-like 2 |
| Cxcl9 | Homo sapiens | chemokine (C-X-C motif) ligand 9 |
| CD27 | Homo sapiens | CD27 molecule |
| IL1R2 | Homo sapiens | interleukin 1 receptor, type II |
| Il28a | Homo sapiens | interleukin 28A (interferon, lambda 2) |
| EPO | Homo sapiens | erythropoietin |
| Clec4e | Homo sapiens | C-type lectin domain family 4, member E |
| LOC727787 | Homo sapiens | killer cell immunoglobulin-like receptor, three domains, long cytoplasmic tail, 2; similar to killer cell immunoglobulin-like receptor 3DL2 precursor (MHC class I NK cell receptor) (Natural killer-associated transcript 4) (NKAT-4) (p70 natural killer cell receptor clone CL-5) (CD158k antigen) |
| KIR3DL2 | Homo sapiens | killer cell immunoglobulin-like receptor, three domains, long cytoplasmic tail, 2; similar to killer cell immunoglobulin-like receptor 3DL2 precursor (MHC class I NK cell receptor) (Natural killer-associated transcript 4) (NKAT-4) (p70 natural killer cell receptor clone CL-5) (CD158k antigen) |
| C7 | Homo sapiens | complement component 7 |
| Fcgr3a | Homo sapiens | Fc fragment of IgG, low affinity IIIa, receptor (CD16a) |
| Cmtm3 | Homo sapiens | CKLF-like MARVEL transmembrane domain containing 3 |
| CLEC4D | Homo sapiens | C-type lectin domain family 4, member D |
| CMTM2 | Homo sapiens | CKLF-like MARVEL transmembrane domain containing 2 |
| CD300E | Homo sapiens | CD300e molecule |
| KLRB1 | Homo sapiens | killer cell lectin-like receptor subfamily B, member 1 |
| IL12B | Homo sapiens | interleukin 12B (natural killer cell stimulatory factor 2, cytotoxic lymphocyte maturation factor 2, p40) |
| IL17RD | Homo sapiens | interleukin 17 receptor D |
| Il31ra | Homo sapiens | interleukin 31 receptor A |
| Batf | Homo sapiens | basic leucine zipper transcription factor, ATF-like |
| LTF | Homo sapiens | lactotransferrin |
| csf3r | Homo sapiens | colony stimulating factor 3 receptor (granulocyte) |
| Il17f | Homo sapiens | interleukin 17F |
| IL17A | Homo sapiens | interleukin 17A |
| IL17C | Homo sapiens | interleukin 17C |
| TNFSF4 | Homo sapiens | tumor necrosis factor (ligand) superfamily, member 4 |
| fcgr1a | Homo sapiens | Fc fragment of IgG, high affinity Ic, receptor (CD64); Fc fragment of IgG, high affinity Ia, receptor (CD64) |
| FCGR1C | Homo sapiens | Fc fragment of IgG, high affinity Ic, receptor (CD64); Fc fragment of IgG, high affinity Ia, receptor (CD64) |
| Ly75 | Homo sapiens | CD302 molecule; lymphocyte antigen 75 |
| CD302 | Homo sapiens | CD302 molecule; lymphocyte antigen 75 |
| crp | Homo sapiens | C-reactive protein, pentraxin-related |
| CD226 | Homo sapiens | CD226 molecule |
| CD1B | Homo sapiens | CD1b molecule |
| CXCR2 | Homo sapiens | interleukin 8 receptor, beta |
| CXCL13 | Homo sapiens | chemokine (C-X-C motif) ligand 13 |
| Ccr3 | Homo sapiens | chemokine (C-C motif) receptor 3 |
| CD1E | Homo sapiens | CD1 e molecule |
| PIK3CG | Homo sapiens | phosphoinositide-3-kinase, catalytic, gamma polypeptide |
| LILRB4 | Homo sapiens | leukocyte immunoglobulin-like receptor, subfamily B (with TM and ITIM domains), member 4 |
| FCGR2A | Homo sapiens | Fc fragment of IgG, low affinity IIa, receptor (CD32) |
| BCAM | Homo sapiens | basal cell adhesion molecule (Lutheran blood group) |
| Csf2 | Homo sapiens | colony stimulating factor 2 (granulocyte-macrophage) |
| Ccl27 | Homo sapiens | chemokine (C-C motif) ligand 27 |
| Pglyrp3 | Homo sapiens | peptidoglycan recognition protein 3 |
| CD1C | Homo sapiens | CD1c molecule |
| Colec12 | Homo sapiens | collectin sub-family member 12 |
| CD1A | Homo sapiens | CD1a molecule |
| IL21 | Homo sapiens | interleukin 21 |
| MS4A3 | Homo sapiens | membrane-spanning 4-domains, subfamily A, member 3 (hematopoietic cell-specific) |
| Lilra5 | Homo sapiens | leukocyte immunoglobulin-like receptor, subfamily A (with TM domain), member 5 |
| CR1 | Homo sapiens | complement component (3b/4b) receptor 1 (Knops blood group) |
| PPP3R2 | Homo sapiens | protein phosphatase 3 (formerly 2B), regulatory subunit B, beta isoform |
| CXCR3 | Homo sapiens | chemokine (C-X-C motif) receptor 3 |
| Pdcd1 | Homo sapiens | programmed cell death 1 |
| LILRB2 | Homo sapiens | leukocyte immunoglobulin-like receptor, subfamily B (with TM and ITIM domains), member 2 |
| ccr4 | Homo sapiens | chemokine (C-C motif) receptor 4 |
| RNASE7 | Homo sapiens | ribonuclease, RNase A family, 7 |
| Msr1 | Homo sapiens | macrophage scavenger receptor 1 |
| ccl25 | Homo sapiens | chemokine (C-C motif) ligand 25 |
| FASLG | Homo sapiens | Fas ligand (TNF superfamily, member 6) |
| CD1D | Homo sapiens | CD1d molecule |
| CCL28 | Homo sapiens | chemokine (C-C motif) ligand 28 |
| A2ML1 | Homo sapiens | alpha-2-macroglobulin-like 1 |
| CEACAM8 | Homo sapiens | carcinoembryonic antigen-related cell adhesion molecule 8 |
| FGFR2 | Homo sapiens | fibroblast growth factor receptor 2 |
| Cd5l | Homo sapiens | CD5 molecule-like |
| Fcar | Homo sapiens | Fc fragment of IgA, receptor for |
| DEFB106A | Homo sapiens | defensin, beta 106A; defensin, beta 106B |
| DEFB106B | Homo sapiens | defensin, beta 106A; defensin, beta 106B |
| IRF8 | Homo sapiens | interferon regulatory factor 8 |
| Igj | Homo sapiens | immunoglobulin J polypeptide, linker protein for immunoglobulin alpha and mu polypeptides |
| ACE | Homo sapiens | angiotensin I converting enzyme (peptidyl-dipeptidase A) 1 |
| MPO | Homo sapiens | myeloperoxidase |
| CD86 | Homo sapiens | CD86 molecule |
| CD40 | Homo sapiens | CD40 molecule, TNF receptor superfamily member 5 |
| CD209 | Homo sapiens | CD209 molecule |
| KIR3DS1 | Homo sapiens | killer cell immunoglobulin-like receptor, three domains, short cytoplasmic tail, 1; killer cell immunoglobulin-like receptor, three domains, long cytoplasmic tail, 1 |
| Kir3dl1 | Homo sapiens | killer cell immunoglobulin-like receptor, three domains, short cytoplasmic tail, 1; killer cell immunoglobulin-like receptor, three domains, long cytoplasmic tail, 1 |
| mpl | Homo sapiens | myeloproliferative leukemia virus oncogene |
| Tnfrsf4 | Homo sapiens | tumor necrosis factor receptor superfamily, member 4 |
| IL17B | Homo sapiens | interleukin 17B |
| PSTPIP1 | Homo sapiens | proline-serine-threonine phosphatase interacting protein 1 |
| DPP4 | Homo sapiens | dipeptidyl-peptidase 4 |
| AIRE | Homo sapiens | autoimmune regulator |
| Cd53 | Homo sapiens | CD53 molecule |
| tbx21 | Homo sapiens | T-box 21 |
| THBD | Homo sapiens | thrombomodulin |
| CCR7 | Homo sapiens | chemokine (C-C motif) receptor 7 |
| CLEC10A | Homo sapiens | C-type lectin domain family 10, member A |
| CD274 | Homo sapiens | CD274 molecule |
| Cxcl6 | Homo sapiens | chemokine (C-X-C motif) ligand 6 (granulocyte chemotactic protein 2) |
| CD69 | Homo sapiens | CD69 molecule |
| CD22 | Homo sapiens | CD22 molecule |
| IL23A | Homo sapiens | interleukin 23, alpha subunit p19 |
| NOD2 | Homo sapiens | nucleotide-binding oligomerization domain containing 2 |
| TNFRSF9 | Homo sapiens | tumor necrosis factor receptor superfamily, member 9 |
| LPO | Homo sapiens | lactoperoxidase |
| nfatc1 | Homo sapiens | nuclear factor of activated T-cells, cytoplasmic, calcineurin-dependent 1 |
| CMTM5 | Homo sapiens | CKLF-like MARVEL transmembrane domain containing 5 |
| IL25 | Homo sapiens | interleukin 25 |
| Lair1 | Homo sapiens | leukocyte-associated immunoglobulin-like receptor 1 |
| LAIR2 | Homo sapiens | leukocyte-associated immunoglobulin-like receptor 2 |
| NOS2 | Homo sapiens | nitric oxide synthase 2, inducible |
| NCR3 | Homo sapiens | natural cytotoxicity triggering receptor 3 |
| SOD3 | Homo sapiens | superoxide dismutase 3, extracellular |
| IL28B | Homo sapiens | interleukin 28B (interferon, lambda 3) |
| RHD | Homo sapiens | Rh blood group, D antigen |
| KLRD1 | Homo sapiens | killer cell lectin-like receptor subfamily D, member 1 |
| Tnfsf14 | Homo sapiens | tumor necrosis factor (ligand) superfamily, member 14 |
| IL9R | Homo sapiens | interleukin 9 receptor |
| il10 | Homo sapiens | interleukin 10 |
| CCR10 | Homo sapiens | chemokine (C-C motif) receptor 10 |
| CR2 | Homo sapiens | complement component (3d/Epstein Barr virus) receptor 2 |
| DEFB105B | Homo sapiens | defensin, beta 105A; defensin, beta 105B |
| DEFB105A | Homo sapiens | defensin, beta 105A; defensin, beta 105B |
| LYZ | Homo sapiens | lysozyme (renal amyloidosis) |
| DEFB103A | Homo sapiens | defensin, beta 103B; defensin, beta 103A |
| DEFB103B | Homo sapiens | defensin, beta 103B; defensin, beta 103A |
| CMTM7 | Homo sapiens | CKLF-like MARVEL transmembrane domain containing 7 |
| LILRB5 | Homo sapiens | leukocyte immunoglobulin-like receptor, subfamily B (with TM and ITIM domains), member 5 |
| IL4 | Homo sapiens | interleukin 4 |

**Table 3**

| **Gene Symbol** | **Species** | **Gene Name** |
|---|---|---|
| CD300LB | Homo sapiens | CD300 molecule-like family member b |
| C1QB | Homo sapiens | complement component 1, q subcomponent, B chain |
| CXCR5 | Homo sapiens | chemokine (C-X-C motif) receptor 5 |
| FUT3 | Homo sapiens | fucosyltransferase 3 (galactoside 3(4)-L-fucosyltransferase, Lewis blood group) |
| EBF1 | Homo sapiens | early B-cell factor 1 |
| Sh2d1a | Homo sapiens | SH2 domain protein 1A |
| tlr9 | Homo sapiens | toll-like receptor 9 |
| LAMP3 | Homo sapiens | lysosomal-associated membrane protein 3 |
| TCF7 | Homo sapiens | transcription factor 7 (T-cell specific, HMG-box) |
| IL1F6 | Homo sapiens | interleukin 1 family, member 6 (epsilon) |
| IL1F5 | Homo sapiens | interleukin 1 family, member 5 (delta) |
| IL1F10 | Homo sapiens | interleukin 1 family, member 10 (theta) |
| NFATC4 | Homo sapiens | nuclear factor of activated T-cells, cytoplasmic, calcineurin-dependent 4 |
| DARC | Homo sapiens | Duffy blood group, chemokine receptor |
| CCR2 | Homo sapiens | chemokine (C-C motif) receptor 2 |
| ITGAL | Homo sapiens | integrin, alpha L (antigen CD11A (p180), lymphocyte function-associated antigen 1; alpha polypeptide) |
| C1QL2 | Homo sapiens | complement component 1, q subcomponent-like 2 |
| Gypa | Homo sapiens | glycophorin A (MNS blood group) |
| Il31 | Homo sapiens | interleukin 31 |
| Ptprc | Homo sapiens | protein tyrosine phosphatase, receptor type, C |
| ITGAD | Homo sapiens | integrin, alpha D |
| HLA-DQB1 | Homo sapiens | major histocompatibility complex, class II, DQ beta 1; similar to major histocompatibility complex, class II, DQ beta 1 |
| LOC100133583 | Homo sapiens | major histocompatibility complex, class II, DQ beta 1; similar to major histocompatibility complex, class II, DQ beta 1 |
| C1qa | Homo sapiens | complement component 1, q subcomponent, A chain |
| IL22RA2 | Homo sapiens | interleukin 22 receptor, alpha 2 |
| CD300A | Homo sapiens | CD300a molecule |
| Tnfsf8 | Homo sapiens | tumor necrosis factor (ligand) superfamily, member 8 |
| Cd300c | Homo sapiens | CD300c molecule |
| Cytl1 | Homo sapiens | cytokine-like 1 |
| Ctla4 | Homo sapiens | cytotoxic T-lymphocyte-associated protein 4 |
| PLA2R1 | Homo sapiens | phospholipase A2 receptor 1, 180kDa |
| Xcr1 | Homo sapiens | chemokine (C motif) receptor 1 |
| STAT4 | Homo sapiens | signal transducer and activator of transcription 4 |
| C1QL3 | Homo sapiens | complement component 1, q subcomponent-like 3 |
| Ncr1 | Homo sapiens | natural cytotoxicity triggering receptor 1 |
| PROM1 | Homo sapiens | prominin 1 |
| CCRL1 | Homo sapiens | chemokine (C-C motif) receptor-like 1 |
| CFP | Homo sapiens | complement factor properdin |
| HLA-DRB5 | Homo sapiens | major histocompatibility complex, class II, DR beta 5 |
| IGLL1 | Homo sapiens | immunoglobulin lambda-like polypeptide 1 |
| CD14 | Homo sapiens | CD14 molecule |
| FCER2 | Homo sapiens | Fc fragment of IgE, low affinity II, receptor for (CD23) |
| itga2b | Homo sapiens | integrin, alpha 2b (platelet glycoprotein IIb of IIb/IIIa complex, antigen CD41) |
| CCR9 | Homo sapiens | chemokine (C-C motif) receptor 9 |
| CCL24 | Homo sapiens | chemokine (C-C motif) ligand 24 |
| Cd3g | Homo sapiens | CD3g molecule, gamma (CD3-TCR complex) |
| CCR5 | Homo sapiens | chemokine (C-C motif) receptor 5 |
| KDR | Homo sapiens | kinase insert domain receptor (a type III receptor tyrosine kinase) |
| Cd8a | Homo sapiens | CD8a molecule |
| HLA-DOA | Homo sapiens | major histocompatibility complex, class II, DO alpha |
| Pglyrp1 | Homo sapiens | peptidoglycan recognition protein 1 |
| Cd79b | Homo sapiens | CD79b molecule, immunoglobulin-associated beta |
| Cr1l | Homo sapiens | complement component (3b/4b) receptor 1-like |
| IL1RN | Homo sapiens | interleukin 1 receptor antagonist |
| Il1f9 | Homo sapiens | interleukin 1 family, member 9 |
| CEACAM1 | Homo sapiens | carcinoembryonic antigen-related cell adhesion molecule 1 (biliary glycoprotein) |
| LOC652799 | Homo sapiens | similar to Mast/stem cell growth factor receptor precursor (SCFR) (Proto-oncogene tyrosine-protein kinase Kit) (c-kit) (CD117 antigen); v-kit Hardy-Zuckerman 4 feline sarcoma viral oncogene homolog |
| LOC653882 | Homo sapiens | similar to Mast/stem cell growth factor receptor precursor (SCFR) (Proto-oncogene tyrosine-protein kinase Kit) (c-kit) (CD117 antigen); v-kit Hardy-Zuckerman 4 feline sarcoma viral oncogene homolog |
| KIT | Homo sapiens | similar to Mast/stem cell growth factor receptor precursor (SCFR) (Proto-oncogene tyrosine-protein kinase Kit) (c-kit) (CD117 antigen); v-kit Hardy-Zuckerman 4 feline sarcoma viral oncogene homolog |
| FCER1A | Homo sapiens | Fc fragment of IgE, high affinity I, receptor for; alpha polypeptide |
| IL12RB2 | Homo sapiens | interleukin 12 receptor, beta 2 |
| rac2 | Homo sapiens | ras-related C3 botulinum toxin substrate 2 (rho family, small GTP binding protein Rac2) |
| CD200R1L | Homo sapiens | CD200 receptor 1-like |
| BTLA | Homo sapiens | B and T lymphocyte associated |
| KEL | Homo sapiens | Kell blood group, metallo-endopeptidase |
| Siglec15 | Homo sapiens | sialic acid binding Ig-like lectin 15 |
| MS4A1 | Homo sapiens | membrane-spanning 4-domains, subfamily A, member 1 |
| SIGLEC5 | Homo sapiens | sialic acid binding Ig-like lectin 5 |
| Bst1 | Homo sapiens | bone marrow stromal cell antigen 1 |
| Ms4a5 | Homo sapiens | membrane-spanning 4-domains, subfamily A, member 5 |
| GP9 | Homo sapiens | glycoprotein IX (platelet) |
| IL29 | Homo sapiens | interleukin 29 (interferon, lambda 1) |
| LILRA4 | Homo sapiens | leukocyte immunoglobulin-like receptor, subfamily A (with TM domain), member 4 |
| CD40LG | Homo sapiens | CD40 ligand |
| GYPC | Homo sapiens | glycophorin C (Gerbich blood group) |
| CCL22 | Homo sapiens | chemokine (C-C motif) ligand 22 |
| GP5 | Homo sapiens | glycoprotein V (platelet) |
| IL16 | Homo sapiens | interleukin 16 (lymphocyte chemoattractant factor) |
| CLEC4M | Homo sapiens | C-type lectin domain family 4, member M |
| EPX | Homo sapiens | eosinophil peroxidase |
| Vcam1 | Homo sapiens | vascular cell adhesion molecule 1 |
| Cd247 | Homo sapiens | CD247 molecule |
| LILRA6 | Homo sapiens | leukocyte immunoglobulin-like receptor, subfamily A (with TM domain), member 6 |
| HLA-DRA | Homo sapiens | major histocompatibility complex, class II, DR alpha |
| IL2 | Homo sapiens | interleukin 2 |
| Ccl17 | Homo sapiens | chemokine (C-C motif) ligand 17 |
| Il3 | Homo sapiens | interleukin 3 (colony-stimulating factor, multiple) |
| plxnc1 | Homo sapiens | plexin C1 |
| CCL19 | Homo sapiens | chemokine (C-C motif) ligand 19 |
| CXCR6 | Homo sapiens | chemokine (C-X-C motif) receptor 6 |
| CD160 | Homo sapiens | CD160 molecule |
| C8B | Homo sapiens | complement component 8, beta polypeptide |
| Cd48 | Homo sapiens | CD48 molecule |
| Tnfsf11 | Homo sapiens | tumor necrosis factor (ligand) superfamily, member 11 |
| SLAMF1 | Homo sapiens | signaling lymphocytic activation molecule family member 1 |
| CD28 | Homo sapiens | CD28 molecule |
| Fcgr2b | Homo sapiens | Fc fragment of IgG, low affinity IIb, receptor (CD32); Fc fragment of IgG, low affinity IIc, receptor for (CD32) |
| FCGR2C | Homo sapiens | Fc fragment of IgG, low affinity IIb, receptor (CD32); Fc fragment of IgG, low affinity IIc, receptor for (CD32) |
| POU2AF1 | Homo sapiens | POU class 2 associating factor 1 |
| CLEC6A | Homo sapiens | C-type lectin domain family 6, member A |
| CLEC12A | Homo sapiens | C-type lectin domain family 12, member A; C-type lectin domain family 12, member B |
| CLEC12B | Homo sapiens | C-type lectin domain family 12, member A; C-type lectin domain family 12, member B |
| Cfhr1 | Homo sapiens | complement factor H-related 1 |
| TLR4 | Homo sapiens | toll-like receptor 4 |
| IFIT1B | Homo sapiens | interferon-induced protein with tetratricopeptide repeats 1-like |
| CD3D | Homo sapiens | CD3d molecule, delta (CD3-TCR complex) |
| ANPEP | Homo sapiens | alanyl (membrane) aminopeptidase |
| C1 qtnf7 | Homo sapiens | C1q and tumor necrosis factor related protein 7 |
| SELP | Homo sapiens | selectin P (granule membrane protein 140kDa, antigen CD62) |
| Cd19 | Homo sapiens | CD19 molecule |
| Ceacam3 | Homo sapiens | carcinoembryonic antigen-related cell adhesion molecule 3 |
| ICOS | Homo sapiens | inducible T-cell co-stimulator |
| C1QTNF2 | Homo sapiens | C1q and tumor necrosis factor related protein 2 |
| TNFSF13B | Homo sapiens | tumor necrosis factor (ligand) superfamily, member 13b |
| CD3E | Homo sapiens | CD3e molecule, epsilon (CD3-TCR complex) |
| THY1 | Homo sapiens | Thy-1 cell surface antigen |
| IL18RAP | Homo sapiens | interleukin 18 receptor accessory protein |
| LY96 | Homo sapiens | lymphocyte antigen 96 |
| LCK | Homo sapiens | lymphocyte-specific protein tyrosine kinase |
| CEBPE | Homo sapiens | CCAAT/enhancer binding protein (C/EBP), epsilon |
| DEFB119 | Homo sapiens | defensin, beta 119 |
| TNFRSF1B | Homo sapiens | tumor necrosis factor receptor superfamily, member 1 B |
| FCER1G | Homo sapiens | Fc fragment of IgE, high affinity I, receptor for; gamma polypeptide |
| IL1RL1 | Homo sapiens | interleukin 1 receptor-like 1 |
| CD80 | Homo sapiens | CD80 molecule |
| TNFRSF17 | Homo sapiens | tumor necrosis factor receptor superfamily, member 17 |
| CD34 | Homo sapiens | CD34 molecule |
| tal1 | Homo sapiens | T-cell acute lymphocytic leukemia 1 |
| CD200R1 | Homo sapiens | CD200 receptor 1 |
| C1qtnf4 | Homo sapiens | C1q and tumor necrosis factor related protein 4 |
| CLEC7A | Homo sapiens | C-type lectin domain family 7, member A |
| FCGR3B | Homo sapiens | Fc fragment of IgG, low affinity IIIb, receptor (CD16b) |
| IL21R | Homo sapiens | interleukin 21 receptor |
| KIR2DS2 | Homo sapiens | killer cell immunoglobulin-like receptor, two domains, short cytoplasmic tail, 2; killer cell immunoglobulin-like receptor, two domains, short cytoplasmic tail, 4 |
| KIR2DS4 | Homo sapiens | killer cell immunoglobulin-like receptor, two domains, short cytoplasmic tail, 2; killer cell immunoglobulin-like receptor, two domains, short cytoplasmic tail, 4 |
| CXCL12 | Homo sapiens | chemokine (C-X-C motif) ligand 12 (stromal cell-derived factor 1) |
| Ccl11 | Homo sapiens | chemokine (C-C motif) ligand 11 |
| Cd79a | Homo sapiens | CD79a molecule, immunoglobulin-associated alpha |
| IL13 | Homo sapiens | interleukin 13 |
| GZMK | Homo sapiens | granzyme K (granzyme 3; tryptase II) |
| CD163 | Homo sapiens | CD163 molecule |
| CFHR5 | Homo sapiens | complement factor H-related 5 |
| MME | Homo sapiens | membrane metallo-endopeptidase |
| CCR1 | Homo sapiens | chemokine (C-C motif) receptor 1 |
| TNFSF15 | Homo sapiens | tumor necrosis factor (ligand) superfamily, member 15 |
| SLC4A1 | Homo sapiens | solute carrier family 4, anion exchanger, member 1 (erythrocyte membrane protein band 3, Diego blood group) |
| zap70 | Homo sapiens | zeta-chain (TCR) associated protein kinase 70kDa |
| ABCB1 | Homo sapiens | ATP-binding cassette, sub-family B (MDR/TAP), member 1 |
| CTSS | Homo sapiens | cathepsin S |
| LOC100133678 | Homo sapiens | similar to hCG2042724; similar to HLA class II histocompatibility antigen, DQ(1) alpha chain precursor (DC-4 alpha chain); major histocompatibility complex, class II, DQ alpha 1 |
| hla-dqa1 | Homo sapiens | similar to hCG2042724; similar to HLA class II histocompatibility antigen, DQ(1) alpha chain precursor (DC-4 alpha chain); major histocompatibility complex, class II, DQ alpha 1 |
| DEFA1B | Homo sapiens | defensin, alpha 1 |
| DEFA1 | Homo sapiens | defensin, alpha 1 |
| Prf1 | Homo sapiens | perforin 1 (pore forming protein) |
| Iyg2 | Homo sapiens | lysozyme G-like 2 |
| EBF2 | Homo sapiens | early B-cell factor 2 |
| ICAM4 | Homo sapiens | intercellular adhesion molecule 4 (Landsteiner-Wiener blood group) |
| IL1F7 | Homo sapiens | interleukin 1 family, member 7 (zeta) |
| CAMP | Homo sapiens | cathelicidin antimicrobial peptide |
| CD207 | Homo sapiens | CD207 molecule, langerin |
| il2rb | Homo sapiens | interleukin 2 receptor, beta |
| IL12RB1 | Homo sapiens | interleukin 12 receptor, beta 1 |
| CFHR3 | Homo sapiens | complement factor H-related 3 |
| Cd6 | Homo sapiens | CD6 molecule |
| cd38 | Homo sapiens | CD38 molecule |
| CXCL1 | Homo sapiens | chemokine (C-X-C motif) ligand 1 (melanoma growth stimulating activity, alpha) |
| Cxcl14 | Homo sapiens | chemokine (C-X-C motif) ligand 14 |
| IL23R | Homo sapiens | interleukin 23 receptor |
| PF4 | Homo sapiens | platelet factor 4 |
| Cx3cl1 | Homo sapiens | chemokine (C-X3-C motif) ligand 1 |
| SELL | Homo sapiens | selectin L |
| IRF5 | Homo sapiens | interferon regulatory factor 5 |
| Ciita | Homo sapiens | class II, major histocompatibility complex, transactivator |
| WIPF1 | Homo sapiens | WAS/WASL interacting protein family, member 1 |
| FOXP3 | Homo sapiens | forkhead box P3 |
| FCRLA | Homo sapiens | Fc receptor-like A |
| DEFB123 | Homo sapiens | defensin, beta 123 |
| IL10RA | Homo sapiens | interleukin 10 receptor, alpha |
| IL13RA2 | Homo sapiens | interleukin 13 receptor, alpha 2 |
| NCF1C | Homo sapiens | neutrophil cytosolic factor 1; neutrophil cytosolic factor 1C pseudogene |
| ncf1 | Homo sapiens | neutrophil cytosolic factor 1; neutrophil cytosolic factor 1C pseudogene |
| KIR2DL1 | Homo sapiens | killer cell immunoglobulin-like receptor, two domains, long cytoplasmic tail, 2; killer cell immunoglobulin-like receptor, two domains, long cytoplasmic tail, 1; killer-cell Ig-like receptor; killer cell immunoglobulin-like receptor, three domains, pseudogene 1 |
| KIR3DP1 | Homo sapiens | killer cell immunoglobulin-like receptor, two domains, long cytoplasmic tail, 2; killer cell immunoglobulin-like receptor, two domains, long cytoplasmic tail, 1; killer-cell Ig-like receptor; killer cell immunoglobulin-like receptor, three domains, pseudogene 1 |
| KIR2DL2 | Homo sapiens | killer cell immunoglobulin-like receptor, two domains, long cytoplasmic tail, 2; killer cell immunoglobulin-like receptor, two domains, long cytoplasmic tail, 1; killer-cell Ig-like receptor; killer cell immunoglobulin-like receptor, three domains, pseudogene 1 |
| Cd96 | Homo sapiens | CD96 molecule |
| SIGLEC6 | Homo sapiens | sialic acid binding Ig-like lectin 6 |
| IL9 | Homo sapiens | interleukin 9 |
| II24 | Homo sapiens | interleukin 24 |
| IL20 | Homo sapiens | interleukin 20 |
| Ceacam6 | Homo sapiens | carcinoembryonic antigen-related cell adhesion molecule 6 (nonspecific cross reacting antigen) |
| Cd101 | Homo sapiens | immunoglobulin superfamily, member 2 |
| CD300LF | Homo sapiens | CD300 molecule-like family member f |
| ITGAM | Homo sapiens | integrin, alpha M (complement component 3 receptor 3 subunit) |
| FCAMR | Homo sapiens | Fc receptor, IgA, IgM, high affinity |
| WASF3 | Homo sapiens | WAS protein family, member 3 |
| CD8B | Homo sapiens | CD8b molecule |
| Il27 | Homo sapiens | interleukin 27 |
| SLAMF6 | Homo sapiens | SLAM family member 6 |
| GZMA | Homo sapiens | granzyme A (granzyme 1, cytotoxic T-lymphocyte-associated serine esterase 3) |
| CXCL11 | Homo sapiens | chemokine (C-X-C motif) ligand 11 |
| CD4 | Homo sapiens | CD4 molecule |
| ccl1 | Homo sapiens | chemokine (C-C motif) ligand 1 |
| CD244 | Homo sapiens | CD244 molecule, natural killer cell receptor 2B4 |
| Ly9 | Homo sapiens | lymphocyte antigen 9 |
| ccl8 | Homo sapiens | chemokine (C-C motif) ligand 8 |
| TCN2 | Homo sapiens | transcobalamin II; macrocytic anemia |
| CSF3 | Homo sapiens | colony stimulating factor 3 (granulocyte) |
| CD5 | Homo sapiens | CD5 molecule |
| CXCL5 | Homo sapiens | chemokine (C-X-C motif) ligand 5 |
| IL19 | Homo sapiens | interleukin 19 |
| masp2 | Homo sapiens | mannan-binding lectin serine peptidase 2 |
| PPBP | Homo sapiens | pro-platelet basic protein (chemokine (C-X-C motif) ligand 7) |
| C6 | Homo sapiens | complement component 6 |
| CMTM8 | Homo sapiens | CKLF-like MARVEL transmembrane domain containing 8 |
| SIGLEC1 | Homo sapiens | sialic acid binding Ig-like lectin 1, sialoadhesin |
| Il1a | Homo sapiens | interleukin 1, alpha |
| CCL20 | Homo sapiens | chemokine (C-C motif) ligand 20 |
| Mbl2 | Homo sapiens | mannose-binding lectin (protein C) 2, soluble (opsonic defect) |
| GYPB | Homo sapiens | glycophorin E; glycophorin B (MNS blood group) |
| GYPE | Homo sapiens | glycophorin E; glycophorin B (MNS blood group) |
| TNFRSF8 | Homo sapiens | tumor necrosis factor receptor superfamily, member 8 |
| Scgb3a1 | Homo sapiens | secretoglobin, family 3A, member 1 |
| irak3 | Homo sapiens | interleukin-1 receptor-associated kinase 3 |
| HTN3 | Homo sapiens | histatin 3 |
| CD248 | Homo sapiens | CD248 molecule, endosialin |
| C8A | Homo sapiens | complement component 8, alpha polypeptide |
| CD164L2 | Homo sapiens | CD164 sialomucin-like 2 |
| BANK1 | Homo sapiens | B-cell scaffold protein with ankyrin repeats 1 |
| IL12A | Homo sapiens | interleukin 12A (natural killer cell stimulatory factor 1, cytotoxic lymphocyte maturation factor 1, p35) |
| CHL1 | Homo sapiens | cell adhesion molecule with homology to L1CAM (close homolog of L1) |
| Spn | Homo sapiens | sialophorin |
| Traf1 | Homo sapiens | TNF receptor-associated factor 1 |
| il22 | Homo sapiens | interleukin 22 |
| Cxcr1 | Homo sapiens | interleukin 8 receptor, alpha |
| TEK | Homo sapiens | TEK tyrosine kinase, endothelial |
| FCRL5 | Homo sapiens | Fc receptor-like 5 |
| Cd300lg | Homo sapiens | CD300 molecule-like family member g |
| DOCK2 | Homo sapiens | dedicator of cytokinesis 2 |
| Cx3cr1 | Homo sapiens | chemokine (C-X3-C motif) receptor 1 |
| PGLYRP2 | Homo sapiens | peptidoglycan recognition protein 2 |
| WFDC12 | Homo sapiens | WAP four-disulfide core domain 12 |
| PLA2G7 | Homo sapiens | phospholipase A2, group VII (platelet-activating factor acetylhydrolase, plasma) |
| SLAMF7 | Homo sapiens | SLAM family member 7 |
| LY86 | Homo sapiens | lymphocyte antigen 86 |
| GZMM | Homo sapiens | granzyme M (lymphocyte met-ase 1) |
| PDCD1 LG2 | Homo sapiens | programmed cell death 1 ligand 2 |
| C1QC | Homo sapiens | complement component 1, q subcomponent, C chain |
| GZMB | Homo sapiens | granzyme B (granzyme 2, cytotoxic T-lymphocyte-associated serine esterase 1) |
| DEFA5 | Homo sapiens | defensin, alpha 5, Paneth cell-specific |
| dcd | Homo sapiens | dermcidin |
| Ccl2 | Homo sapiens | chemokine (C-C motif) ligand 2 |
| CLEC4A | Homo sapiens | C-type lectin domain family 4, member A |
| IL1RAPL1 | Homo sapiens | interleukin 1 receptor accessory protein-like 1 |
| IL26 | Homo sapiens | interleukin 26 |
| C1ql4 | Homo sapiens | complement component 1, q subcomponent-like 4 |
| IFNG | Homo sapiens | interferon, gamma |
| CEACAM5 | Homo sapiens | carcinoembryonic antigen-related cell adhesion molecule 5 |
| TNFRSF18 | Homo sapiens | tumor necrosis factor receptor superfamily, member 18 |
| POMC | Homo sapiens | proopiomelanocortin |
| PTPN22 | Homo sapiens | protein tyrosine phosphatase, non-receptor type 22 (lymphoid) |
| Itgax | Homo sapiens | integrin, alpha X (complement component 3 receptor 4 subunit) |
| DEFB4A | Homo sapiens | defensin, beta 4 |
| Ncam1 | Homo sapiens | neural cell adhesion molecule 1 |
| CD7 | Homo sapiens | CD7 molecule |
| TLR5 | Homo sapiens | toll-like receptor 5 |
| GNLY | Homo sapiens | granulysin |
| Gpr183 | Homo sapiens | G protein-coupled receptor 183 |
| Ncf4 | Homo sapiens | neutrophil cytosolic factor 4, 40kDa |
| CSF2RB | Homo sapiens | colony stimulating factor 2 receptor, beta, low-affinity (granulocyte-macrophage) |
| Defa4 | Homo sapiens | defensin, alpha 4, corticostatin |
| TNFRSF13B | Homo sapiens | tumor necrosis factor receptor superfamily, member 13B |
| DEFA6 | Homo sapiens | defensin, alpha 6, Paneth cell-specific |
| TLR1 | Homo sapiens | toll-like receptor 1 |
| AICDA | Homo sapiens | activation-induced cytidine deaminase |
| PTAFR | Homo sapiens | platelet-activating factor receptor |
| CCL13 | Homo sapiens | chemokine (C-C motif) ligand 13 |
| ALK | Homo sapiens | anaplastic lymphoma receptor tyrosine kinase |
| HRH2 | Homo sapiens | histamine receptor H2 |
| C3AR1 | Homo sapiens | complement component 3a receptor 1 |
| XCL1 | Homo sapiens | chemokine (C motif) ligand 1 |
| IFNB1 | Homo sapiens | interferon, beta 1, fibroblast |
| TNFRSF13C | Homo sapiens | tumor necrosis factor receptor superfamily, member 13C |
| CD84 | Homo sapiens | CD84 molecule |
| XCL2 | Homo sapiens | chemokine (C motif) ligand 2 |
| TLR2 | Homo sapiens | toll-like receptor 2 |
| CCL7 | Homo sapiens | chemokine (C-C motif) ligand 7 |
| Vpreb1 | Homo sapiens | pre-B lymphocyte 1 |
| CLEC4C | Homo sapiens | C-type lectin domain family 4, member C |
| NFATC2 | Homo sapiens | nuclear factor of activated T-cells, cytoplasmic, calcineurin-dependent 2 |
| LAX1 | Homo sapiens | lymphocyte transmembrane adaptor 1 |
| Cfi | Homo sapiens | complement factor I |
| Icp2 | Homo sapiens | lymphocyte cytosolic protein 2 (SH2 domain containing leukocyte protein of 76kDa) |
| FLT3 | Homo sapiens | fms-related tyrosine kinase 3 |
| IL2RA | Homo sapiens | interleukin 2 receptor, alpha |
| CD74 | Homo sapiens | CD74 molecule, major histocompatibility complex, class II invariant chain |
| CD93 | Homo sapiens | CD93 molecule |
| pdgfra | Homo sapiens | platelet-derived growth factor receptor, alpha polypeptide |
| CFHR4 | Homo sapiens | complement factor H-related 4 |
| CTSG | Homo sapiens | cathepsin G |
| Sirpa | Homo sapiens | signal-regulatory protein alpha |
| IL8 | Homo sapiens | interleukin 8 |
| CD36 | Homo sapiens | CD36 molecule (thrombospondin receptor) |
| MYLK | Homo sapiens | myosin light chain kinase |
| Hamp | Homo sapiens | hepcidin antimicrobial peptide |
| IL1RAPL2 | Homo sapiens | interleukin 1 receptor accessory protein-like 2 |
| SERPING1 | Homo sapiens | serpin peptidase inhibitor, clade G (C1 inhibitor), member 1 |
| cmklr1 | Homo sapiens | chemokine-like receptor 1 |
| CCR6 | Homo sapiens | cyclin L2; chemokine (C-C motif) receptor 6 |
| Ccnl2 | Homo sapiens | cyclin L2; chemokine (C-C motif) receptor 6 |
| btk | Homo sapiens | Bruton agammaglobulinemia tyrosine kinase |
| ITGA4 | Homo sapiens | integrin, alpha 4 (antigen CD49D, alpha 4 subunit of VLA-4 receptor) |
| CD180 | Homo sapiens | CD180 molecule |
| NCR2 | Homo sapiens | natural cytotoxicity triggering receptor 2 |
| PRG2 | Homo sapiens | proteoglycan 2, bone marrow (natural killer cell activator, eosinophil granule major basic protein) |
| KIR2DL4 | Homo sapiens | killer cell immunoglobulin-like receptor, two domains, long cytoplasmic tail, 4 |
| HRH4 | Homo sapiens | histamine receptor H4 |
| Cd2 | Homo sapiens | CD2 molecule |
| RHAG | Homo sapiens | Rh-associated glycoprotein |
| LILRA1 | Homo sapiens | leukocyte immunoglobulin-like receptor, subfamily A (with TM domain), member 1 |
| LILRB1 | Homo sapiens | leukocyte immunoglobulin-like receptor, subfamily B (with TM and ITIM domains), member 1 |
| SELE | Homo sapiens | selectin E |
| Il1rl2 | Homo sapiens | interleukin 1 receptor-like 2 |
| Cxcl9 | Homo sapiens | chemokine (C-X-C motif) ligand 9 |
| CD27 | Homo sapiens | CD27 molecule |
| IL1R2 | Homo sapiens | interleukin 1 receptor, type II |
| Il28a | Homo sapiens | interleukin 28A (interferon, lambda 2) |
| EPO | Homo sapiens | erythropoietin |
| Clec4e | Homo sapiens | C-type lectin domain family 4, member E |
| LOC727787 | Homo sapiens | killer cell immunoglobulin-like receptor, three domains, long cytoplasmic tail, 2; similar to killer cell immunoglobulin-like receptor 3DL2 precursor (MHC class I NK cell receptor) (Natural killer-associated transcript 4) (NKAT-4) (p70 natural killer cell receptor clone CL-5) (CD158k antigen) |
| KIR3DL2 | Homo sapiens | killer cell immunoglobulin-like receptor, three domains, long cytoplasmic tail, 2; similar to killer cell immunoglobulin-like receptor 3DL2 precursor (MHC class I NK cell receptor) (Natural killer-associated transcript 4) (NKAT-4) (p70 natural killer cell receptor clone CL-5) (CD158k antigen) |
| C7 | Homo sapiens | complement component 7 |
| Fcgr3a | Homo sapiens | Fc fragment of IgG, low affinity IIIa, receptor (CD16a) |
| Cmtm3 | Homo sapiens | CKLF-like MARVEL transmembrane domain containing 3 |
| CLEC4D | Homo sapiens | C-type lectin domain family 4, member D |
| CMTM2 | Homo sapiens | CKLF-like MARVEL transmembrane domain containing 2 |
| CD300E | Homo sapiens | CD300e molecule |
| KLRB1 | Homo sapiens | killer cell lectin-like receptor subfamily B, member 1 |
| IL12B | Homo sapiens | interleukin 12B (natural killer cell stimulatory factor 2, cytotoxic lymphocyte maturation factor 2, p40) |
| IL17RD | Homo sapiens | interleukin 17 receptor D |
| Il31ra | Homo sapiens | interleukin 31 receptor A |
| Batf | Homo sapiens | basic leucine zipper transcription factor, ATF-like |
| LTF | Homo sapiens | lactotransferrin |
| csf3r | Homo sapiens | colony stimulating factor 3 receptor (granulocyte) |
| Il17f | Homo sapiens | interleukin 17F |
| IL17A | Homo sapiens | interleukin 17A |
| IL17C | Homo sapiens | interleukin 17C |
| TNFSF4 | Homo sapiens | tumor necrosis factor (ligand) superfamily, member 4 |
| fcgr1a | Homo sapiens | Fc fragment of IgG, high affinity Ic, receptor (CD64); Fc fragment of IgG, high affinity Ia, receptor (CD64) |
| FCGR1 C | Homo sapiens | Fc fragment of IgG, high affinity Ic, receptor (CD64); Fc fragment of IgG, high affinity Ia, receptor (CD64) |
| Ly75 | Homo sapiens | CD302 molecule; lymphocyte antigen 75 |
| CD302 | Homo sapiens | CD302 molecule; lymphocyte antigen 75 |
| crp | Homo sapiens | C-reactive protein, pentraxin-related |
| CD226 | Homo sapiens | CD226 molecule |
| CD1B | Homo sapiens | CD1b molecule |
| CXCR2 | Homo sapiens | interleukin 8 receptor, beta |
| CXCL13 | Homo sapiens | chemokine (C-X-C motif) ligand 13 |
| Ccr3 | Homo sapiens | chemokine (C-C motif) receptor 3 |
| CD1E | Homo sapiens | CD1 e molecule |
| PIK3CG | Homo sapiens | phosphoinositide-3-kinase, catalytic, gamma polypeptide |
| LILRB4 | Homo sapiens | leukocyte immunoglobulin-like receptor, subfamily B (with TM and ITIM domains), member 4 |
| FCGR2A | Homo sapiens | Fc fragment of IgG, low affinity IIa, receptor (CD32) |
| BCAM | Homo sapiens | basal cell adhesion molecule (Lutheran blood group) |
| Csf2 | Homo sapiens | colony stimulating factor 2 (granulocyte-macrophage) |
| Ccl27 | Homo sapiens | chemokine (C-C motif) ligand 27 |
| Pglyrp3 | Homo sapiens | peptidoglycan recognition protein 3 |
| CD1C | Homo sapiens | CD1c molecule |
| Colec12 | Homo sapiens | collectin sub-family member 12 |
| CD1A | Homo sapiens | CD1a molecule |
| IL21 | Homo sapiens | interleukin 21 |
| MS4A3 | Homo sapiens | membrane-spanning 4-domains, subfamily A, member 3 (hematopoietic cell-specific) |
| Lilra5 | Homo sapiens | leukocyte immunoglobulin-like receptor, subfamily A (with TM domain), member 5 |
| CR1 | Homo sapiens | complement component (3b/4b) receptor 1 (Knops blood group) |
| PPP3R2 | Homo sapiens | protein phosphatase 3 (formerly 2B), regulatory subunit B, beta isoform |
| CXCR3 | Homo sapiens | chemokine (C-X-C motif) receptor 3 |
| Pdcd1 | Homo sapiens | programmed cell death 1 |
| LILRB2 | Homo sapiens | leukocyte immunoglobulin-like receptor, subfamily B (with TM and ITIM domains), member 2 |
| ccr4 | Homo sapiens | chemokine (C-C motif) receptor 4 |
| RNASE7 | Homo sapiens | ribonuclease, RNase A family, 7 |
| Msr1 | Homo sapiens | macrophage scavenger receptor 1 |
| ccl25 | Homo sapiens | chemokine (C-C motif) ligand 25 |
| FASLG | Homo sapiens | Fas ligand (TNF superfamily, member 6) |
| CD1D | Homo sapiens | CD1d molecule |
| CCL28 | Homo sapiens | chemokine (C-C motif) ligand 28 |
| A2ML1 | Homo sapiens | alpha-2-macroglobulin-like 1 |
| CEACAM8 | Homo sapiens | carcinoembryonic antigen-related cell adhesion molecule 8 |
| FGFR2 | Homo sapiens | fibroblast growth factor receptor 2 |
| Cd5l | Homo sapiens | CD5 molecule-like |
| Fcar | Homo sapiens | Fc fragment of IgA, receptor for |
| DEFB106A | Homo sapiens | defensin, beta 106A; defensin, beta 106B |
| DEFB106B | Homo sapiens | defensin, beta 106A; defensin, beta 106B |
| IRF8 | Homo sapiens | interferon regulatory factor 8 |
| Igj | Homo sapiens | immunoglobulin J polypeptide, linker protein for immunoglobulin alpha and mu polypeptides |
| ACE | Homo sapiens | angiotensin I converting enzyme (peptidyl-dipeptidase A) 1 |
| MPO | Homo sapiens | myeloperoxidase |
| CD86 | Homo sapiens | CD86 molecule |
| CD40 | Homo sapiens | CD40 molecule, TNF receptor superfamily member 5 |
| CD209 | Homo sapiens | CD209 molecule |
| KIR3DS1 | Homo sapiens | killer cell immunoglobulin-like receptor, three domains, short cytoplasmic tail, 1; killer cell immunoglobulin-like receptor, three domains, long cytoplasmic tail, 1 |
| Kir3dl1 | Homo sapiens | killer cell immunoglobulin-like receptor, three domains, short cytoplasmic tail, 1; killer cell immunoglobulin-like receptor, three domains, long cytoplasmic tail, 1 |
| mpl | Homo sapiens | myeloproliferative leukemia virus oncogene |
| Tnfrsf4 | Homo sapiens | tumor necrosis factor receptor superfamily, member 4 |
| IL17B | Homo sapiens | interleukin 17B |
| PSTPIP1 | Homo sapiens | proline-serine-threonine phosphatase interacting protein 1 |
| DPP4 | Homo sapiens | dipeptidyl-peptidase 4 |
| AIRE | Homo sapiens | autoimmune regulator |
| Cd53 | Homo sapiens | CD53 molecule |
| tbx21 | Homo sapiens | T-box 21 |
| THBD | Homo sapiens | thrombomodulin |
| CCR7 | Homo sapiens | chemokine (C-C motif) receptor 7 |
| CLEC10A | Homo sapiens | C-type lectin domain family 10, member A |
| CD274 | Homo sapiens | CD274 molecule |
| Cxcl6 | Homo sapiens | chemokine (C-X-C motif) ligand 6 (granulocyte chemotactic protein 2) |
| CD69 | Homo sapiens | CD69 molecule |
| CD22 | Homo sapiens | CD22 molecule |
| IL23A | Homo sapiens | interleukin 23, alpha subunit p19 |
| NOD2 | Homo sapiens | nucleotide-binding oligomerization domain containing 2 |
| TNFRSF9 | Homo sapiens | tumor necrosis factor receptor superfamily, member 9 |
| LPO | Homo sapiens | lactoperoxidase |
| nfatc1 | Homo sapiens | nuclear factor of activated T-cells, cytoplasmic, calcineurin-dependent 1 |
| CMTM5 | Homo sapiens | CKLF-like MARVEL transmembrane domain containing 5 |
| IL25 | Homo sapiens | interleukin 25 |
| Lair1 | Homo sapiens | leukocyte-associated immunoglobulin-like receptor 1 |
| LAIR2 | Homo sapiens | leukocyte-associated immunoglobulin-like receptor 2 |
| NOS2 | Homo sapiens | nitric oxide synthase 2, inducible |
| NCR3 | Homo sapiens | natural cytotoxicity triggering receptor 3 |
| SOD3 | Homo sapiens | superoxide dismutase 3, extracellular |
| IL28B | Homo sapiens | interleukin 28B (interferon, lambda 3) |
| RHD | Homo sapiens | Rh blood group, D antigen |
| KLRD1 | Homo sapiens | killer cell lectin-like receptor subfamily D, member 1 |
| Tnfsf14 | Homo sapiens | tumor necrosis factor (ligand) superfamily, member 14 |
| IL9R | Homo sapiens | interleukin 9 receptor |
| il10 | Homo sapiens | interleukin 10 |
| CCR10 | Homo sapiens | chemokine (C-C motif) receptor 10 |
| CR2 | Homo sapiens | complement component (3d/Epstein Barr virus) receptor 2 |
| DEFB105B | Homo sapiens | defensin, beta 105A; defensin, beta 105B |
| DEFB105A | Homo sapiens | defensin, beta 105A; defensin, beta 105B |
| LYZ | Homo sapiens | lysozyme (renal amyloidosis) |
| DEFB103A | Homo sapiens | defensin, beta 103B; defensin, beta 103A |
| DEFB103B | Homo sapiens | defensin, beta 103B; defensin, beta 103A |
| CMTM7 | Homo sapiens | CKLF-like MARVEL transmembrane domain containing 7 |
| LILRB5 | Homo sapiens | leukocyte immunoglobulin-like receptor, subfamily B (with TM and ITIM domains), member 5 |
| IL4 | Homo sapiens | interleukin 4 |

The expression of any gene listed in the above Tables 1 to 3 may be inhibited by the nucleic acid molecule of the invention, the fusion construct of the invention, the vector of the invention, the cell of the invention, the polypeptide of the invention or a combination thereof. This is because the expression of otherwise silent genes in the course of an immune response requires the *de novo* recruitment of TFIIB to the promoter sites of these genes. As discussed herein above, the nucleic acid molecule of the invention, the fusion construct of the invention, the vector of the invention, the cell of the invention, the polypeptide of the invention or a combination thereof interfere with this *de novo* recruitment of TFIIB. It is to be understood that the genes in Tables 1 to 3 are non-limiting examples of genes whose expression may be inhibited by the nucleic acid molecule of the invention, the fusion construct of the invention, the vector of the invention, the cell of the invention, the polypeptide of the invention or a combination thereof. For example, also the expression of genes may be affected which are regulated through one of the genes in Tables 1 to 3. The examples below show that interferon beta expression is regulated through the ML fragements of the invention. It follows that also taget genes of interferon beta are affected as the result of the inhibition of interferon beta expression.

It is furthermore to be understood that also in connection with the other aspects of the present invention as far as referring to "inhibiting gene expression" the gene the expression of which is to be inhibited is preferably selected from the genes in Tables 1, 2 and/or 3. Again, the gene may be selected from any one of Tables 1, 2 and 3, Tables 1 and 3, Tables 2 and 3, Table 1, Table 2 or Table 3.

In an eighth aspect the present invention relates to a pharmaceutical composition comprising the nucleic acid molecule of the invention, the fusion construct of the invention, the vector of the invention, the cell of the invention, the polypeptide of the invention or a combination thereof.

Also comprised by the invention is a method of treating a disease, comprising administering to the patient a therapeutically effective amount of a pharmaceutical composition comprising the nucleic acid molecule of the invention, the fusion construct of the invention, the vector of the invention, the cell of the invention, the polypeptide of the invention or a combination thereof. In this connection the disease is preferably one of the diseases described herein below.

In accordance with the present invention, the term "pharmaceutical composition" relates to a composition for administration to a patient, preferably a human patient. The pharmaceutical composition in accordance with the present invention can be formulated in conventional manner according to methods found in the art, using one or more physiological carriers or excipient, see, for example Alle and Ansel, "Pharmaceutical Dosage Forms and Drug Delivery Systems", 10th edition, Lippincott Raven, 2013. The pharmaceutical composition may, accordingly, be administered orally, parenterally, such as subcutaneously, intravenously, intramuscularly, intraperitoneally, intrathecally, transdermally, transmucosally, subdurally, locally or topically via iontopheresis, sublingually, by inhalation spray, aerosol or rectally and the like in dosage unit formulations optionally comprising conventional pharmaceutically acceptable excipients.

The pharmaceutical composition of the invention may be administered as the sole active ingredient or in conjunction with, e.g. as an adjuvant to or in combination with, other drugs, e.g. immunosuppressive or immune modulating agents or other anti-inflammatory agents, e.g. for the treatment or prevention of diseases discussed herein below. For example, the polypeptide of the invention may be used in combination with immunosuppressive monoclonal antibodies, e.g. monoclonal antibodies with affinity to leukocyte receptors.

The pharmaceutical composition is to be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g units per day. However, a more preferred dosage might be in the range of 0.01 mg to 500 mg, even more preferably 0.01 mg to 50 mg and most preferably 0.01 mg to 10 mg per day.

In accordance with a preferred embodiment the pharmaceutical composition of the invention is for use in treating a disease selected from the group consisting of a disease caused by overreaction of the immune system, an autoimmune disease, an inflammatory disease, a viral infection, an immunopathy induced by a pathogen and/or a toxin, an allergy, septic shock, tissue damage, a hyperproliferative disease, and a metabolic disorder.

All these diseases are known to be associated with the expression of particular genes, in particular genes which are normally not expressed or essentially not expressed in a cell in the absence of the respective disease. Consequently, genes become newly activated in the course of these diseases and this activation *inter alia* requires *de novo* recruitment of TFIIB to the promoter sites of these genes. The inhibition of the expression of these genes by the pharmaceutical composition of the present invention is therefore expected to be of benefit for the treatment of these diseases.

In accordance with a more preferred embodiment the pharmaceutical composition of the invention is for use in treating an autoimmune and/or autoinflammatory disease selected from rheumatoid arthritis, multiple sclerosis, corneal transplant rejection, transplant rejection, uveitis, Type I diabetes, schizophrenia, alopecia areata, psoriasis vulgaris, vitiligo, pemphigus vulgaris, Alzheimer's, Parkinson's disease, Crohn's disease, lupus erythematosus, Sjorgen's syndrome, epidermolysis bullosa, sarcoidosis, autoinflammatory diseases (such as FMF, NOMID, TRAPS), and coeliac disease.

As is well known, autoimmune and autoinflammatory diseases arise from an abnormal immune response of the body against substances and tissues normally present in the body. Said abnormal immune response involves the *de novo* expression of genes and consequently also the recruitment of TFIIB to the promoter sites of these genes. The above list of autoimmune diseases provides non-limiting examples of autoimmune diseases which may be treated by the pharmaceutical composition of the invention.

In accordance with another more preferred embodiment the pharmaceutical composition of the invention is for use in treating a viral infection being caused by a DNA virus or RNA virus with a DNA intermediate, such as a herpes virus, an adeno virus, a papova viruses, a hepadna virus or a retrovirus.

The listed virus classes and virus families are known to rely on the cellular polymerase machinery including *de novo* recruitment of TFIIB. It follows that in particular viral infections caused by these virus classes and virus families will benefit from treatment with pharmaceutical composition of the invention.

In accordance with a further more preferred embodiment the pharmaceutical composition of the invention is for use in treating an inflammatory disease selected from inflammatory bowel disease, glomerulonephritis, pelvic inflammatory disease, vasculitis, a chronic inflammation of an organ, ankylosing spondylitis, asthma, atherosclerosis, gastritis, colitis, dermatitis, diverticulitis, hepatitis, conjunctivitis, sinusitis, ulcerative colitis, psoriasis, allergic reactions, eczema, laryngitis, pharyngitis, tonsilitis, acne, hypersensitivity, and reperfusion injury.

As commonly known, an inflammation is a protective response of the body that involves the *de novo* expression of genes and consequently also the recruitment of TFIIB to the promoter sites of these genes. The above listed inflammatory diseases provide non-limiting examples of inflammatory diseases which may be treated by the pharmaceutical composition of the invention.

As regards the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The above considerations apply *mutatis murandis* to all attached claims. To give a few examples, the combination of claims 3 and 1 (b) is clearly and unambiguously envisaged in view of the claim structure. The same applies for the combinations of claims 3 and 1 (c), and, to give a few further examples which are not limiting, the combination of claim 11 as far as referring back to claim 8 with claim 12 or combination the combination of claim 11 as far as referring back to claims 1 to 3 with claim 12.

The Figures show:
**Fig. 1****. THOV ML inhibits IFN production by interacting with general transcription factor TFIIB, but not SOLH.** A) Type-I IFN levels after infection with THOV wt or dML 24 h.p.i. SN from infected HeLa cells were applied on 293T Mx1-luc indicator cells and compared with a type-I IFN standard. B) Expression control western blot for A) to ensure equal infecton. C) Volcano plot of proteins enriched in ML vs. M pulldown and identified by AP-LC-MS/MS. D) Schematic representation of THOV M and ML proteins. Mutations to Ala are indicated with arrows. E) IP of GST-tagged M or ML (wt and mut) and co-IP of FLAG-tagged SOLH and TFIIB (transiently overexpressed in 293 cells). F) IFN levels after infection with THOV wt, dML or mut 24 h.p.i. SN from infected HeLa cells were applied on 293T Mx1-luc. G) Expression control western blot for F) to ensure equal infecton.

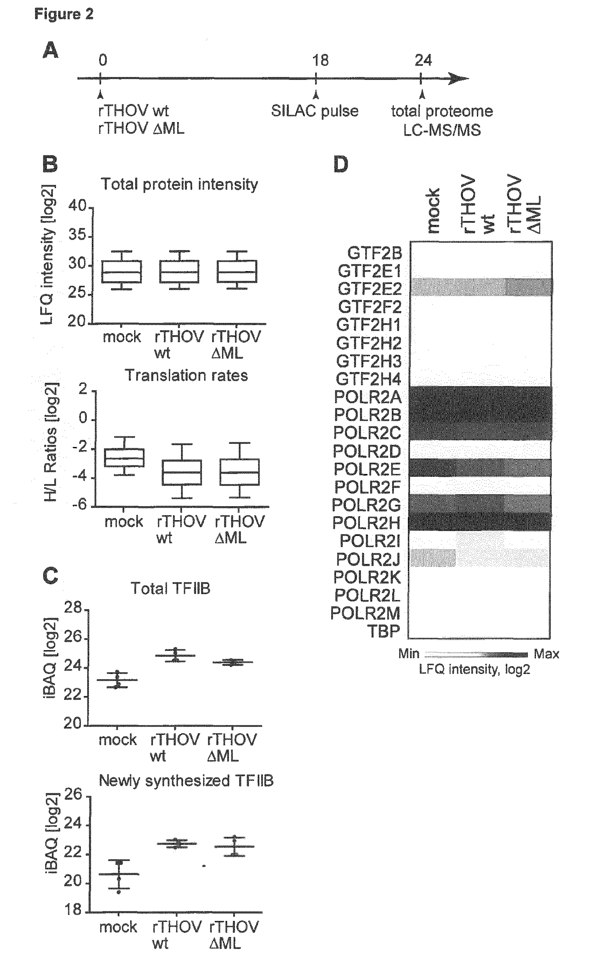

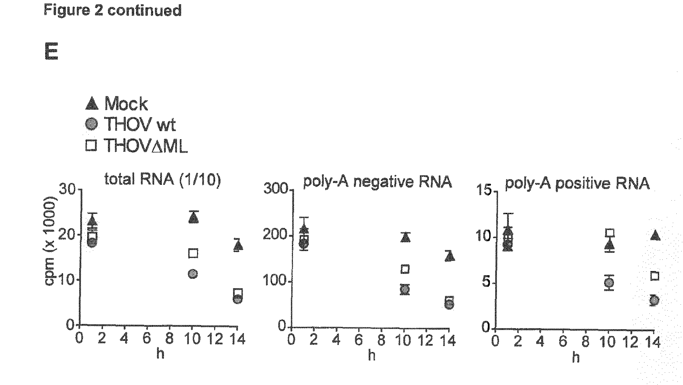

**Fig. 2****. ML presence does not affect TFIIB protein levels or other polymerase II complex components.** A) Schematic representation of pulse SILAC experiment. HeLa cells were infected with THOV wt or dML, after 18 hours, the cells were starved in medium lacking Lys and Arg for 30 min and then pulsed with SILAC medium containing heavy Lys8 and Arg10 for another 6 hours. Cells were harvested and subjected to the whole proteome LC-MS/MS analysis. B) Top panel: total protein intensity as defined by label-free quantification. Bottom panel: translation rates of identified proteins determined from H (newly synthesized)/L (total) ratios presented as box-whisker plots with whiskers showing 10-90 percentile. C) Top panel: total TFIIB intensity as defined by label-free quantification. Bottom panel: levels of newly synthesized TFIIB as determined from heavy intensities. D) Effect of ML presence on the levels of polymerase II subunits and general transcription factors after THOV wt or dML infection. Presented are log2-transformed LFQ intensities. E) RNA synthesis rate in THOV infected Vero cells. Newly synthesized RNA was labelled with [3H]-5-Uridine at 1, 10 and 14 hours post infection. Presented are total, polyA-negative and polyA-enriched fractions.

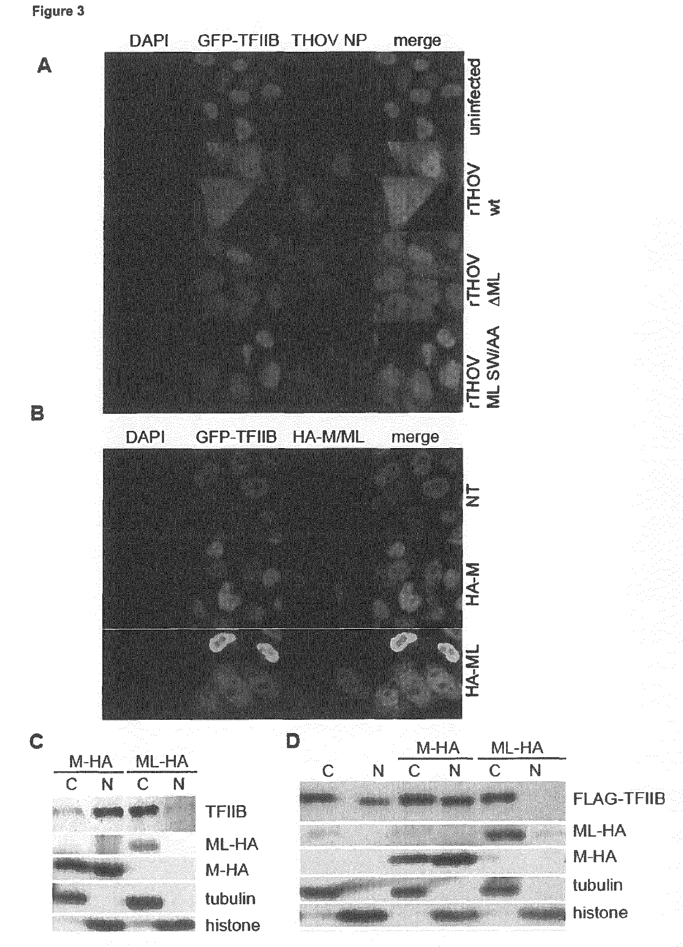

**Fig. 3****. ML overexpression leads to the translocation of TFIIB into the cytoplasm.** A) Confocal immunofluorescense analysis of HeLa Kyoto cells stably expressing GFP-TFIIB infected with THOV wt, THOV ΔML or THOV ML (SW/AA) mutants for 24 hours at MOI 3. B) Confocal immunofluorescense analysis of HeLa Kyoto cells stably expressing GFP-TFIIB and transiently transfected with HA-M or HA-ML for 16 hours. HeLa cells were treated as indicated, fixed and stained with GFP-DyLight488, THOV NP+rbAlexa546 and DAPI and subjected to confocal microscopy. C) Cytoplasmic-nuclear fractionation of Vero cells transiently transfected with HA-tagged M or ML for 24 hours. Depicted is endogenous TFIIB. D) Cytoplasmic-nuclear fractionation of Vero cells transiently transfected with FLAG-TFIIB and HA-tagged M or ML for 24 hours.

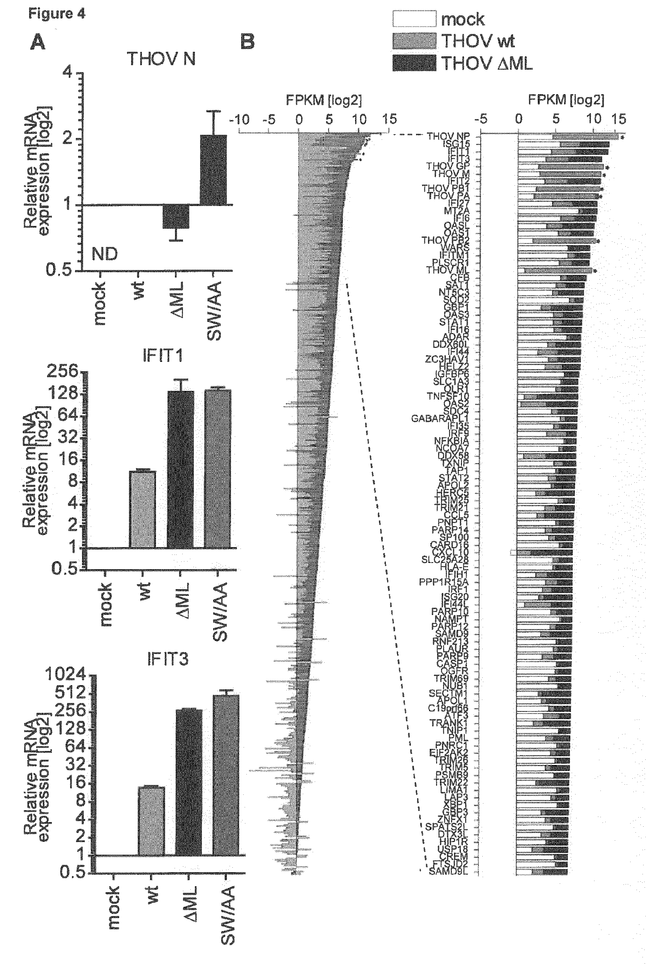

**Fig. 4****. Gene expression after THOV infection.** A) qPCR analysis for the indicated genes in HeLa cells infected with THOV wt, THOV ΔML and THOV ML (SW/AA) for 24 hours. B) Next generation sequencing analysis results: Ranked log2 FPKM values for THOV ΔML presented for genes showing 1.5 (log2) fold upregulation or downregulation compared to mock; corresponding FPKM values of mock (grey) and wt (blue) are superimposed. Close-up shows top100 changing genes in THOV ΔML.

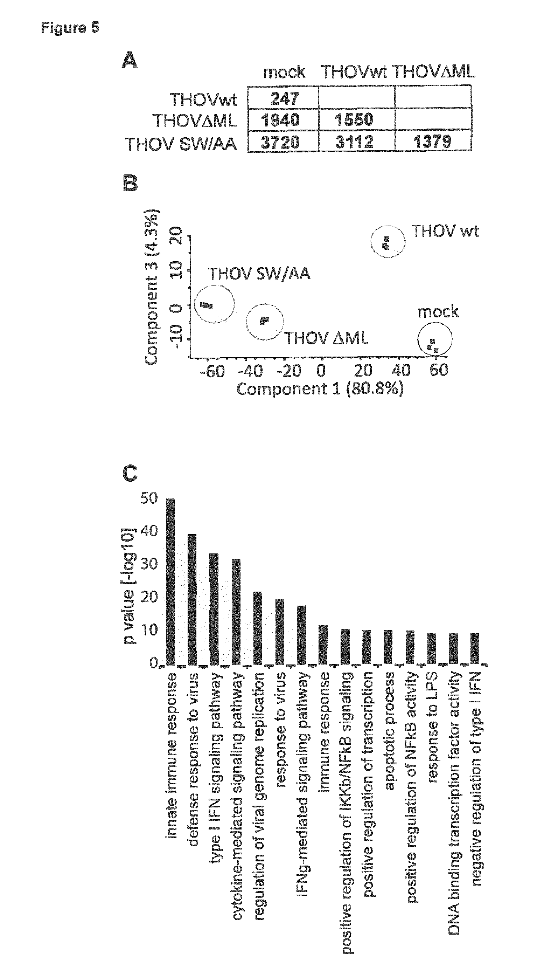

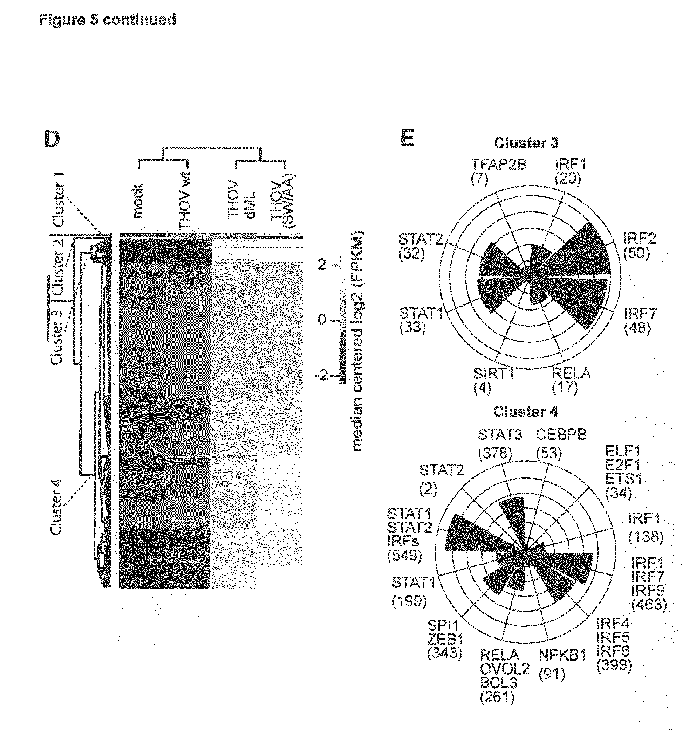

**Fig. 5****. Transcriptome analysis of HeLa cells infected with THOV viruses suggests that ML inhibits virus induced gene expression.** A) Matrix showing the number of changes between mock, THOV wt, THOV ΔML and THOV ML (SW/AA). B) Clustering of replicates based on the highest variance component 1 (immune response genes) and component 3 (viral genes). C) GO term over-representation analysis of differentially upregulated genes by THOV ΔML and THOV ML (SW/AA) compared to THOV wt performed with InnateDB analysis tool. D) Heat map of hierarchically clustered log2 FPKM values normalized by subtracting median of changing genes identified by transcriptome analysis (RNA-seq) . E) Polar charts representing enriched transcription factors and numbers of regulated genes, identified by upstream regulator analysis of enriched clusters from (B) (iRegulon).

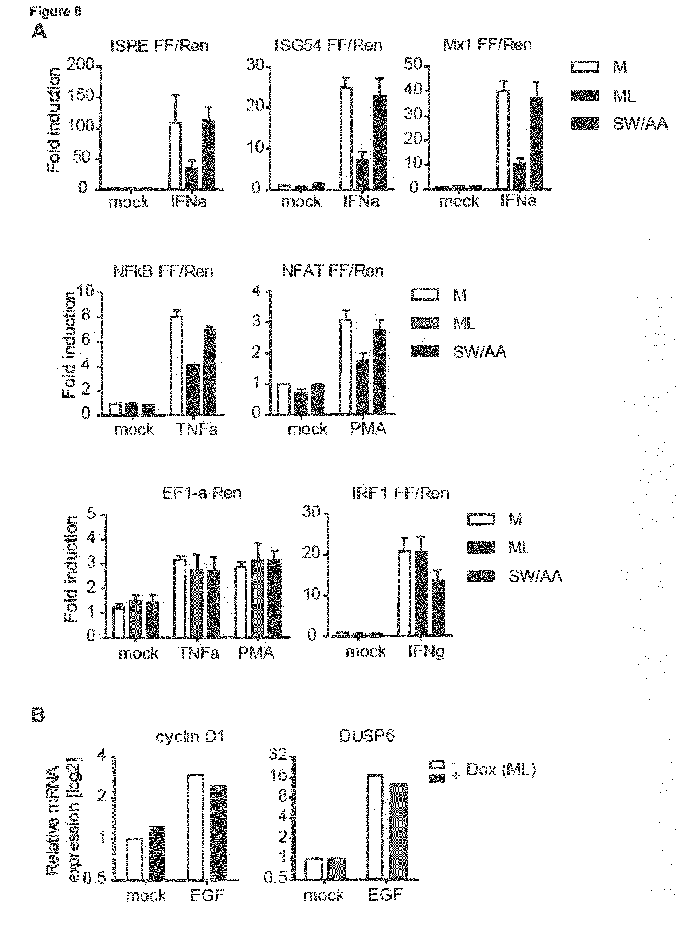

**Fig. 6****. Multiple, but not all inducible promoters are repressed by ML.** (A) Reporter assays in 293 cells, where Firefly luciferase under indicated promoters were transfected together with EF-1a Renilla and M/ML/ML-SW/AA. 24 h.p.t. cells were treated with indicated stimuli for 16 hours and luciferase activity was measured. Shown is fold induction over untreated cells. B) quantitative (q)PCR analysis of HeLa Flipln cells expressing stably integrated ML from Tet-On promoter. HeLa cells were left untreated or treated with Doxycycline for 24h and subsequently stimulated with EGF for 16 hours. Total RNA was extracted and expression of indicated genes measured by qPCR. Shown are expression levels in relation to the non Doxycyline treated unstimulated condition.

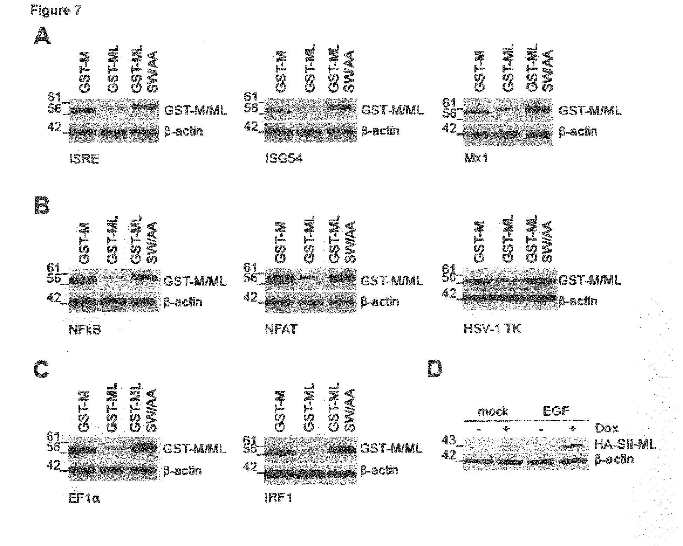

**Fig. 7****. Expression check of M and ML proteins for** **Fig. 6****.** (A-C) Western blot analysis of 293 cells transiently transfected with GST-tagged M or ML. D) Western blot analysis of HeLa Flipln cells expressing stably integrated ML from Tet-On promoter after Doxycycline induction.

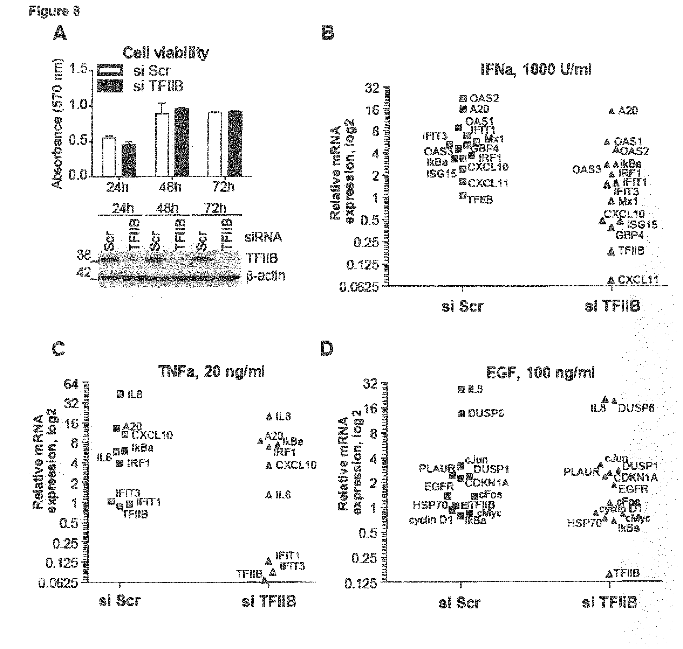

**Fig. 8****. TFIIB is required for** gene **expression regulated by *de novo* recruitment of polymerase II.** A) Cell viability of HeLa cells after TFIIB knockdown at indicated time points. HeLa cells were treated with indicated siRNAs for 24, 48 and 72 hours and knockdown was validated by Western blot analysis. Cell viability was assessed by MTT assay. B-D) qPCR analysis of HeLa cells before and after TFIIB knockdown. HeLa cells were electroporated with indicated siRNAs, 16 h.p.t. the cells were stimulated with B) 1000 U/ml IFNa or C) 20ng/ml TNFa, D) 100ng/ml EGF for 4 hours. Genes, whose expression is significantly changing (p<0.05) after TFIIB knockdown, are shown in grey.
**Fig. 9****. Binding of c-terminal ML to TFIIB and inhibition of the ISRE promoter.** A) Schematic representation of ML fragments and c-terminal sequence. B) TFIIB interaction with GFP-fused ML fragments. Radioactively labelled GFP-fusion proteins were incubated with recombinant GST or GST-TFIIB. GST-associated radioactive GFP fragments were precipitated and analysed by SDS-PAGE autoradiography. C) Reporter assay in 293 cells, where Firefly luciferase under ISRE promoter was co-transfected with EF1-a Renilla and GFP-ML fragments. Luciferase activity was measured 16h after stimulation with IFNa. Correct expression of ML fragments was analysed by Western blot. D) as in C) but Cherry-tagged ML fragments were used.

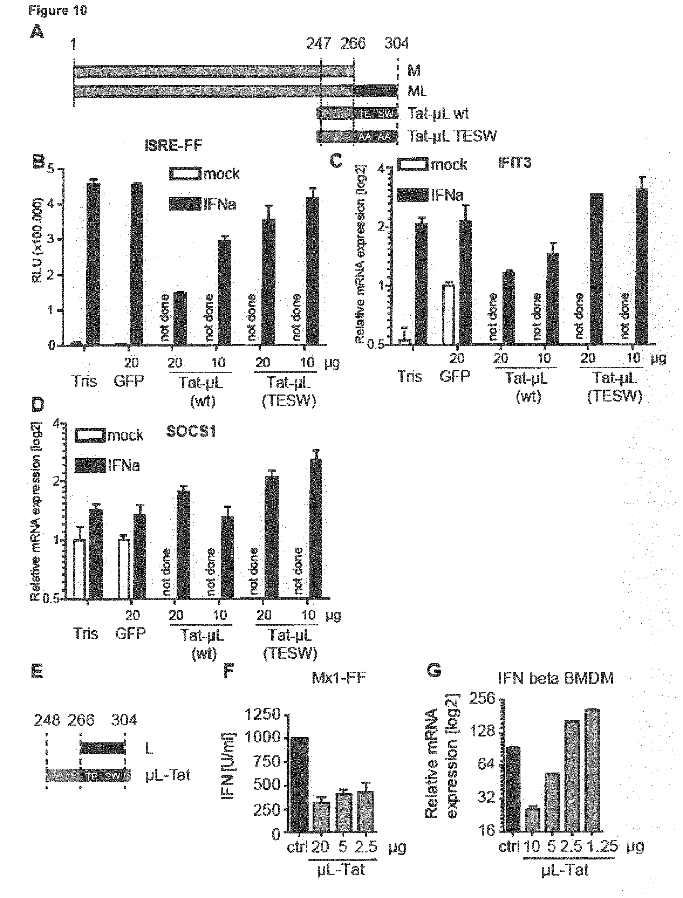

**Fig. 10****. Effect of recombinant Tat-µL on the inhibition of genes that require TFIIB.** A) Schematic representation of Tat-µL wt and µL (TESW/AAAA) mutant generated in E. coli. B) 293 cells were co-transfected with ISRE-Firefly and Tat-µL recombinant proteins and cells stimulated with IFN-a/b for 16h. C) qPCR analysis of HeLa cells transfected with Tat-µL recombinant proteins and treated with IFN-a/b for 4h. E) chemically synthesized µL-Tat peptide. F) Inhibition of IFNa stimulated Mx1 promoter driven luciferase in 293-Mx1-luc cells by synthetic µL-Tat. F) Inhibition of IFN beta mRNA expression in LPS stimulated murine Bone-marrow derived macrophages (BMDMs).

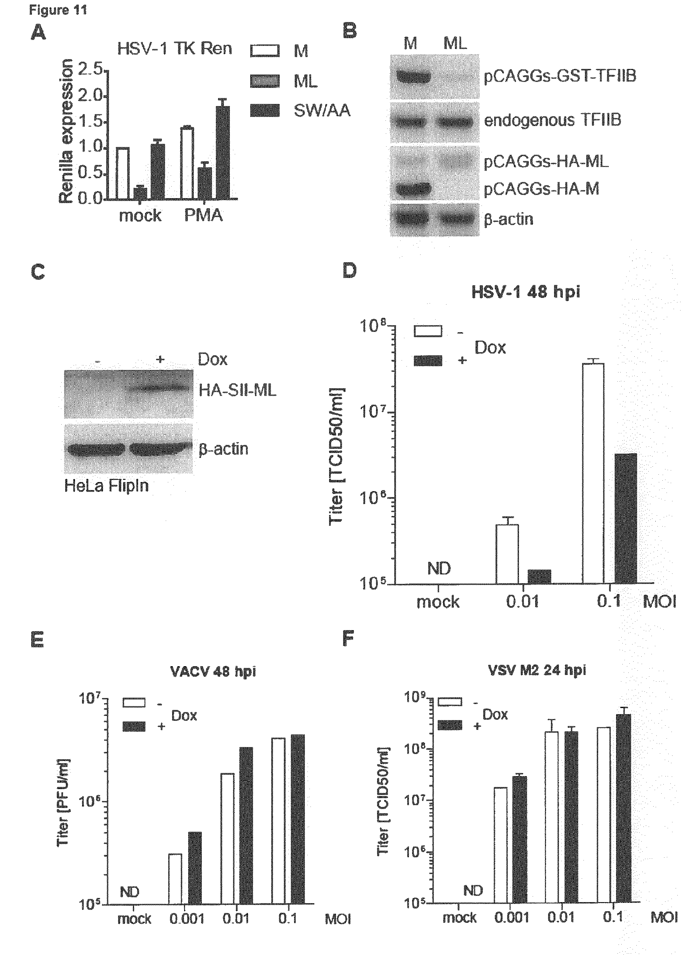

**Fig. 11****. Effect of ML on herpesvirus growth inhibition.** A) Reporter assay in 293 cells where Renilla under HSV-1 TK promoter was co-transfected with GST-tagged M, ML wt or SW/AA mutant. B) Western blot analysis of 293 cells transfected with HA-tagged M or ML and GST-TFIIB expressed from CMV IE promoter. C) Western blot analysis of HeLa Flipln cells expressing stably integrated ML from Tet-On promoter after Doxycycline induction. D) Titers of HSV-1, VACV and VSV M2 viruses before and after ML induction. HSV-1 and VSV M2 titers were assessed by TCID50 assay, VACV - by plaque assay.

The examples illustrate the invention.

### Example 1 - Thogoto virus ML protein inhibits IFN production by interacting with TFIIB

All viruses that replicate successfully have evolved mechanisms to escape interferon response. In line with published results (Vogt et al., 2008), infection of HeLa cells with recombinant (r) THOV wt induced only minimal amounts of IFN-a/b. In contrast wild-type THOV ML, a mutant rTHOV ΔML - said mutant having a genetic deletion of the viral interferon inhibitory protein ML (Fig. 1D) - potently induced IFN-a/b (Fig. 1A, B).

To identify specific interactors of the viral ML protein affinity purification was used followed by liquid chromatography and tandem mass spectrometry (AP-LC-MS/MS). To this end, HA-tagged viral M and ML protein were expressed in HEK293 cells and used for affinity purification followed by LC-MS/MS analysis. When comparing M and ML precipitates, two highly significant interactors that were selectively enriched in ML samples were identified. These two candidates were the general transcription factor IIB (TFIIB) and the largely uncharacterized Small optic lobes homolog (SOLH), a member of the calpain family of proteases (Fig. 1 C).

To validate the binding of ML to TFIIB and SOLH, co-immunoprecipitation of GST-tagged M and ML with FLAG-tagged SOLH and TFIIB was performed (Fig. 1E). C-terminal point mutants of ML proteins bearing alanine replacement of positions S283A/W284A and T270A/E271A were also used (Fig. 1D). As published previously (Vogt et al., 2008), ML (SW/AA) lost the ability to interact with TFIIB, whereas ML (TE/AA) bound to TFIIB similarly well to the wt protein. Conversely, it was found that SOLH could not be precipitated with exogenously expressed ML (TE/AA) but bound TFIIB comparably well to wt ML protein (Fig. 1E). Thus, critical point mutants were identified that allow for specifically testing the biological consequences of ML engagement of TFIIB and SOLH, respectively. To this end, HeLa cells were infected with rTHOV wt and ML mutants and accumulation of IFN-a/b in the cell supernatant was measured. rTHOV lacking ML or expressing ML(SW/AA) (leading to loss in TFIIB interaction) failed to inhibit IFN production (Fig 1 F). However, mutant THOV expressing ML(TE/AA) (leading to loss in SOLH binding) was still able effectively inhibit IFN-a/b induction (Fig. 1 F). Thus, the ability to interact with TFIIB is the most decisive property of ML to interfere with IFN-a/b induction during virus infection.

### Example 2- ML sequesters TFIIB from the nucleus, but does not cause its degradation

Various viruses have evolved mechanisms involving targeting of cellular transcription machinery to evade the IFN response. For instance, RVFV NSs protein sequesters p44 subunit of TFIIH (Le May et al., 2004) and recruits an E3 ligase complex to p62 subunit leading to its proteasomal degradation (Kainulainen et al., 2014) resulting in destabilization of TFIIH and host shutdown. It was tested whether binding of ML to TFIIB results in similar drastic effects. To this aim, a pulse-SILAC LC-MS/MS approach (Fig. 2A) was employed that allows to assess proteome-wide rates of translation and degradation. Compared to controls or THOV ΔML, infection with wt THOV did not affect rates of translation in general (Fig. 2B) or the abundance of TFIIB in particular (Fig. 2C). Other components of the pre-initiation complex were not affected by infection with wt THOV either (Fig. 2D). These analyses supported the notion that THOV does not impair general protein expression and further showed that THOV does not lead to TFIIB degradation. Next the transcription of newly synthesized genes was tested using pulse-labelling with radioactive nucleotides. Total RNA transcription, transcription of poly-A RNA or non-polyadenylated RNA were not affected by infection with wt THOV as compared to THOV ΔML (Fig. 2E). Collectively, these data suggest that ML binds to a general transcription factor (TFIIB), but this interaction does not lead to a general shutdown of transcription and translation or TFIIB abundance. To further understand how ML interferes with TFIIB, distribution of TFIIB on a subcellular level by confocal imaging was investigated. In uninfected cells TFIIB shows nuclear localization, consistent with its function in RNA transcription. However, in the presence of THOV wt, TFIIB showed a dramatic re-localization into the cytoplasm (Fig. 3A). This re-localization was specific to the virus expressing functional ML, since infection with THOV that failed to interact with TFIIB, such as THOV ΔML and THOV ML (SW/AA), resulted in normal nuclear TFIIB localization (Fig. 3A). To test whether the observed effect was indeed dependent on ML activity, ML was transiently transfected and the subcellular localization of TFIIB was analyzed. Transfection of ML but not transfection with M led to accumulation of TFIIB in the cytoplasm (Fig. 3B). TFIIB re-localization was confirmed by subcellular fractionation of ML transfected cells. In cells expressing M, endogenous (Fig. 3C), as well as overexpressed TFIIB (Fig. 3D) localized to the nucleus, whereas cells that received ML showed cytoplasmic localization of TFIIB. Of note, ML did not generally affect nuclear/cytoplasmic distribution since histones still localized to the nucleus. All together, these data suggested that ML interferes with TFIIB function by altering its subcellular distribution and thus the assembly of pre-initiation complex.

### Example 3- Transcriptome analysis of THOV induced changes suggests broad but specific effect of ML

Based on the ability of ML to impair cellular localization of a central component of the polymerase complex, it was questioned whether ML has a more broad effect on transcription than previously anticipated. To test this, transcriptome analysis of HeLa cells that were left uninfected or infected with THOV wt, THOV ΔML or THOV ML (SW/AA) for 24 hours was performed. The expression levels of viral NP were comparable between THOV wt and THOV ΔML and slightly higher in THOV ML (SW/AA) (Fig. 4A). As expected, infection with THOV ΔML and THOV ML (SW/AA) elicited expression IFIT-1 and -3, whereas expression of IFITs was -50 fold lower in cells infected with THOV wt, validating successful infection and triggering of an innate immune response by the mutant viruses (Fig. 4A). Infection with THOV wt only led to modest changes on the gene expression profile (n= 247) (Fig. 5A), which is in line with our above findings that targeting of TFIIB by THOV ML does not significantly impact general transcription rates in a negative or positive manner. Comparing gene expression in cells that were infected with THOV ΔML or THOV ML (SW/AA) with mock infected cells revealed ~600 significantly changed genes that showed more than 2-fold up or downregulation in expression levels. Principal component analysis (PCA), and unsupervised hierarchical clustering showed that the samples grouped according to the pathogen used for infection, validating the quality of the dataset (Fig. 5B-D). In PCA, component 1, responsible for 80.8% of data variation separated mock and THOV wt infected cells from cells infected with THOV ΔML or THOV ML (SW/AA), confirming the minimal effect of THOV wt on transcription and suggesting that the effect of ML in HeLa cells is predominantly attributable to its ability to interfere with TFIIB function (Fig. 5B). General properties of genes significantly upregulated by THOV ΔML and THOV ML (SW/AA) were related to innate antiviral response as determined by GO term over-representation analysis (Fig. 5C). Individual clusters of genes changing after THOV infection were analyzed (Fig. 5D). Cluster 1 shows expression of viral genes which, as expected, are similarly upregulated in all infected samples. Clusters 3 and 4 contain genes that are highly (cluster 3) or moderately (cluster 4) upregulated by infection with THOV ΔML or THOV ML (SW/AA), but not regulated by THOV wt (Fig. 5D). In order to gain deeper insights into the regulation of changed genes, upstream regulator analysis using iRegulon (Janky et al., 2014) was performed, which screens promoter regions of co-regulated genes for putative transcription factor binding sites - regulatory motifs alongside with candidate transcription factors. As seen from the analysis, the majority of genes changing in cluster 3 and cluster 4 are controlled by IRFs or STATs (as a result of pathogen recognition or IFN-a/b production, respectively) (Fig. 5E). In addition to genes regulated through IRFs or STATs activity, cluster 3 identified RELA, a key component of the NF-kB pathway, TFAP2B, a member of AP-2 family, and deacetylase Sirtuin (SIRT) 1. Cluster 4 contained genes regulated by the transcription factors SPI1, RELA, NFKB1, ELFs and CEBPB. Consistently with GO term over-representation analysis, upstream regulator analysis indeed showed IRFs and STATs enrichment. It can be concluded from these experiments that THOV ΔML and THOV ML (SW/AA) trigger activation of genes regulated by many transcription factors, suggesting that ML inhibits a broad array of genes.

### Example 4 - Multiple but not all inducible promoters are repressed by ML

Transcriptome profiling suggested that cells infected with THOV wt show minimal changes in gene expression compared to mock infected cells. However, general transcription was not affected. This prompted us to test, whether ML may selectively inhibit any dynamic changes in gene expression without affecting ongoing transcriptional processes. To test this and to delineate whether ML is not just inhibiting upstream processes resulting in broad spectrum of changes, reporter constructs that allow measuring the transcriptional activity of selected promoters after stimulation with activating ligands directly were used. In agreement with minimal influence on constitutive gene expression, the activity of EF1a-promoter driven Renilla luciferase was not affected by co-transfection of M, ML or ML (SW/AA) (Fig. 6A, 7C). Next reporter constructs that are downstream of signaling cascades which can be activated by defined ligands were used. Intriguingly, when using type-I IFN driven luciferase reporter constructs, such as promoters for interferon stimulated response element (ISRE), the ISG54 promoter or the Mx1, all of which contain STAT1 binding sites and thus directly responsive to type-I IFN treatment, co-expression of ML impaired their activity, whereas M or ML (SW/AA) did not show this effect (Fig. 6A and 7A). The inhibitory effect of ML, but not M or ML (SW/AA) could also be seen for other inducible promoters containing NFkB, or NFAT sites (Fig. 6A, 7B). Surprisingly, IRF1 promoter, even though showing a dynamic change after IFN-g stimulation, was not affected by ML overexpression (Fig. 6A, 7C). Similarly, quantitative RT-PCR analysis suggested that EGF target genes cyclin D1 and DUSP6 were induced equally after EGF engagement, despite presence of ML (Fig. 6B, 7D). It can be concluded from these experiments that ML represses dynamic changes in gene expression, but this inhibitory effect is limited to the activity and regulation of selected promoters. In general two different modes of transcriptional regulation exist: gene expression can be regulated through pausing of polymerase activity on the promoter and stimulus-dependent activation releases this pausing activity to drive expression of genes. Prototypic genes falling in this category of gene regulation are targets of the EGF signaling cascade (Gardini et al., 2014; Saunders et al., 2006; Tullai et al., 2007). Another mechanism to regulate gene expression is *de novo* recruitment of polymerase II to transcriptional start sites. This mode of regulation is best described for a subset, but not all genes associated with inflammatory processes (Freaney et al., 2013). A notable exception for the latter point is the regulation of IRF1 and the NF-κB inhibitory gene A20, both of which are regulated by transcriptional pausing. The activity of ML in reporter assays described here suggest that ML impairs gene regulation that requires *de novo* recruitment of polymerase but not other modes of gene transcriptional control.

### Example 5 - TFIIB depletion preferentially affects genes requiring de novo polymerase II recruitment

Since ML affects gene expression by interacting with TFIIB, it was hypothesized that TFIIB depletion would mimic the effect of ML. To validate this, siRNA-mediated knockdown of TFIIB was performed and it was tested whether this affected expression from inducible promoters. Transient depletion of TFIIB did not affect cell viability (Fig. 8A), which is in line with recent report (Gelev et al., 2014). Expression profiles of genes were tested with different modes of transcriptional regulation. TFIIB depletion did not significantly affect basal or induced expression of any of the EGF targets tested (Fig. 8D) suggesting that induced gene expression of genes that are regulated by polymerase pausing do not require TFIIB. In contrast, TFIIB was required for some IFN-a/b stimulated targets: IFN-a/b dependent induction of OAS2, GBP4, CXCL10, CXCL11, ISG15, Mx1 and IFITs was more than 2-fold impaired in the absence of TFIIB (Fig. 8B), whereas induction of other genes such as A20, IRF1, IkBa OAS1 and OAS3 was only not affected (Fig. 8B). Similarly, targets of TNFa signaling fell in two categories: affected genes included IL6, IL8, CXCL10 and IFIT proteins (Fig. 8C), whereas induction of IRF1, A20 and IkBa was not significantly altered (Fig. 8C). In sum, these experiments show that TFIIB is essential for induced expression of genes that require *de novo* polymerase II recruitment, but that TFIIB is dispensable for induced expression of genes regulated through polymerase pausing. These data are fully consistent with the inhibitory activity of ML on reporter constructs.

### Example 6 - Mapping of the ML functional inhibitory domain

Selective regulation of gene expression would allow for the possibility to interfere with *de novo* transcription, which would be of particular interest to modulate inflammatory processes associated with pathogenicity and disease. Compared to M, ML has a 38 AA C-terminal extension and it was hypothesized that expression of this fragment may be sufficient to regulate TFIIB activity. GFP-fusion constructs containing C-terminal fragments of ML (Fig. 9A) were generated and tested for their ability to directly associate with TFIIB using *in vitro* translated and [35S]-Methionine-Cysteine-labelled GFP-ML fragments for co-precipitation experiments with recombinant TFIIB. The 38 AA bearing GFP-ML (266-304) protein failed to bind TFIIB, while GFP-ML (257-304; C-terminal 47 AA), GFP-ML (247-304; C-terminal 57 AA) and GFP-ML (257-294) successfully precipitated with TFIIB. This suggests that physical association of TFIIB requires amino acids encoded in the L region of ML and also in the C-terminus of M. To assess their functionality, the same fragments with ISRE reporter into HEK293 cells were co-transfected. After stimulation with IFN-a/b, only the 57 AA ML fragment (GFP-ML (247-304)) was able to inhibit ISRE promoter activation (Fig. 9C). Despite binding of TFIIB, GFP-ML (257-304) and GFP-ML (247-294) were inactive in this assay (Fig. 9B, C). The minimal length of ML required to inhibit ISRE activity was mapped to region 249-304 (Fig. 9D). *E. coli* expressed recombinant 247-304 fragment (here named µL) fused with Tat cell permeable peptide was purified, as well as a µl (TESW mutant) bearing TE/AA and SW/AA mutations to exclude any binding to cellular proteins and associated inhibitory activity (Fig. 10A). Purified proteins were verified in their integrity by size exclusion chromatography and total mass spectrometry (data not shown). To assess their function, ISRE reporter and purified proteins were co-transfected into HEK293 cells and the cells were stimulated with IFN-a/b. Consistent with regulatory properties, Tat-µL wt was able to inhibit ISRE promoter activity, while Tat-µL (TESW/AAAA) failed to impair IRSE activity (Fig. 10B). Furthermore, Tat-µL, but not Tat-µL (TESW/AAAA) was also able to suppress IFN-a/b induced IFIT3 gene expression (Fig. 10C, left). At the same time SOCS1 expression (that is regulated through polymerase pausing) was not affected after Tat-µL transfection (Fig. 10C, right) clearly indicating specificity of Tat-µL. A chemically synthesised version of the µL peptide (µL-Tat) (Fig. 10E) also had the ability to inhibit IFN-a/b activated Mx1-luciferase in 293-Mx1-luc cells. Furthermore, µL-Tat inhibited LPS-induced IFN beta in bone marrow derived macrophages (BMDMs). In sum, this demonstrates, that a C-terminal fragment of ML is sufficient to bind TFIIB and inhibit its associated activity, recapitulating the phenotype of full-length protein.

### Example 7 - Impairment of herpes simplex virus 1 replication by ML and µL

Herpesviruses are known to recruit the cellular transcription machinery to express their genes (Boehmer and Nimonkar, 2003; Costanzo et al., 1977). It has been shown that HSV-1 requires TFIIB for successful replication (Gelev et al., 2014). Furthermore, in case of KSHV, TFIIB was directly detected at the promoter of a lytic gene (Davis et al., 2015). Given potential susceptibility of herpesviruses to TFIIB absence, it was tested whether ML can inhibit herpesviral replication. Herpesviral DNA promoter sequences are often used to drive expression from plasmids. In agreement with the requirement of viral promoters to utilize cellular transcription factors, HSV-1 Thymidine kinase driven Renilla expression was impaired by co-transfection of ML expression plasmid, but not when ML (SW/AA) was used (Fig. 11A). Similarly, Cytomegalovirus Immediate early promoter (CMV IE) driven TFIIB expression was inhibited by ML expression (Fig. 11 B), whereas expression of endogenous TFIIB was not affected by ML presence. Interestingly, ML expression itself was only minimal when expressed from CMV IE promoter (Fig. 11 B). In order to check, whether the replication of herpesviruses is compromised in the presence of ML, HeLa Flipln cells with stably integrated doxycycline inducible ML were generated (Fig. 11C). These cells were induced to express ML and subsequently infected with HSV-1, Vaccinia virus strain Western reserve (VACV WR) or Vesicular stomatitis virus M2 variant (VSV M2). As expected, replication of HSV-1 was severely inhibited in cells expressing ML, while replication of VACV (DNA virus encoding its own RNA polymerase machinery) and of VSV M2 (RNA virus with its own RNA polymerase machinery) was not affected (Fig. 11 D). In sum, these experiments indicate that ML impairs TFIIB activity and does not in general cellular activities and highlights the possibility that ML and µL can be used for treatment of TFIIB-dependent viruses.

### References

Boehmer, P.E., and Nimonkar, A.V. (2003). Herpes virus replication. IUBMB life 55, 13-22.
Bushnell, D.A., Westover, K.D., Davis, R.E., and Kornberg, R.D. (2004). Structural basis of transcription: an RNA polymerase II-TFIIB cocrystal at 4.5 Angstroms. Science 303, 983-988.
Chen, Z., Rijnbrand, R., Jangra, R.K., Devaraj, S.G., Qu, L., Ma, Y., Lemon, S.M., and Li, K. (2007). Ubiquitination and proteasomal degradation of interferon regulatory factor-3 induced by Npro from a cytopathic bovine viral diarrhea virus. Virology 366, 277-292.
Costanzo, F., Campadelli-Fiume, G., Foa-Tomasi, L., and Cassai, E. (1977). Evidence that herpes simplex virus DNA is transcribed by cellular RNA polymerase B. Journal of virology 21, 996-1001.
Davis, Z.H., Verschueren, E., Jang, G.M., Kleffman, K., Johnson, J.R., Park, J., Von Dollen, J., Maher, M.C., Johnson, T., Newton, W., et al. (2015). Global mapping of herpesvirus-host protein complexes reveals a transcription strategy for late genes. Molecular cell 57, 349-360.
Freaney, J.E., Kim, R., Mandhana, R., and Horvath, C.M. (2013). Extensive cooperation of immune master regulators IRF3 and NFkappaB in RNA Pol II recruitment and pause release in human innate antiviral transcription. Cell reports 4, 959-973.
Gardini, A., Baillat, D., Cesaroni, M., Hu, D., Marinis, J.M., Wagner, E.J., Lazar, M.A., Shilatifard, A., and Shiekhattar, R. (2014). Integrator regulates transcriptional initiation and pause release following activation. Molecular cell 56, 128-139.
Gelev, V., Zabolotny, J.M., Lange, M., Hiromura, M., Yoo, S.W., Orlando, J.S., Kushnir, A., Horikoshi, N., Paquet, E., Bachvarov, D., et al. (2014). A new paradigm for transcription factor TFIIB functionality. Scientific reports 4, 3664.
Hagmaier, K., Jennings, S., Buse, J., Weber, F., and Kochs, G. (2003). Novel gene product of Thogoto virus segment 6 codes for an interferon antagonist. Journal of virology 77, 2747-2752.
Hilton, L., Moganeradj, K., Zhang, G., Chen, Y.H., Randall, R.E., McCauley, J.W., and Goodbourn, S. (2006). The NPro product of bovine viral diarrhea virus inhibits DNA binding by interferon regulatory factor 3 and targets it for proteasomal degradation. Journal of virology 80, 11723-11732.
Janky, R., Verfaillie, A., Imrichova, H., Van de Sande, B., Standaert, L., Christiaens, V., Hulselmans, G., Herten, K., Naval Sanchez, M., Potier, D., et al. (2014). iRegulon: from a gene list to a gene regulatory network using large motif and track collections. PLoS computational biology 10, e1003731.
Jennings, S., Martinez-Sobrido, L., Garcia-Sastre, A., Weber, F., and Kochs, G. (2005). Thogoto virus ML protein suppresses IRF3 function. Virology 331, 63-72.
Kainulainen, M., Habjan, M., Hubel, P., Busch, L., Lau, S., Colinge, J., Superti-Furga, G., Pichlmair, A., and Weber, F. (2014). Virulence factor NSs of rift valley fever virus recruits the F-box protein FBXO3 to degrade subunit p62 of general transcription factor TFIIH. Journal of virology 88, 3464-3473.
Kandiah, E., Trowitzsch, S., Gupta, K., Haffke, M., and Berger, I. (2014). More pieces to the puzzle: recent structural insights into class II transcription initiation. Current opinion in structural biology 24, 91-97.
Le May, N., Dubaele, S., Proietti De Santis, L., Billecocq, A., Bouloy, M., and Egly, J.M. (2004). TFIIH transcription factor, a target for the Rift Valley hemorrhagic fever virus. Cell 116, 541-550.
Pichlmair, A., Buse, J., Jennings, S., Haller, O., Kochs, G., and Staeheli, P. (2004). Thogoto virus lacking interferon-antagonistic protein ML is strongly attenuated in newborn Mx1-positive but not Mx1-negative mice. Journal of virology 78, 11422-11424.
Saunders, A., Core, L.J., and Lis, J.T. (2006). Breaking barriers to transcription elongation. Nature reviews Molecular cell biology 7, 557-567.
Tang, H., Sun, X., Reinberg, D., and Ebright, R.H. (1996). Protein-protein interactions in eukaryotic transcription initiation: structure of the preinitiation complex. Proceedings of the National Academy of Sciences of the United States of America 93, 1119-1124.
Tullai, J.W., Schaffer, M.E., Mullenbrock, S., Sholder, G., Kasif, S., and Cooper, G.M. (2007). Immediate-early and delayed primary response genes are distinct in function and genomic architecture. The Journal of biological chemistry 282, 23981-23995.
Vogt, C., Preuss, E., Mayer, D., Weber, F., Schwemmle, M., and Kochs, G. (2008). The interferon antagonist ML protein of thogoto virus targets general transcription factor IIB. Journal of virology 82, 11446-11453.

## Claims

1. A nucleic acid molecule encoding a polypeptide capable of binding to TFIIB and inhibiting gene expression, wherein the polypeptide
(a) has the amino acid sequence of SEQ ID NO: 1;
(b) is a fragment of the amino acid sequence of (a), said fragment lacking up to five amino acids at the C-terminus of the amino acid sequence of (a); or
(c) has an amino acid sequence being at least 80% identical to the amino acid sequence of (a) or (b).

2. The nucleic acid molecule of claim 1, wherein the polypeptide of (a) has the amino acid sequence of SEQ ID NO: 2, and preferably of SEQ ID NO: 3.

3. The nucleic acid molecule of claim 1, wherein the polypeptide of (a) is selected from the group consisting of SEQ ID NOs 4 to 9.

4. A fusion construct comprising the nucleic acid molecule of any one of claims 1 to 3 fused with a heterologous polynucleotide.

5. A vector comprising the nucleic acid molecule of any of claims 1 to 3 or the fusion construct of claim 4.

6. A cell comprising the nucleic acid molecule of any of claims 1 to 3, the fusion construct of claim 4, the vector of claim 5 or a combination thereof.

7. A method of producing a polypeptide capable of binding to TFIIB and inhibiting gene expression, wherein the method comprises
(a) culturing the cell of claim 6, and
(b) isolating the produced polypeptide capable of binding to TFIIB and inhibiting gene expression.

8. A polypeptide capable of binding to TFIIB and inhibiting gene expression encoded by the nucleic acid molecule of any one of claims 1 to 3, the fusion construct of claims 4, the vector of claim 5 or produced by the method of claim 7.

9. An *in vitro* method for inhibiting the expression of a gene, said method comprising contacting
(i) a cell comprising the gene, or
(ii) an *in vitro* expression system comprising the gene
with the nucleic acid molecule of any of claims 1 to 3, the fusion construct of claim 4, the vector of claim 5, the cell of claim 6, the polypeptide of claim 8 or a combination thereof.

10. The *in vitro* method of claim 9, wherein the gene requires transcription Factor II B (TFIIB).

11. A pharmaceutical composition comprising the nucleic acid molecule of any of claims 1 to 3, the fusion construct of claim 4, the vector of claim 5, the cell of claim 6, the polypeptide of claim 8 or a combination thereof.

12. The pharmaceutical composition of claim 11 for use in treating a disease selected from the group consisting of a disease caused by overreaction of the immune system, an autoimmune disease, an inflammatory disease, a viral infection, an immunopathy induced by a pathogen and/or a toxin, an allergy, septic shock, tissue damage, a hyperproliferative disease, and a metabolic disorder.

13. The pharmaceutical composition for use of claim 12, wherein the autoimmune and/or autoinflammatory disease is selected from rheumatoid arthritis, multiple sclerosis, corneal transplant rejection, transplant rejection, uveitis, Type I diabetes, schizophrenia, alopecia areata, psoriasis vulgaris, vitiligo, pemphigus vulgaris, Alzheimer's, Parkinson's disease, Crohn's disease, lupus erythematosus, Sjorgen's syndrome, epidermolysis bullosa, sarcoidosis, autoinflammatory diseases (such as FMF, NOMID, TRAPS), and coeliac disease.

14. The pharmaceutical composition for use of claim 12, wherein the viral infection is caused by a DNA virus or RNA virus with a DNA intermediate, such as a herpes virus, an adeno virus, a papova viruses, a hepadna virus, or a retrovirus.

15. The pharmaceutical composition for use of claim 12, wherein the inflammatory disease is selected from inflammatory bowel disease, nephritis, pelvic inflammatory disease, vasculitis, a chronic inflammation of an organ, ankylosing spondylitis, asthma, atherosclerosis, gastritis, colitis, dermatitis, diverticulitis, hepatitis, conjunctivitis, sinusitis, ulcerative colitis, psoriasis, allergic reactions, eczema, laryngitis, pharyngitis, tonsilitis, acnce, hypersensitivity, and reperfusion injury.
